# EUROPEAN PATENT APPLICATION

(11) **EP 4 261 222 A1**
(43) Date of publication of application: **18.10.2023**
(21) Application number: 20964996.1
(22) Date of filing: 09.12.2020
(51) Int. Cl.: C07K 16/28, C07K 16/24, A61P 29/00, A61P 37/00, G01N 33/563, G01N 33/564, A61K 39/00

(54) **ANTI-OX40L ANTIBODY, ANTI-OX40L/ANTI-TNFalpha BISPECIFIC ANTIBODY, AND USES THEREOF**

(71) Applicant: HK inno.N Corporation, Cheongju-si, Chungcheongbuk-do 28158 (KR); Y-Biologics Inc., Daejeon 34014 (KR); Imbiologics Corp., Suwon-si Gyeonggi-do 16229 (KR)
(72) Inventor: YOO, Jung Min, Seoul 01417 (KR); LEE, Chung Min, Seongnam-si Gyeonggi-do 13629 (KR); LEE, Yoon Jung, Yongin-si Gyeonggi-do 16874 (KR); KANG, Hyeon Ju, Yongin-si Gyeonggi-do 17047 (KR); JUNG, Seung Hee, Yongin-si Gyeonggi-do 16846 (KR); CHOI, Jong Ryoul, Guri-si Gyeonggi-do 11920 (KR); CHO, Kyu Eun, Seoul 05274 (KR); HA, Gyong Sik, Seoul 07287 (KR); KIM, Soo Young, Daejeon 35235 (KR); PARK, Bum Chan, Daejeon 34140 (KR); PARK, Jae Eun, Sejong-si 30062 (KR); SHIM, Eun Young, Daejeon 34304 (KR); LEE, Hyun Mi, Sejong-si 30124 (KR)
(74) Representative: HGF
(86) International application number: PCT/IB2020/061683
(87) International publication number: WO 2022/123293

(57) **Abstract**

The present invention relates to a novel antibody specifically binding to OX40L and a bispecific antibody specifically binding to OX40L and TNFα and, specifically, to an antibody or a bispecific antibody specifically binding to human OX40L to effectively inhibit the binding of OX40 to an OX40 receptor; a nucleic acid encoding the antibody; an expression vector comprising the nucleic acid; a transformant comprising the expression vector; a method for preparing the antibody; a pharmaceutical composition for treating autoimmune diseases or inflammatory diseases, comprising the antibody; a composition for diagnosing autoimmune diseases or inflammatory diseases, comprising the antibody; a method for diagnosing autoimmune diseases or inflammatory diseases by using the antibody; a method for providing information on diagnosis of autoimmune diseases or inflammatory diseases by using the antibody; and a kit for providing the same.

## Description

### Technical Field

The present invention relates to a novel antibody specifically binding to OX40L and a bispecific antibody specifically binding to OX40L and TNFα, specifically, to an antibody or a bispecific antibody specifically binding to human OX40L to effectively inhibit the binding of OX40 to an OX40 receptor; a nucleic acid encoding the antibody; an expression vector including the nucleic acid; a transformant including the expression vector; a method for preparing the antibody; a pharmaceutical composition for preventing or treating autoimmune diseases or inflammatory diseases, including the antibody; a composition for diagnosing autoimmune diseases or inflammatory diseases, including the antibody; a method for diagnosing autoimmune diseases or inflammatory diseases by using the antibody; a method for providing information on diagnosis of autoimmune diseases or inflammatory diseases by using the antibody; and a kit for providing the same.

### Background

Autoimmune diseases or inflammatory diseases are caused by abnormal activation of human immunity. In the case of rheumatoid arthritis, which is a representative condition of autoimmune diseases, TNFα inhibitors have accounted for 68% of the therapeutics market.

Tumor necrosis factor α (TNFα) is a cytokine produced by a number of cell types, including monocytes and macrophages, and has been first identified by its ability to induce necrosis of certain mouse tumors [see Old, L. (1985) Science230: 630-632]. After that, it was found that a factor named cachectin, which is associated with cachexia, is the same molecule as TNFα. TNFα is involved in mediating shock [see Beutler, B. and Cerami, A. (1988) Annu. Rev.Biochem. 57: 505-518; Beutler, B. and Cerami, A. (1989) Annu. Rev. Immunol. 7: 625-655]. In addition, TNFα has been implicated in the pathophysiology of various human diseases and disorders, including sepsis, infectious diseases, autoimmune diseases, transplant rejection and transplant-versus-host disease [see Vasilli, P. (1992) Annu. Rev. Immunol. 10: 411-452; Tracey, K. J. and Cerami, A. (1994) Annu. Rev. Med. 45: 491- 503].

Because of the deleterious role of human TNFα (hTNFa) in various diseases, therapeutic strategies have been designed to inhibit or counteract hTNFa activity. In particular, antibodies which bind to and neutralize hTNFa have been used as a means to inhibit the hTNFa activity. The hTNFa-neutralizing antibodies include a mouse monoclonal antibody (mAb) secreted by hybridomas obtained from lymphocytes of mice immunized with hTNFa [see Hahn T; et al., (1985) Proc Natl Acad Sci USA 82: 3814-3818; Liang, C-M., et al. (1986) Biochem. Biophys. Res. Commun. 137:847-854; Hirai, M., et al. (1987) J. Immunol. Methods 96: 57-62; Fendly, B. M., et al. (1987) Hybridoma 6: 359-370; Muller, A., et al L. (1990) Cytokine 2: 162-169; U.S Patent No. 5,231,024 (Moeller et al); EP Patent Publication No. 186833 B1 (Wallach, D.); EP Patent Publication No. 218 868 A1 (Old et al.); EP Patent Publication No. 260 610 B1 (Moeller, A., et. al)] or a chimera antibody [see Knight, D. M, et al. (1993) Mol. Immunol.30: 1443-1453; PCT Unexamined Publication WO 92/16553 (Daddona, P. E., et al.)] or a humanized monoclonal antibody [see PCT Unexamined Publication WO 92/11383 (Adair, J. R., et al.)] or a human monoclonal antibody [see U.S. 10-1142825], or the like. These anti-hTNFa antibodies may have high affinity for hTNFa (e.g., Kd ≤ 10⁻⁹M) and neutralize the hTNFa activity. These anti-hTNFa antibodies have been used as therapeutic agents in various autoimmune diseases, infections, transplant rejection, graft-versus-host disease, and the like.

However, about 50% of a patient group is refractory to TNFα inhibitors such as these anti-hTNFa antibodies (Nature Reviews Rheumatology vol. 11, 276-289(2015). In addition, the recently developed targets for autoimmune diseases are CTLA-4, IL-6, JAK1, JAK2, CD20 and the like, but the drugs derived therefrom have failed to show efficacy as much as TNFα inhibitors (Nature Reviews Rheumatology vol. 11, 276-289(2015).

In particular, autoimmune diseases such as rheumatoid arthritis, etc., are not simply caused by an abnormality of one type of immune cell, but rather occur as a problem of the entire immune system. Thus, an existing method for developing a therapeutic agent to inhibit only a single target has a limit to enhancing the efficacy of the therapeutic agent. Thus, in order to overcome such limit of efficacy, a bispecific or multi-specific antibody, which controls two or more targets having different mechanisms of action at one time, has been developed now. However, an existing bispecific antibody acts with a limitation to specific cells in the innate or adaptive immune system, and thus fails to improve the overall homeostasis of the immune system.

### Detailed Description of the Invention

### Technical Problem

An object of the present invention is to provide an anti-OX40L antibody or an antigen-binding fragment thereof specifically binding to OX40L (OX40 ligand).

An object of the present invention is to provide an anti-OX40L antibody specifically binding to OX40L, which recognizes a conformational epitope of OX40L, including: amino acid sequence nos. 93 to 100 represented by SEQ ID NO: 3, and amino acid sequence nos. 141 to 151 represented by SEQ ID NO: 4 in an amino acid sequence of OX40L protein represented by SEQ ID NO: 1.

An object of the present invention is to provide a bispecific antibody, which includes an anti-OX40L antibody or an antigen-binding fragment thereof specifically binding to OX40L (OX40 ligand); and an antibody or an antigen-binding fragment thereof specifically binding to tumor necrosis factor α (TNEα).

An object of the present invention is to provide a nucleic acid encoding the anti-OX40L antibody, the binding fragment thereof or the bispecific antibody; an expression vector having the nucleic acid introduced thereinto; or a host cell having the expression vector introduced thereinto.

An object of the present invention is to provide a method for producing an anti-OX40L antibody, an antigen-binding fragment thereof, or a bispecific antibody by using the host cell.

An object of the present invention is to provide a pharmaceutical composition for preventing or treating autoimmune diseases or inflammatory diseases, including the anti-OX40L antibody, the antigen-binding fragment thereof, or the bispecific antibody.

An object of the present invention is to provide a composition for diagnosing autoimmune diseases or inflammatory diseases, including the anti-OX40L antibody, the antigen-binding fragment thereof, or the bispecific antibody.

An object of the present invention is to provide a composition for detecting at least one of above OX40L and TNFα, including the anti-OX40L antibody, the antigen-binding fragment thereof, or the bispecific antibody.

An object of the present invention is to provide a method for providing information on the diagnosis of autoimmune diseases or inflammatory diseases by using the anti-OX40L antibody, the antigen-binding fragment thereof, or the bispecific antibody.

An object of the present invention is to provide a kit for providing information on the diagnosis of autoimmune diseases or inflammatory diseases, including the anti-OX40L antibody, the antigen-binding fragment thereof, or the bispecific antibody.

An object of the present invention is to provide a method for preventing or treating autoimmune diseases or inflammatory diseases, including administering a pharmaceutically effective amount of the anti-OX40L antibody or the antigen-binding fragment thereof, or the bispecific antibody.

An object of the present invention is to provide a use of the anti-OX40L antibody or the antigen-binding fragment thereof, or the bispecific antibody in the manufacture of a medicament for preventing or treating autoimmune diseases or inflammatory diseases.

An object of the present invention is to provide a use of the anti-OX40L antibody or the antigen-binding fragment thereof, or the bispecific antibody for preventing or treating autoimmune diseases or inflammatory diseases.

### Technical Solution

Each description and embodiment disclosed in the present invention may be applied to other descriptions and embodiments thereof, respectively. In other words, all the combinations of various elements disclosed in the present invention fall within the scope of the present invention.

In addition, it may not be seen that the scope of the present invention is limited to the specific descriptions described below.

The present invention may provide an anti-OX40L antibody or a binding fragment thereof, specifically binding to OX40L and inhibiting an interaction between OX40L and an OX40 receptor.

As used herein, the term "antibody" may refer to a protein molecule which serves as a receptor specifically recognizing an antigen including an immunoglobulin molecule immunologically having reactivity with a certain antigen, and this term may include a polyclonal antibody, a monoclonal antibody, a whole antibody, and a binding fragment all. In addition, the above term may include a chimeric antibody (for example, a humanized murine antibody), a humanized antibody, a human antibody and a bivalent or bispecific molecule (for example, a bispecific antibody), a diabody, a triabody or a tetrabody.

Typically, immunoglobulin may have a heavy chain and a light chain, and each of the heavy chain and the light chain may include a constant region and a variable region (the region is also known as a domain). The variable region of the heavy chain and the light chain may include three multi-variable regions and four framework regions, which are called a complementarity-determining region (CDR). The CDR may play a role in mainly binding to an epitope of an antigen. The CDR of each chain may sequentially refer to CDR1, CDR2 and CDR3, typically starting from an N-terminus, and may be also identified by a chain in which a certain CDR is positioned.

The whole antibody may take on a structure of having two full-length light chains and two full-length heavy chains, in which each light chain is linked to a heavy chain by a disulfide bond. The whole antibody may include IgA, IgD, IgE, IgM and IgG, and IgG may include IgG₁, IgG₂, IgG₃ and IgG₄ as a subtype. A heavy chain constant region may have gamma (γ), mu (µ), alpha (α), delta (δ) and epsilon (ε) types, and may also have gamma 1 (γ1), gamma 2 (γ2), gamma 3 (γ3), gamma 4 (γ4), alpha 1 (α1) and alpha 2 (a2) as a subclass. A light chain constant region may have kappa (κ) and lambda (λ) types.

As used herein, the terms "fragment," "antibody fragment," "antigen-binding fragment" and "binding fragment" may refer to any fragment of the antibody of the present invention, which holds an antigen-binding function, and may be interchangeably used with each other and may include Fab, Fab', F(ab')₂, Fv, and the like.

The Fab may have one antigen-binding site, which takes on a structure of having a variable region of a light chain and a heavy chain, a constant region of a light chain, and a first constant region (CH1 domain) of a heavy chain. The Fab' may have a difference from Fab, in that the Fab' has a hinge region including at least one cysteine residue at C terminus of a heavy chain CH1 domain. The F(ab')₂ antibody may be produced in such a way that a cysteine residue of the hinge region of Fab' forms a disulfide bond. The variable fragment (Fv) may refer to a minimum antibody fragment having only a heavy chain variable region and a light chain variable region. In case of double-stranded Fv (dsFv), a heavy chain variable region and a light chain variable region are linked to each other by a disulfide bond. In case of single-chain Fv (scFv), a heavy chain variable region and a light chain variable region are generally linked to each other by a covalent bond through a peptide linker. The binding fragment may be obtained by using protease (for example, Fab may be obtained by performing restriction digestion of a whole antibody with papain, while F(ab')₂ fragment may be obtained by doing so with pepsin), and may be prepared, for example, through a gene recombination technique.

As used herein, the term "monoclonal antibody" may refer to an antibody molecule of a single molecular composition obtained from a group of substantially the same antibodies, and this monoclonal antibody may show single binding specificity and affinity for a certain epitope. In examples of the present invention, an anti-OX40L antibody specifically binding to OX40L or a bispecific antibody specifically binding to OX40L and TNFα according to the present invention may be a single molecular antibody. Specifically, the anti-OX40L antibody may refer to an antibody of a single molecular composition specifically binding to a certain epitope of OX40L, and the bispecific antibody may refer to a bispecific antibody of a single molecular composition specifically binding to certain epitopes of OX40L and TNFα at the same time.

In examples of the present invention, the anti-OX40L antibody and the bispecific antibody specifically binding to OX40L and TNFα may be a chimeric antibody, a humanized antibody, or a human antibody, but are not limited thereto.

As used herein, the term "chimera antibody" may be an antibody created by recombining a variable region of a mouse antibody and a constant region of a human antibody, and may be an antibody which has a great improvement in immune responses compared to the mouse antibody.

As used herein, the term "humanized antibody" may refer to an antibody, which is modified in such a way that a protein sequence of an antibody derived from non-human species becomes similar to an antibody variant naturally produced from humans. As an example, the humanized antibody may be prepared in such a way that a mouse-derived CDR is recombined with a human antibody-derived FR to prepare a humanized variable region, and then recombined with a constant region of a desired human antibody. However, if only CDR grafting is simply performed, the affinity of the humanized antibody may become low. Thus, such low affinity may be raised up to the same level as the affinity of an original mouse antibody, in such a way that several important FR amino acid residues considered to have an influence on a three-dimensional structure of the CDR are allowed to have more affinity with those of the mouse antibody.

As used herein, the term "human antibody" may refer to a molecule derived from human immunoglobulin, in which all the amino acid sequences forming an antibody including a complementarity-determining region and a framework region are composed of the amino acid sequences of human immunoglobulin. Human antibodies are commonly used in the treatment of human diseases, and may have three or more potential advantages. Firstly, human antibodies may interact better with the human immune system and thus more efficiently destroy target cells, for example, through complement-dependent cytotoxicity (CDC) or antibody-dependent cell mediated cytotoxicity (ADCC). Secondly, there is an advantage in that the human immune system does not recognize those antibodies as heterogenous. Thirdly, there is an advantage in that the half-life of the human antibodies in the human circulatory system is similar to that of naturally occurring antibodies even when a smaller amount of the drug is administered less frequently. In examplets of the present invention, an anti-OX40L antibody specifically binding to OX40L and a bispecific antibody specifically binding to OX40L and TNFα according to the present invention may be a human antibody. Thus, the human anti-OX40L antibody of the present invention and the human bispecific antibody of the present invention may show strong affinity for OX40L, so as to effectively inhibit the binding of OX40L-expressing cells (e.g., monocytes) to the OX40 receptor, and may be also useful in the treatment of diseases such as autoimmune diseases or inflammatory diseases, since the domains of both heavy chain and light chain are derived from humans, thereby showing low immunogenicity.

As used herein, the term "OX40L" may refer to a ligand for an OX40 protein as a receptor, and specifically refer to a protein binding to the OX40 receptor. Information on OX40L may be obtained from publicly known databases such as GenBank of the National Institutes of Health, etc., and may include, for example, information on OX40L, which includes an amino acid sequence of SEQ ID NO: 1, in which an accession number is Gene ID:54567, NCBI Reference Sequence: NM_003326.5 (TNFSF4 ver1), and NM_001297562.2 (TNFSF4 ver2).

OX40L may be overexpressed in antigen-presenting cells (APC) and may be known to activate several immune cells. Like TNFα and INFγ, OX40L was specifically observed to be excessively produced in patients with autoimmune diseases (Eur. J. Immunol. 2000. 30: 2815-2823). Unlike TNFα, which is distributed all over the body, OX40L may be produced only in activated immune cells and may be mainly distributed at a lesion site. OX40L may be a multiple immunomodulatory protein which is involved in regaining homeostasis of the immune system by simultaneously participating in the antigen-presenting cells (APC) of the innate immune system and the T helper cells of the adaptive immune system (Nature Reviews Rheumatology vol. 12, 74-76(2016), (Clinic Rev Allerg Immunol, 2016).

As used herein, the term "OX40" may refer to a protein which mediates OX40/OX40L signaling. Above OX40 may include any protein without limitation as long as the protein mediates OX40/OX40L signaling.

As used herein, the term "inhibiting an interaction between OX40L and OX40" or "inhibiting an interaction between OX40L and the OX40 receptor" may mean that an interaction between OX40L and OX40 is suppressed or inhibited in such a way that an anti-OX40L antibody or a binding fragment thereof specifically binding to OX40L of the present invention binds to OX40L. In case that an anti-OX40L antibody or a binding fragment thereof binds to OX40L, the biological functions of OX40L may be suppressed or inhibited so as not to cause OX40 signaling. In other words, the interaction between OX40L and OX40 may be suppressed or inhibited by the binding of the anti-OX40L antibody or the binding fragment thereof to OX40L, so that OX40 signaling may be suppressed or inhibited.

In the present invention, "anti-OX40L antibody specifically binding to OX40L" may refer to an antibody which specifically binds to OX40L so as to suppress or reduce the biological activity of OX40L. The antibody may suppress or inhibit the biological activity of OX40L, thereby suppressing or inhibiting the interaction between OX40L and the OX40 receptor. In the present specification, "anti-OX40L antibody specifically binding to OX40L" may be used interchangeably with "antibody specifically binding to OX40L" or "anti-OX40L antibody."

The form of the anti-OX40L antibody may include both the whole antibody and the binding fragment thereof as described above. The anti-OX40L antibody of the present invention may specifically bind to human OX40L and inhibit the interaction between OX40L and the OX40 receptor, and thus may be useful in the treatment of diseases such as autoimmune diseases or inflammatory diseases, and may also specifically bind to human OX40L, which is overexpressed in autoimmune diseases or inflammatory diseases, thereby maximizing a therapeutic effect while minimizing side effects.

In examples of the present invention, the anti-OX40L antibody or the antigen-binding fragment thereof specifically binding to OX40L and inhibiting the interaction between OX40L and the OX40 receptor may bind to human OX40L with a K_{D} of by 3 × 10⁻⁹ M or less. Specifically, the anti-OX40L antibody or the antigen-binding fragment thereof may bind with a K_{D} of 1.5 × 10⁻⁹ M, 1.3 × 10⁻⁹ M, particularly, 1 × 10⁻⁹ M or less.

As used herein, the term "association constant (Kₒₙ)" may refer to a ratio of association in a specific antibody-antigen interaction, and the term "dissociation constant (K_{off})" may refer to a ratio of dissociation in a specific antibody-antigen interaction. In addition, the term "affinity for antigen (K_{D})" as used herein may refer to a ratio of K_{off} : Kon (i.e., K_{off} /Kₒₙ) expressed as a molar concentration (M). A K_{D} value for an antibody may be measured by using methods well established in the art. For example, the method for measuring the K_{D} value of an antibody may include surface plasmon resonance analysis using the BioCore^{™} system, but is not limited thereto.

According to the present invention, the anti-OX40L antibody or the antigen-binding fragment thereof may show high binding force for OX40L so as to suppress or inhibit the activity of OX40L even at a low concentration, and thus may show an excellent therapeutic effect on autoimmune diseases or inflammatory diseases.

In examples of the present invention, the anti-OX40L antibody or the antigen-binding fragment thereof may recognize a conformational epitope of human OX40L. For example, the anti-OX40L antibody or the antigen-binding fragment thereof according to the present invention may bind to amino acid sequences nos. 93 to 100 and 141 to 151 in the amino acid sequence of the human OX40L protein represented by SEQ ID NO: 1 with a K_{D} of 3 × 10⁻⁹ M or less. Specifically, the anti-OX40L antibody or the antigen-binding fragment thereof may bind to amino acid sequences nos. 93 to 100 and 141 to 151 in the amino acid sequence of the human OX40L protein represented by SEQ ID NO: 1 with a K_{D} of 1.5 × 10⁻⁹ M, 1.3 × 10⁻⁹ M, particularly, 1 × 10⁻⁹ M or less.

In examples of the present invention, the anti-OX40L antibody or the antigen-binding fragment thereof may bind to the amino acid sequence of the human OX40L protein represented by SEQ ID NO: 3 or SEQ ID NO: 4 with high binding force, specifically with a K_{D} of 3 × 10⁻⁹ M or less. Specifically, the anti-OX40L antibody or the antigen-binding fragment thereof may bind with a K_{D} of 1.5 × 10⁻⁹ M, 1.3 × 10⁻⁹ M, particularly, 1 × 10⁻⁹ M or less.

In examples of the present invention, K_{D} for human OX40L of the anti-OX40L antibody or the antigen-binding fragment thereof may be the one measured by surface plasmon resonance (Biacore) analysis.

The anti-OX40L antibody or the antigen-binding fragment thereof may specifically include the sequences as mentioned below, but is not limited thereto.

In examples of the present invention, the anti-OX40L antibody or the antigen-binding fragment thereof may include:
a heavy chain variable region including heavy chain CDR1 represented by one amino acid sequence selected from the group consisting of SEQ ID NOs: 12, 13 and 14; heavy chain CDR2 represented by one amino acid sequence selected from the group consisting of SEQ ID NOs: 15, 16, 17 and 18; and heavy chain CDR3 represented by one amino acid sequence selected from the group consisting of SEQ ID NOs: 19, 20, 21 and 22; and
a light chain variable region including light chain CDR1 represented by one amino acid sequence selected from the group consisting of SEQ ID NOs: 23 and 24; light chain CDR2 represented by one amino acid sequence selected from the group consisting of SEQ ID NOs: 25 and 26; and light chain CDR3 represented by one amino acid sequence selected from the group consisting of SEQ ID NOs: 27, 28, 29 and 30.

As used herein, the term "heavy chain" may include both a full-length heavy chain and a fragment thereof including a variable region domain VH and three constant region domains CH1, CH2 and CH3, which include an amino acid sequence having a variable region sequence enough to give specificity to an antigen.

In addition, the term "light chain" as used herein may include both a full-length light chain and a fragment thereof including a variable region domain VL and a constant region domain CL, which include an amino acid sequence having a variable region sequence enough to give specificity to an antigen.

In examples of the present invention, the anti-OX40L antibody or the antigen-binding fragment thereof may be an antibody including, but not limited to:
a heavy chain variable region including heavy chain CDR1 represented by SEQ ID NO: 12; heavy chain CDR2 represented by SEQ ID NO: 15; and heavy chain CDR3 represented by SEQ ID NO: 19; and
a light chain variable region including light chain CDR1 represented by SEQ ID NO: 23; light chain CDR2 represented by SEQ ID NO: 25; and light chain CDR3 represented by SEQ ID NO: 27. In examples of the present invention, the antibody was named 02C09.

In examples of the present invention, the anti-OX40L antibody or the antigen-binding fragment thereof may be an antibody including, but not limited to:
a heavy chain variable region including heavy chain CDR1 represented by SEQ ID NO: 13; heavy chain CDR2 represented by SEQ ID NO: 16; and heavy chain CDR3 represented by SEQ ID NO: 20; and
a light chain variable region including light chain CDR1 represented by SEQ ID NO: 24; light chain CDR2 represented by SEQ ID NO: 26; and light chain CDR3 represented by SEQ ID NO: 28. In examples of the present invention, the antibody was named hu3F07 and I3F07.

In examples of the present invention, the anti-OX40L antibody or the antigen-binding fragment thereof may be an antibody including, but not limited to:
a heavy chain variable region including heavy chain CDR1 represented by SEQ ID NO: 13; heavy chain CDR2 represented by SEQ ID NO: 17; and heavy chain CDR3 represented by SEQ ID NO: 21; and
a light chain variable region including light chain CDR1 represented by SEQ ID NO: 24; light chain CDR2 represented by SEQ ID NO: 26; and light chain CDR3 represented by SEQ ID NO: 29. In embodiments of the present invention, the antibody was named 10H07.

In examples of the present invention, the anti-OX40L antibody or the antigen-binding fragment thereof may be an antibody including, but not limited to:
a heavy chain variable region including heavy chain CDR1 represented by SEQ ID NO: 14; heavy chain CDR2 represented by SEQ ID NO: 18; and heavy chain CDR3 represented by SEQ ID NO: 22; and
a light chain variable region including light chain CDR1 represented by SEQ ID NO: 24; light chain CDR2 represented by SEQ ID NO: 26; and light chain CDR3 represented by SEQ ID NO: 30. In examples of the present invention, the antibody was named 21G07.

In examples of the present invention, the anti-OX40L antibody or the antigen-binding fragment thereof may include:
a heavy chain variable region represented by one amino acid sequence selected from the group consisting of SEQ ID NOs: 37, 41, 45, 49 and 53; and
a light chain variable region represented by one amino acid sequence selected from the group consisting of SEQ ID NOs: 38, 42, 46, 50 and 54.

In examples of the present invention, the anti-OX40L antibody or the antigen-binding fragment thereof may include:
(a) a heavy chain variable region represented by SEQ ID NO: 37 and a light chain variable region represented by SEQ ID NO: 38;
(b) a heavy chain variable region represented by SEQ ID NO: 41 and a light chain variable region represented by SEQ ID NO: 42;
(c) a heavy chain variable region represented by SEQ ID NO: 45 and a light chain variable region represented by SEQ ID NO: 46;
(d) a heavy chain variable region represented by SEQ ID NO: 49 and a light chain variable region represented by SEQ ID NO: 50; or
(e) a heavy chain variable region represented by SEQ ID NO: 53 and a light chain variable region represented by SEQ ID NO: 54.

In examples of the present invention, the anti-OX40L antibody or the antigen-binding fragment thereof may include:
a heavy chain constant region represented by one amino acid sequence selected from the group consisting of SEQ ID NOs: 5, 6, 7, and 8; and
a light chain constant region represented by an amino acid sequence of SEQ ID NO: 10.

In examples of the present invention, the anti-OX40L antibody or the binding fragment thereof may be an antibody or a binding fragment thereof including:
(a) a heavy chain variable region represented by SEQ ID NO: 37 and a light chain variable region represented by SEQ ID NO: 38; (b) a heavy chain variable region represented by SEQ ID NO: 41 and a light chain variable region represented by SEQ ID NO: 42; (c) a heavy chain variable region represented by SEQ ID NO: 45 and a light chain variable region represented by SEQ ID NO: 46; (d) a heavy chain variable region represented by SEQ ID NO: 49 and a light chain variable region represented by SEQ ID NO: 50; or (e) a heavy chain variable region represented by SEQ ID NO: 53 and a light chain variable region represented by SEQ ID NO: 54; and
   a heavy chain constant region represented by one amino acid sequence selected from the group consisting of SEQ ID NOs: 5, 6, 7, and 8; and a light chain constant region represented by an amino acid sequence of SEQ ID NO: 10.

In this case, the antibody including the variable regions of (a) and (b) may be a humanized antibody, and the antibody including the variable regions of (c) to (d) may be a chimeric antibody.

In examples of the present invention, the anti-OX40L antibody or the antigen-binding fragment thereof may have physicochemical properties enough to show sufficient effects in the human body, and may have excellent thermal stability. For example, the anti-OX40L antibody or the antigen-binding fragment thereof may be melted at a temperature of more than 50°C, specifically 59°C or more, and may have a half-life of about two weeks or more in the human body.

In examples of the present invention, in case that the anti-OX40L antibody of the present invention includes a constant region, such antibody may include a constant region derived from IgG, IgA, IgD, IgE, IgM, or a combination thereof or a hybrid thereof.

As used herein, the term "combination" may mean that a polypeptide encoding a single chain immunoglobulin constant region of the same origin forms a bond with a single chain polypeptide of a different origin in case of forming a dimer or multimer. For example, it is possible to form a dimer or multimer from two or more constant regions selected from the group consisting of constant regions of IgG, IgA, IgD, IgE and IgM.

As used herein, the term "hybrid" may mean that a sequence corresponding to an immunoglobulin heavy chain constant region of two or more different origins exists in a single chain immunoglobulin heavy chain constant region. As an example, there may be a domain hybrid including one to four domains selected from the group consisting of CH1, CH2, CH3 and CH4 of IgG, IgA, IgD, IgE and IgM.

Meanwhile, it is possible to have the combination or hybridization of heavy chain constant regions of IgG1, IgG2, IgG3 and IgG4, which are subtypes of IgG. The combination or hybridization may be the same as described as above.

In examples of the present invention, the IgG1 heavy chain constant region may be an IgG1 heavy chain constant region represented by SEQ ID NO: 5; an IgG1 N297A heavy chain constant region may be a heavy chain constant region represented by SEQ ID NO: 6; an IgG4 heavy chain constant region may be an IgG4 heavy chain constant region represented by SEQ ID NO: 7; and an IgG4 S228P heavy chain constant region may be an IgG4 heavy chain constant region represented by SEQ ID NO: 8, but are not limited thereto.

In addition, in case that the anti-OX40L antibody specific to OX40L of the present invention includes a light chain constant region, the light chain constant region may be derived from lambda (λ) or kappa (κ) light chain. In case that the light chain constant region of the antibody is derived from the kappa (κ) light chain, this light chain constant region may be a kappa (κ) light chain constant region represented by SEQ ID NO: 10, but is not limited thereto.

In the present invention, the antibody may include both a mouse antibody produced from a mouse and a variant thereof obtained by substituting, adding and/or deleting a part of the amino acid sequence of a parent antibody in order to improve the affinity, immunity, etc., of the antibody therefrom. The variant may include, for example, a chimeric antibody, a humanized antibody, an affinity-optimized antibody, etc., but is not limited thereto. As used herein, the term "affinity-optimized antibody" may be a variant in which a part of the CDR sequence of a certain antibody is substituted, added, or deleted, and may refer to an antibody having improved binding affinity for an antigen while binding to the same antigen epitope as the certain antibody.

In the present invention, the variant may comprehensively refer to an antibody in which a part of the CDR amino acid sequence of a parent antibody is mutated (substituted, added or deleted) under the conditions of including the same CDR as the parent antibody or targeting the same epitope. This variant maybe appropriately adjusted by those skilled in the art in order to improve the affinity, immunity and the like of the antibody within the range of maintaining a binding capacity for the same epitope.

According to the present invention, the anti-OX40L antibody or the antigen-binding fragment thereof may include a sequence of the anti-OX40L antibody described herein as well as a biological equivalent thereof, within the range of specifically recognizing OX40L. For example, an amino acid sequence of the antibody may be further given a change, in order to more improve the binding affinity and/or other biological properties of the antibody. Such modification may include, for example, the deletion, insertion and/or substitution of an amino acid sequence residue of the antibody. Such amino acid mutation may be performed on the basis of the relative similarity of amino acid side chain substituents, e.g., hydrophobicity, hydrophilicity, charges, sizes, etc. According to the analysis of sizes, shapes and types of the amino acid side chain substituents, it may be understood that arginine, lysine and histidine are all positively charged residues; alanine, glycine and serine have similar sizes; and phenylalanine, tryptophan and tyrosine have similar shapes. Thus, based on these considerations, it might be understood that arginine, lysine and histidine; alanine, glycine and serine; and phenylalanine, tryptophan and tyrosine are biologically functional equivalents. For example, in examples of the present invention, the anti-OX40L antibody or the antigen-binding fragment thereof may include a change in conservative amino acids in one or more residues of the amino acid sequence represented by SEQ ID NOs herein, and the change in conservative amino acids may include a substitution as shown in Table 1 below.

**[Table 1] Substitution of conservative amino acids**

| | |
|---|---|
| Basic: | Arginine |
| | Lysine |
| | Histidine |
| Acidic: | Glutamic acid |
| | Aspartic acid |
| Polar: | Glutamine |
| | Asparagine |
| Hydrophobic: | Leucine |
| | Isoleucine |
| | Valine |
| Aromatic: | Phenylalanine |
| | Tryptophan |
| | Tyrosine |
| Small type: | Glycine |
| | Alanine |
| | Serine |
| | Threonine |
| | Methionine |

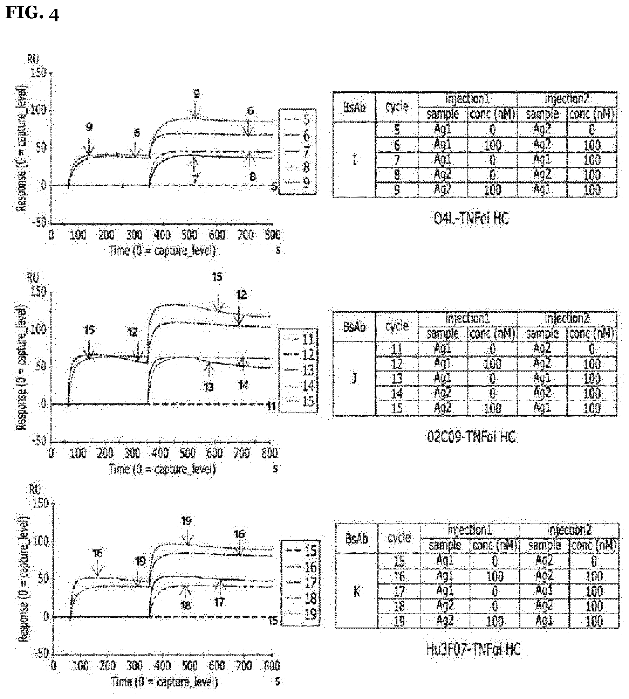

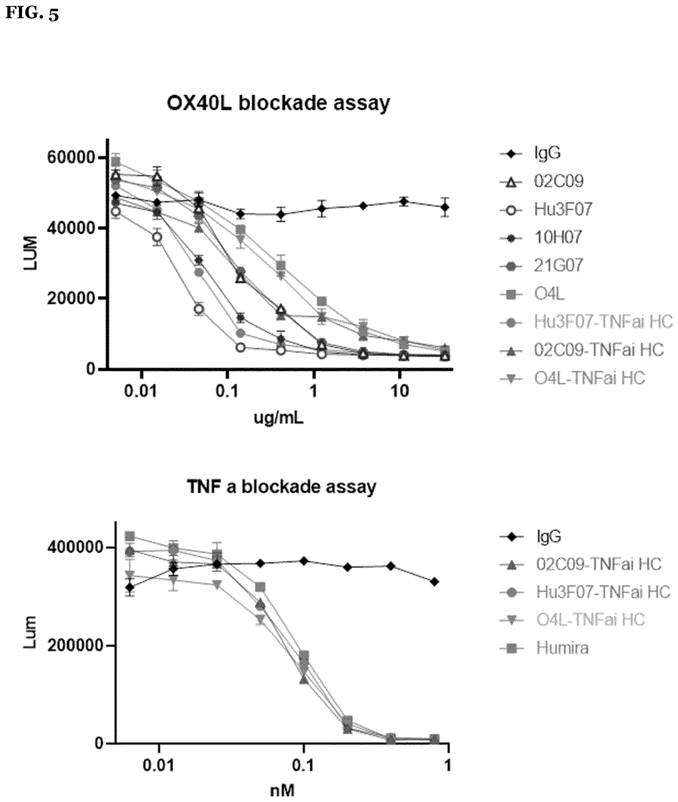

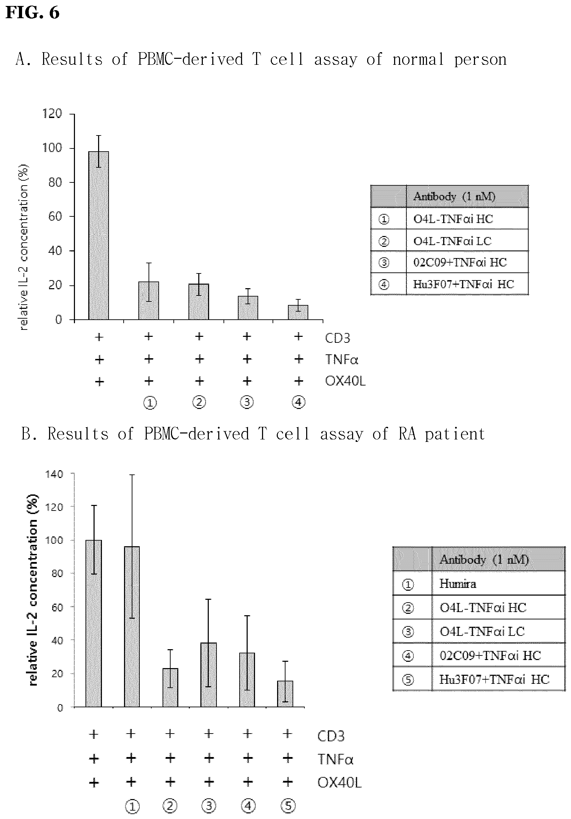

According to an example of the present invention, a novel antibody targeting OX40L was prepared. Antibodies specific to OX40L, such as 02C09, hu3F07, 10H07, and 21G07, were prepared from a library prepared from a mouse immunized with human OX40L (hOX40L) and a human library. It was confirmed that the antibodies specifically bind to OX40L with a high affinity of 0.2 to 0.7 nM (Table 32, FIG. 4), and show an in vitro OX40L inhibitory activity of 0.2 to 0.9 nM, which is remarkably more excellent than a control antibody (FIG. 5). In addition, the antibodies showed the result of blocking the immune activity of OX40L in T cells (FIG. 6). These results indicate that the anti-OX40L antibody specific to OX40L of the present invention efficiently blocks the binding to the OX40 receptor and suppresses OX40/OX40L signaling, so as to show a remarkably excellent effect on the treatment of autoimmune diseases and inflammatory diseases, thereby suggesting that such antibody may minimize side effects and selectively treat autoimmune diseases and inflammatory diseases while maintaining the homeostasis of the immune system.

According to the present invention, the anti-OX40L antibody or the antigen-binding fragment thereof may be effectively used in the treatment of autoimmune diseases or inflammatory diseases by suppressing the function of OX40L.

The human immune system may consist of two types: an innate immune system and an adaptive immune system, and autoimmune diseases may occur when the innate and adaptive immune systems are abnormally activated.

It is known that, when OX40L and the OX40 receptor bind to each other, immune cells involved in the innate and adaptive immune systems may become overactivated to cause various diseases.

Above OX40L may be simultaneously involved in the antigen-presenting cells (APC) of the innate immune system and the T helper cells of the adaptive immune system, and thus involved in the homeostasis of the immune system (Nature Reviews Rheumatology vol. 12, 74-76(2016), (Clinic Rev Allerg Immunol, 2016). In addition, since above OX40L is intensively distributed at a lesion site, unlike systemically distributed cytokines, the anti-OX40L antibody or the binding fragment thereof according to the present invention has a high possibility of binding to OX40L around the lesion.

Thus, the anti-OX40L antibody or the antigen-binding fragment thereof may bind to OX40L intensively distributed around the lesion, thereby maximizing a therapeutic effect on autoimmune diseases and inflammatory diseases, and reducing side effects to improve safety.

In addition, the adaptive immune system may show a slower reaction rate, but a higher persistence than that of the innate immune system, and thus may serve as a major factor in the treatment of autoimmune diseases caused by hyperactivity of the immune system. The anti-OX40L antibody or the antigen-binding fragment thereof according to the present invention may have a great advantage in that its suppression or inhibition of OX40L regulates not only innate immune cells, but also adaptive immune cells, on which conventional autoimmune therapeutic agents have failed to have an influence.

In other words, according to the present invention, the anti-OX40L antibody or the antigen-binding fragment thereof specifically binding to OX40L may effectively suppress and inhibit an interaction between OX40L and OX40, and thus may be effectively used in the treatment of autoimmune diseases and inflammatory diseases.

The present invention may provide a nucleic acid (polynucleotide) encoding the anti-OX40L antibody or the antigen-binding fragment thereof; an expression vector including the nucleic acid; and a transformant having the expression vector introduced thereinto.

As used herein, the term "nucleic acid" or "polynucleotide" may have a meaning comprehensively including DNA and RNA molecules. The nucleotide, which is a basic constituent unit of the nucleic acid molecule, may include a natural nucleotide as well as an analogue with a sugar or base site modified therein (Scheit, Nucleotide Analogs, John Wiley, NewYork(1980); Uhlman and Peyman, Chemical Reviews, (1990) 90:543-584).

According to the present invention, a sequence of the nucleic acid molecule encoding a light chain variable region and a heavy chain variable region may be modified, in which the modification may include the addition, deletion, or non-conservative or conservative substitution of the nucleotide.

The nucleic acid of the present invention may be interpreted to include a nucleotide sequence which shows substantial identity to the nucleotide sequence described above. In the present invention, the substantial identity may refer to a nucleotide sequence which shows at least 80% homology, specifically at least 90% homology, and more specifically at least 95% homology, in case that the above-described nucleotide sequence of the present invention and any other sequence are aligned to correspond to each other as much as possible and the aligned sequence is analyzed by using an algorithm commonly used in the art.

As used herein, the term "vector" or "expression vector," which serves as a means for expressing a target gene in a host cell, may include a plasmid vector; a cosmid vector; a virus vector such as a bacteriophage vector, an adenovirus vector, a retrovirus vector and an adeno-associated virus vector; and the like, and may be specifically the plasmid vector, but is not limited thereto.

In the vector of the present invention, the nucleic acid molecule encoding a light chain variable region and the nucleic acid molecule encoding a heavy chain variable region may be operatively linked to a promoter.

As used herein, the term "operatively linked" may refer to a functional linkage between a nucleic acid expression regulatory sequence (e.g., a promoter, a signal sequence or an array of transcriptional regulatory factor binding sites) and another nucleic acid sequence, whereby the regulatory sequence may regulate the transcription and/or decoding of above another nucleic acid sequence.

The recombinant vector system of the present invention may be constructed through various methods known in the art. For example, a detailed method thereof is disclosed in Sambrook et al., Molecular Cloning, A Laboratory Manual, Cold Spring Harbor Laboratory Press (2001), the contents of which are hereby incorporated by reference.

In the present invention, an expression vector, which includes a nucleic acid (polynucleotide) encoding the anti-OX40L antibody or the antigen-binding fragment thereof, may be a vector which may replicate and/or express the nucleic acid in eukaryotic or prokaryotic cells, including mammalian cells (for example, human, monkey, rabbit, rat, hamster, mouse cells, etc. ), plant cells, yeast cells, insect cells, or bacterial cells (for example, E. coli, etc.) without particular limitation. Specifically, the expression vector may be a vector which includes at least one selection marker and is operatively linked to an appropriate promoter so that the nucleic acid maybe expressed in a host cell, and more specifically may be a vector in which the nucleic acid is introduced into phage, plasmid, cosmid, mini-chromosome, virus, retroviral vector, etc.

The expression vector including the nucleic acid (polynucleotide) encoding the anti-OX40L antibody may be an expression vector which includes the nucleic acid encoding a heavy chain or a light chain of the anti-OX40L antibody, respectively, or may be an expression vector which includes the nucleic acid encoding a heavy chain or a light chain all.

In the present invention, a transformant having the expression vector introduced thereinto may include bacterial cells such as E. coli, streptomyces, salmonella typhimurium, etc., which are transformed by having the expression vector introduced thereinto; yeast cells; fungal cells such as Pichia pastoris, etc.; insect cells such as drosophila, spodoptera Sf9 cells, etc.; animal cells such as Chinese hamster ovary cells (CHO), SP2/0 (mouse myeloma), human lymphoblastoid, COS, NSO (mouse myeloma), 293T, bow melanoma cells, HT-1080, baby hamster kidney cells (BHK), human embryonic kidney cells (HEK), PERC.6 (human retinal cells), etc.; or plant cells, but is not particularly limited thereto. In examples of the present invention, HEK cells, etc., were used as a host cell.

As used herein, the term "introduction/transfer" may refer to a method for delivering a vector including a nucleic acid (polynucleotide) encoding an anti-OX40L antibody or an antigen-binding fragment thereof to a host cell. Such introduction may be carried out by several methods known in the art, such as a calcium phosphate-DNA coprecipitation method, a DEAE-dextran-mediated transfection method, a polybrene-mediated transfection method, an electric shock method, a microinjection method, a liposome fusion method, a lipofectamine and protoplast fusion method, and the like. In addition, transduction may refer to transferring a target object into cells by using viral particles through infection. In addition, the vector may be introduced into the host cell by gene bombardment, etc. In the present invention, the introduction may be used interchangeably with transformation.

The present invention may provide a method for preparing an anti-OX40L antibody or an antigen-binding fragment thereof.

According to the present invention, the anti-OX40L antibody or the antigen-binding fragment thereof may be easily prepared by a known technique for preparing monoclonal antibodies. For example, a method for preparing monoclonal antibodies may be performed by preparing a hybridoma through B lymphocytes obtained from an immunized animal (Koeher and Milstein, 1976, Nature, 256:495), or through a phage display technique, but is not limited thereto.

An antibody library using the phage display technique may be a method for expressing an antibody on the surface of phage by obtaining an antibody gene directly from B lymphocytes without preparing a hybridoma. By using the phage display technique, it is possible to overcome many existing difficulties associated with the production of monoclonal antibodies by B-cell immortalization. In general, the phage display technique may include the following steps of: 1) inserting a random sequence of oligonucleotide into a gene site corresponding to the N-terminus of coat protein pIII (or pIV) of phage; 2) expressing a fusion protein of a polypeptide encoded by the random sequence of oligonucleotide as well as a portion of the native coat protein; 3) treating a receptor material capable of binding to the polypeptide encoded by the oligonucleotide; 4) eluting the peptide-phage particles bound to the receptor by using a molecule with a low pH or binding competitiveness; 5) amplifying the phage eluted by panning in a host cell; 6) repeating the above method to obtain a desired amount; and 7) determining the sequence of an active peptide from the DNA sequences of the phage clones selected by panning.

In examples of the present invention, the method for preparing the anti-OX40L antibody or the antigen-binding fragment thereof according to the present invention may be performed by using a phage display technique. Those skilled in the art may easily perform each step of the preparation method of the present invention with reference to the phage display technique, for example, the method known in the thesis of Barbas et al. (METHODS: A Companion to Methods in Enzymology 2:119, 1991, and J. Virol. 2001 Jul;75(14):6692-9), Winter et al. (Ann. Rev.Immunol. 12:433, 1994), and the like. The phage, which may be used to construct an antibody library, may include, for example, fd, M13, f1, If1, Ike, Zj/Z, Ff, Xf, Pf1 or Pf3 phage as a filamentous phage, but is not limited thereto. In addition, vectors which may be used for expression of heterogeneous genes on the surface of the filamentous phage may include, for example, phage vectors such as fUSEs, fAFF1, fd-CAT1, fdtetDOG, or the like, or phagemid vectors such as pHEN1, pComb3, pComb8, pSEX, or the like, but are not limited thereto. In addition, helper phage which may be used to provide a wild-type coat protein required for successful reinfection of a recombinant phage for amplification may include, for example, M13K07, VSCM13, or the like, but is not limited thereto.

According to the present invention, a polynucleotide encoding a phage display clone may be easily isolated and sequenced by using a conventional procedure. As an example, it is possible to use an oligonucleotide primer designed to specifically amplify the heavy and light chain coding regions from hybridoma or phage template DNA in the art. Once the polynucleotide is isolated, the polynucleotide may be inserted into an expression vector, after which the expression vector may be introduced into an appropriate host cell so as to produce a desired monoclonal antibody from a transformed host cell (i.e., a transformant). Thus, the method for preparing the human monoclonal antibody of the present invention may be a method for preparing a human monoclonal antibody, including a step of amplifying an expression vector including a polynucleotide encoding a human monoclonal antibody, but is not limited thereto.

According to the present invention, the anti-OX40L antibody or the antigen-binding fragment thereof may be prepared by the known recombinant means or biochemical methods, and the antibody may be recovered from a culture fluid of transformants, in which an expression vector including a nucleic acid encoding the antibody in an appropriate host cell is introduced.

In examples of the present invention, the method for preparing (producing) the anti-OX40L antibody or the antigen-binding fragment thereof specifically binding to OX40L may include the steps of:
(a) producing an anti-OX40L antibody or an antigen-binding fragment thereof by culturing the transformant; and
(b) recovering the anti-OX40L antibody or the antigen-binding fragment thereof produced in above step (a).

In examples of the present invention, the anti-OX40L antibody or the antigen-binding fragment thereof may be isolated by a known isolation method, and may be appropriately isolated from a culture medium, for example, by conventional immunoglobulin purification procedures, such as protein A-sepharose, gel electrophoresis, dialysis or affinity chromatography, but is not limited thereto.

The present invention may provide a bispecific antibody which includes an anti-OX40L antibody or an antigen-binding fragment thereof; and an antibody or an antigen-binding fragment thereof specifically binding to tumor necrosis factor α (TNFα).

As used herein, the term "bispecific antibody" may refer to an antibody capable of binding to two different kinds of antigens (target proteins). Specifically, the bispecific antibody may not exist in nature, but may take on a form prepared by genetic engineering or any method.

The bispecific antibody of the present invention may be an antibody capable of binding to two different targets, and the bispecific antibody may bind to OX40L and TNFα.

The "bispecific antibody" of the present invention may be interchangeably used with "dual-targeted protein", "dual antibody" or "dual antibody protein."

An antibody or a binding fragment thereof specifically binding to OX40L, which is a component of the bispecific antibody of the present invention, may include an antibody or a binding fragment thereof capable of specifically binding to OX40L to block the OX40L/OX40L signaling pathway. In examples of the present invention, the antibody or the binding fragment thereof specifically binding to OX40L, which is a component of the bispecific antibody of the present invention, may be substantially the same anti-OX40L antibody or the binding fragment thereof according to the present invention as described above in the anti-OX40L antibody or the binding fragment thereof specifically binding to OX40L, if not contradictory to each other. In addition, in examples of the present invention, the antibody or the binding fragment thereof specifically binding to OX40L, which is a component of the bispecific antibody of the present invention, may be an antibody specifically binding to OX40L to block the OX40L/OX40L signaling pathway, which includes the antibody or the binding fragment thereof described in WO 2018083248, WO 2009141239, US 2017260279, WO 2006029879, or WO 2011073180.

The antibody or the binding fragment thereof specifically binding to OX40L, which is a component of the bispecific antibody of the present invention, may specifically bind to OX40L overexpressed in immune cells, and thus may not only concentrate the bispecific antibody of the present invention on the immune cells expressing TNFα, but also bind to TNFα, thereby providing the ability to reduce the activity of immune cells per se.

In addition, OX40L may be one of higher layer signals, which may be overexpressed in APC and may interact with an OX40 receptor expressed on T cells so as to induce the proliferation/differentiation/activation of immune cells and induce the secretion of various inflammatory cytokines, thereby activating innate immunity and adaptive immunity at the same time. The anti-OX40L antibody or the antigen-binding fragment thereof may inhibit OX40/OX40L signaling, thereby reducing immune responses which are excessively activated in patients with autoimmune diseases and inflammatory diseases.

In addition, the anti-TNFa antibody or the binding fragment thereof may also show an effect on patients with autoimmune diseases and inflammatory diseases, which show resistance to treatment for TNFα, which is a subsignal of the inflammatory responses.

As used herein, the term "a bispecific antibody including an antibody or an antigen-binding fragment thereof specifically binding to OX40L and an antibody or an antigen-binding fragment thereof specifically binding to TNFα" may include any bispecific protein without limitation, as long as the bispecific protein is capable of inhibiting two signaling pathways of OX40L and TNFα at the same time. An antibody or an antigen-binding fragment thereof specifically binding to TNFα, which constitutes the bispecific antibody, and an antibody or an antigen-binding fragment thereof specifically binding to OX40L may include both forms of a full-length antibody and an antibody fragment as described above in the anti-OX40L antibody or the binding fragment thereof.

As used herein, the term "inhibiting an interaction between OX40L and OX40" may mean that a bispecific antibody specifically binding to OX40L of the present invention binds to OX40L so as to inhibit an interaction between OX40L and OX40, so that the interaction between OX40L and OX40 is inhibited by the binding of the specific antibody and a structural change in OX40L is not caused by the binding of OX40L to OX40, thereby making it impossible to bring about hydrolyzation and OX40 signaling.

As used herein, the term "antibody specifically binding to TNFα" may include any antibody as long as the antibody specifically binds to TNFα as an antigen in a wide range of the body. In examples of the present invention, the antibody specifically binding to TNFα may be a therapeutic antibody targeting TNFα, including, but not limited to, the antibodies or the binding fragments thereof described in WO 1997029131, WO 2003045400, WO 2004050683, WO 1998011917, EP 1097945, WO 2001037874, US 2006024310, WO 2006125229, WO 2007056540, WO 1994006476, WO 2000059530, or WO 2001000229. In examples of the present invention, the antibody specifically binding to TNFα may be a therapeutic antibody called adalimumab (trade name Humira, abbvie), which may be stably used as approved by the US FDA, the European EMA and the like, but is not limited thereto. The antibody specifically binding to TNFα as above may be in the form of an IgG antibody, including, but not limited to, both forms of the full-length antibody and the antibody fragment as described above.

TNFα is a cytokine which regulates immune cells, acts as a pyrogen in the body to induce apoptosis by generating heat, and produces inflammatory cytokines, thereby causing autoimmune diseases and inflammatory diseases. TNFα is mainly secreted by activated macrophages, but is also secreted by other various immune cells including neurons. The most important role of TNFα may be the regulation of immune cells. Suppressing the excessively expressed TNFα may lead to suppressing autoimmune diseases and inflammatory diseases.

The bispecific antibody may bind to TNFα to suppress or inhibit an interaction between human TNFα and a TNFα receptor (TNFα Rc). Specifically, the bispecific antibody specific for TNFα, which is a component of the bispecific antibody, may bind to TNFα to suppress or inhibit the interaction between TNFα and the TNFα receptor, but is not limited thereto.

For the purpose of the present invention, the TNFα receptor may include any protein without limitation as long as the protein binds to TNFα of mammals, but may specifically refer to a protein binding to human TNFα.

By inhibiting the interaction between TNFα and the TNFα receptor through the bispecific antibody specific for TNFα or the binding fragment thereof according to the present invention, it is possible to suppress TNFα/TNFα receptor signaling caused by the binding of TNFα to the TNFα receptor. In case that TNFα and the TNFα receptor bind to each other in the immune system, TNFα/TNFα receptor signaling may be activated in immune cells, and thus regulate the differentiation of immune cells, etc., through a mechanism different from a mechanism of action of OX40L/OX40 signaling pathway, thereby providing a use as a therapeutic agent for various autoimmune diseases.

Thus, the bispecific antibody specific for OX40L and TNFα according to the present invention may show the ability to suppress hyperactive immune cells through different mechanisms, and thus may be used as a therapeutic agent capable of more excellently treating autoimmune diseases and inflammatory diseases.

Accordingly, the bispecific antibody or the antigen-binding fragment thereof specifically binding to OX40 and TNFα according to the present invention, which effectively suppresses OX40L/OX40 signaling and TNFα/TNFα receptor signaling, may effectively treat autoimmune diseases and inflammatory diseases and maximize a therapeutic effect while minimizing side effects.

In examples of the present invention, the anti-TNFa antibody or the antigen-binding fragment thereof specifically binding to TNFα may include:
a heavy chain variable region including heavy chain CDR1 represented by SEQ ID NO: 89; heavy chain CDR2 represented by SEQ ID NO: 90; and heavy chain CDR3 represented by SEQ ID NO: 91, and
a light chain variable region including light chain CDR1 represented by SEQ ID NO: 92; light chain CDR2 represented by SEQ ID NO: 93; and light chain CDR3 represented by SEQ ID NO: 94.

In examples of the present invention, the anti-TNFa antibody or the antigen-binding fragment thereof specifically binding to TNFα may include a heavy chain variable region represented by an amino acid sequence of SEQ ID NO: 35, and a light chain variable region represented by an amino acid sequence of SEQ ID NO: 36, and may specifically include the variable region of Humira.

In examples of the present invention, the form of the bispecific antibody is particularly limited, but may be provided in such a way that a binding fragment is linked to an antibody in the form of IgG by a linker. Specifically, in the case of the bispecific antibody of the present invention, an antibody or an antigen-binding fragment thereof specifically binding to OX40L, and an antibody or an antigen-binding fragment thereof specifically binding to TNFα may be linked to each other by a linker.

In examples of the present invention, the linker may be a peptide or non-peptide represented by a sequence of SEQ ID NO: 31 or SEQ ID NO: 32.

As used herein, the term "linker" may basically refer to a connection body which may connect two different fusion partners (for example, biological polymers, etc.) to each other by using a hydrogen bond, electrostatic interaction, van der Waals force, disulfide bond, salt bridge, hydrophobic interaction, covalent bond, etc., and specifically may have at least one cysteine which may participate in at least one disulfide bond under physiological conditions or other standard peptide conditions (for example, peptide purification conditions and peptide storage conditions), and may play a role of giving a certain sized gap between the fusion partners, or serve as a hinge to provide flexibility or rigidity to a fusion body, in addition to a role of simply connecting each of the fusion partners. The linker may be a non-peptide linker or a peptide linker, and may include any one directly connected by a peptide bond, a disulfide bond, etc.

In the present invention, the linker may be specifically a polypeptide capable of linking an antibody specifically binding to OX40L and an antibody specifically binding to TNFα to each other, and more specifically may be a peptide linker capable of linking an antibody specifically binding to TNFα with the C-terminus of the Fc region or the C-terminus of the light chain region of the antibody specifically binding to OX40L, and much more specifically may be a peptide linker consisting of an amino acid sequence in the form of repeated GGGGS motifs, but is not particularly limited thereto. The GGGGS motif may be repeated 1 to 10 times, and most specifically the peptide linker may consist of an amino acid sequence of SEQ ID NO: 31 in which the GGGGS motif is repeated 3 times or an amino acid sequence of SEQ ID NO: 32 in which the GGGGS motif is repeated 4 times, but is not limited thereto, and various linkers may be used within the range of being easily derived by those skilled in the art.

In the present invention, the term "non-peptide linker" may refer to a biocompatible linker in which two or more repeating units are bonded to each other, and the repeating units may be linked to each other through any covalent bond, not a peptide bond.

The non-peptide linker of the present invention may be a biodegradable polymer, a lipid polymer, chitins, hyaluronic acid, or a combination thereof, such as polyethylene glycol (PEG) homopolymer, polypropylene glycol homopolymer, ethylene glycol-propylene glycol copolymer, polyoxyethylated polyol, polyvinyl alcohol, polysaccharide, dextran, polyvinyl ethyl ether. Specifically, the non-peptide linker may be a polyethylene glycol homopolymer, and derivatives thereof already known in the art and derivatives which may be easily prepared at the technical level of the art may also be included in the scope of the present invention.

More specifically, the non-peptide linker may be a polyethylene glycol homopolymer having a molecular weight of 1 to 5 kDa, and most specifically may be a linker capable of connecting an antibody specifically binding to OX40L and an antibody specifically binding to TNFα at both termini of about 3.4 kDa in the form of bifunctional aldehyde. In particular, the non-peptide linker may be effective in minimizing nonspecific reactions in the case of having an effector of a reactive aldehyde group at both ends.

The site directly or indirectly linked through the linker may be an Fc portion, Fab', F(ab')₂, Fab, Fv, or the like, but is not particularly limited thereto. The bispecific antibody may take on a form in which all or a part (fragment) of the antibody specifically binding to OX40L and all or a part (fragment) of the antibody specifically binding to TNFα are connected, but is not particularly limited thereto.

In addition, the bispecific antibody may take on a form in which all or a part of a protein specifically binding to OX40L and all or a part of a heavy chain of an antibody specifically binding to TNFα are connected by a peptide linker; a form in which all or a part of a protein specifically binding to OX40L and all or a part of a light chain of an antibody specifically binding to TNFα are connected by a peptide linker; or a combination thereof.

In examples of the present invention, the bispecific antibody may take on a form in which an antibody specifically binding to OX40L in the form of immunoglobulin G (IgG) and an antibody specifically binding to TNFα in the form of a full-length antibody, Fab', F(ab')₂, Fab, Fv, rIgG, or scFv are connected by a linker.

In examples of the present invention, the bispecific antibody may take on a form in which an antibody specifically binding to TNFα in the form of immunoglobulin G (IgG) and an antibody specifically binding to OX40L in the form of a full-length antibody, Fab', F(ab')₂, Fab, Fv, rIgG, or scFv are connected by a linker, but is not limited thereto.

As used herein, the term "binding fragment" may include an antigen-binding form of an antibody, including fragments having antigen-binding ability, for example, Fab', F(ab')₂, Fab, Fv, rIgG and scFv. In particular, the term may include a single-chain variable fragment (scFv), and may include a bivalent or diabody, triabody, and tetrabody.

As used herein, the term "single-chain variable fragment (scFv)" may refer to a minimum antibody fragment having a complete antigen-recognizing and antigen-binding site, and include the VH and VL domains of an antibody, in which the domains may be present in a single polypeptide chain.

In examples of the present invention, the bispecific antibody may be the one, in which an anti-TNFa antibody or an antigen-binding fragment thereof specifically binding to TNFα is linked to at least one terminus of the light and heavy chains of an anti-OX40L antibody.

In examples of the present invention, the bispecific antibody may be the one in which the anti-TNFa antibody or the antigen-binding fragment thereof is linked to at least one C-terminus of the light and heavy chains of the anti-OX40L antibody. Specifically, in the case of the bispecific antibody, the anti-TNFa antibody or the antigen-binding fragment thereof may be linked to at least one C-terminus of the light and heavy chains of the anti-OX40L antibody through a linker. For example, in the case of the bispecific antibody, the anti-TNFa antibody or the antigen-binding fragment thereof may be linked to at least one C-terminus of the light and heavy chains of the anti-OX40L antibody through a linker having an amino acid sequence represented by SEQ ID NO: 31 or SEQ ID NO: 32, but is not particularly limited thereto.

In examples of the present invention, the bispecific antibody may be the one in which an anti-OX40L antibody or an antigen-binding fragment thereof specifically binding to OX40L is linked to at least one terminus of the light and heavy chains of an anti-TNFa antibody.

In examples of the present invention, the bispecific antibody may be the one in which the anti-OX40L antibody or the antigen-binding fragment thereof is linked to at least one C-terminus of the light and heavy chains of the anti-TNFa antibody. Specifically, the bispecific antibody may be the one in which the anti-OX40L antibody or the antigen-binding fragment thereof is linked to at least one C-terminus of the light and heavy chains of the anti-TNFa antibody by a linker. For example, the anti-OX40L antibody or the antigen-binding fragment thereof may be linked to at least one C-terminus of the light and heavy chains of the anti-TNFa antibody through a linker having the amino acid sequence represented by SEQ ID NO: 31, but is not particularly limited thereto.

In examples of the present invention, the bispecific antibody may be the one in which a binding fragment of an anti-OX40L antibody specifically binding to OX40L and a binding fragment of an anti-TNFa antibody are linked through a linker. The linker may be a linker having an amino acid sequence represented by SEQ ID NO: 31 or SEQ ID NO: 32.

In examples of the present invention, the bispecific antibody may be the one, in which the followings are linked to each other through a linker:
a) an anti-OX40L antibody or a binding fragment thereof specifically binding to OX40L including: a heavy chain variable region including heavy chain CDR1 represented by one amino acid sequence selected from the group consisting of SEQ ID NOs: 12, 13 and 14; heavy chain CDR2 represented by one amino acid sequence selected from the group consisting of SEQ ID NOs: 15, 16, 17 and 18; and heavy chain CDR3 represented by one amino acid sequence selected from the group consisting of SEQ ID NOs: 19, 20, 21 and 22, and a light chain variable region including light chain CDR1 represented by one amino acid sequence selected from the group consisting of SEQ ID NOs: 23 and 24; light chain CDR2 represented by one amino acid sequence selected from the group consisting of SEQ ID NOs: 25 and 26; and light chain CDR3 represented by one amino acid sequence selected from the group consisting of SEQ ID NO: 27, 28, 29 and 30; and
b) an anti-TNFa antibody or a binding fragment thereof specifically binding to TNFα including: a heavy chain variable region including heavy chain CDR1 of an amino acid sequence represented by SEQ ID NO: 89; heavy chain CDR2 of an amino acid sequence represented by SEQ ID NO: 90; and heavy chain CDR3 of an amino acid sequence represented by SEQ ID NO: 91, and a light chain variable region including light chain CDR1 of an amino acid sequence represented by SEQ ID NO: 92; light chain CDR2 of an amino acid sequence represented by SEQ ID NO: 93; and light chain CDR3 of an amino acid sequence represented by SEQ ID NO: 94.

The linker may be a linker having an amino acid sequence represented by SEQ ID NO: 31 or SEQ ID NO: 32.

In examples of the present invention, the bispecific antibody may be the one, in which the followings are linked to each other through a linker:
a)
   (i) an anti-OX40L antibody or a binding fragment thereof including: a heavy chain variable region including heavy chain CDR1 represented by SEQ ID NO: 12; heavy chain CDR2 represented by SEQ ID NO: 15; and heavy chain CDR3 represented by SEQ ID NO: 19, and a light chain variable region including heavy chain CDR1 represented by SEQ ID NO: 23; heavy chain CDR2 represented by SEQ ID NO: 25; and light chain CDR3 represented by SEQ ID NO: 27;
   (ii) an anti-OX40L antibody or an antigen-binding fragment thereof including: a heavy chain variable region including heavy chain CDR1 represented by SEQ ID NO: 13; heavy chain CDR2 represented by SEQ ID NO: 16; and heavy chain CDR3 represented by SEQ ID NO: 20, and a light chain variable region including light chain CDR1 represented by SEQ ID NO: 24; light chain CDR2 represented by SEQ ID NO: 26; and light chain CDR3 represented by SEQ ID NO: 28;
   (iii) an anti-OX40L antibody or an antigen-binding fragment thereof including: a heavy chain variable region including heavy chain CDR1 represented by SEQ ID NO: 13; heavy chain CDR2 represented by SEQ ID NO: 17; and heavy chain CDR3 represented by SEQ ID NO: 21, and a light chain variable region including light chain CDR1 represented by SEQ ID NO: 24; light chain CDR2 represented by SEQ ID NO: 26; and light chain CDR3 represented by SEQ ID NO: 29; or
   (iv) an anti-OX40L antibody or an antigen-binding fragment thereof including: a heavy chain variable region including heavy chain CDR1 represented by SEQ ID NO: 14; heavy chain CDR2 represented by SEQ ID NO: 18; and heavy chain CDR3 represented by SEQ ID NO: 22, and a light chain variable region including light chain CDR1 represented by SEQ ID NO: 24; light chain CDR2 represented by SEQ ID NO: 26; and light chain CDR3 represented by SEQ ID NO: 30; and
b) an anti-TNFa antibody or a binding fragment thereof specifically binding to TNFα including: a heavy chain variable region including heavy chain CDR1 of an amino acid sequence represented by SEQ ID NO: 89; heavy chain CDR2 of an amino acid sequence represented by SEQ ID NO: 90; and heavy chain CDR3 of an amino acid sequence represented by SEQ ID NO: 91, and a light chain variable region including light chain CDR1 of an amino acid sequence represented by SEQ ID NO: 92; light chain CDR2 of an amino acid sequence represented by SEQ ID NO: 93; and light chain CDR3 of an amino acid sequence represented by SEQ ID NO: 94.

The linker may be a linker having an amino acid sequence represented by SEQ ID NO: 31 or SEQ ID NO: 32.

In examples of the present invention, the bispecific antibody may be the one, in which:
a) an anti-OX40L antibody or a binding fragment thereof including: a heavy chain variable region represented by one amino acid sequence selected from the group consisting of SEQ ID NOs: 33, 37, 41, 45, 49 and 53; and a light chain variable region represented by one amino acid sequence selected from the group consisting of SEQ ID Nos: 34, 38, 42, 46, 50 and 54; and
b) an anti-TNFa antibody or an antigen-binding fragment thereof including a heavy chain variable region represented by SEQ ID NO: 35 and a light chain variable region represented by SEQ ID NO: 36
   are linked to each other through a linker.

The linker may be a linker having an amino acid sequence represented by SEQ ID NO: 31 or SEQ ID NO: 32.

In examples of the present invention, the bispecific antibody may be the one, in which:
a) an anti-OX40L antibody or a binding fragment thereof including: (a) a heavy chain variable region represented by SEQ ID NO: 37 and a light chain variable region represented by SEQ ID NO: 38; (b) a heavy chain variable region represented by SEQ ID NO: 41 and a light chain variable region represented by SEQ ID NO: 42; (c) a heavy chain variable region represented by SEQ ID NO: 45 and a light chain variable region represented by SEQ ID NO: 46; (d) a heavy chain variable region represented by SEQ ID NO: 49 and a light chain variable region represented by SEQ ID NO: 50; (e) a heavy chain variable region represented by SEQ ID NO: 53 and a light chain variable region represented by SEQ ID NO: 54; or (f) a heavy chain variable region having an amino acid sequence represented by SEQ ID NO: 33 and a light chain variable region having an amino acid sequence represented by SEQ ID NO: 34 and
b) an anti-TNFa antibody or an antigen-binding fragment thereof including a heavy chain variable region represented by SEQ ID NO: 35 and a light chain variable region represented by SEQ ID NO: 36
   are linked to each other through a linker.

The linker may be a linker having an amino acid sequence represented by SEQ ID NO: 31 or SEQ ID NO: 32.

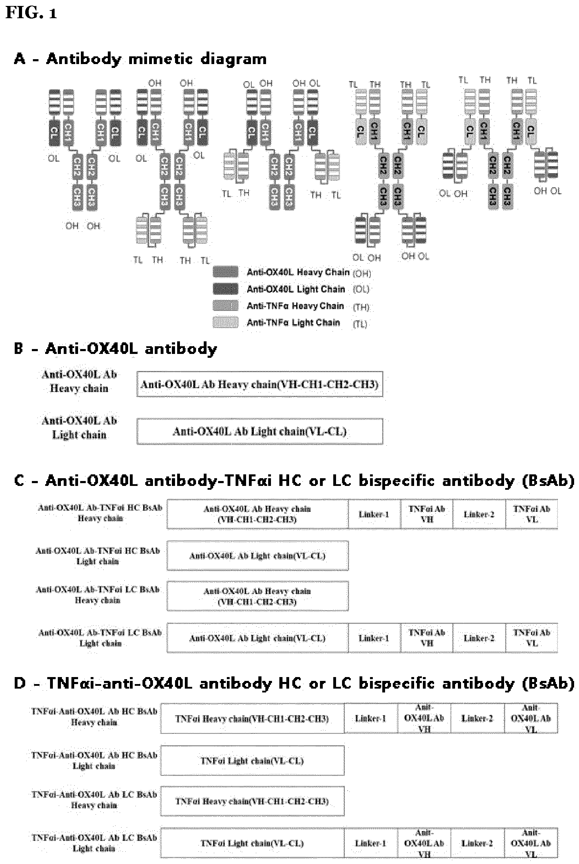

For example, a structure of the bispecific antibody may have a structure in the same form as shown in Fig. 1.

As another example, the bispecific antibody may have a structure in which an antigen-binding fragment of an anti-OX40L antibody and an antigen-binding fragment of an anti-TNFa antibody are linked to each other.

In case that the bispecific antibody of the present invention includes a constant region, the bispecific antibody may specifically include a heavy chain constant region represented by one amino acid sequence selected from the group consisting of SEQ ID NOs: 5, 6, 7 and 8; and all or a part of a light chain constant region represented by an amino acid sequence of SEQ ID NO: 10, but is not limited thereto. In case that the bispecific antibody includes a constant region means the case that an anti-OX40L antibody or a binding fragment thereof specifically binding to OX40L or an anti-TNFa antibody or a binding fragment thereof specifically binding to TNFα, which constitute the bispecific antibody, include all or a part of a constant region.

The bispecific antibody of the present invention may have physicochemical properties enough to exhibit sufficient effects in the human body, and may have excellent thermal stability. For example, the bispecific antibody may be melted at a temperature of more than 50°C, specifically 59°C or more, and may maintain its binding for a long period of time, and may have a half-life of about 2 weeks or more in the human body.

In other words, a bispecific antibody including an antibody or an antigen-binding fragment thereof specifically binding to TNFα and an antibody or an antigen-binding fragment thereof specifically binding to OX40 according to the present invention may have a strong affinity for human-derived TNFα and OX40L so as to effectively inhibit the binding of OX40L-expressing cells (e.g., immune cells) to OX40 and inhibit inflammatory responses by TNFα binding to the TNF receptor, thereby providing a more remarkable therapeutic effect in the treatment of diseases such as autoimmune diseases.

In the bispecific antibody of the present invention, an antibody or an antigen-binding fragment thereof specifically binding to TNFα and an antibody or an antigen-binding fragment thereof specifically binding to OX40L may maintain a specific binding, respectively and in particular, may simultaneously suppress two targets (antigens) without decreasing affinity for each of the targets, so as to simultaneously suppress two signals, and thus may be more effective than suppressing by binding to a single target.

In examples of the present invention, an OX40L and TNFα bispecific antibody specifically binding to OX40L and TNFα was prepared by inserting a nucleic acid (polynucleotide) encoding the bispecific antibody of the present invention into a vector and introducing the same into an animal cell to express and isolate an OX40L- and TNFα-binding bispecific antibody.

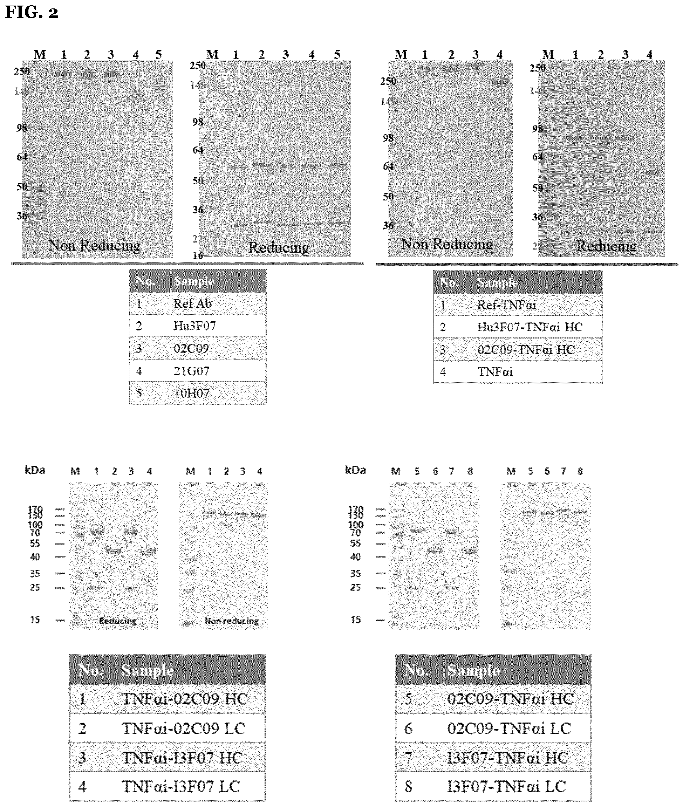

The bispecific antibody molecule may have a structure in which an OX40L IgG antibody molecule and a TNFa-binding scFv are connected by a linker or a structure in which a TNFα IgG antibody molecule and an OX40L-binding scFv are connected by a linker (FIG. 1). The OX40L- and TNFa-binding bispecific antibody introduced and expressed in the animal cell was isolated, and the expression and purity thereof were confirmed by SDS-PAGE (FIG. 2).

In addition, as a result of analyzing the binding assay of the bispecific antibody for OX40L and TNFα with regard to OX40L and TNFα through an enzyme-linked immunosorbent assay (ELISA), it was confirmed that the OX40L- and TNFa-binding bispecific antibody specifically binds to OX40L and TNFα, which are targets of the bispecific antibody (Table 32).

In examples of the present invention, the bispecific antibody may bind to human OX40L with a K_{D} of 3 × 10⁻⁹ M or less. Specifically, the anti-OX40L antibody or the antigen-binding fragment thereof may bind with a K_{D} of 1.5 × 10⁻⁹ M, 1.3 × 10⁻⁹ M, or 1 × 10⁻⁹ M or less, and may bind to human TNFα with K_{D} of 1 × 10⁻⁹ M or less. Specifically, as a result of measuring the equilibrium dissociation constant (K_{D}) values for OX40L and TNFα, which are antigens of the bispecific antibody, through the Biacore analysis method, it was confirmed that the bispecific antibody has a K_{D} value of 0.4 to 0.5 nM for human OX40L and a K_{D} value of 0.2 to 0.5 nM for human TNFα (Table 32, FIG. 4). In addition, it was confirmed that an in vitro OX40L inhibitory ability is 0.2 to 1.3 nM and remarkably more excellent than that of a control antibody and an in vitro TNFα inhibitory ability is 0.05 to 0.07 nM and excellent (Table 35, FIG. 5). In addition, the bispecific antibody showed the result of blocking the immune activity in T cells (FIG. 6).

Thus, the bispecific antibody of the present invention may effectively treat diseases associated with the target by simultaneously binding to OX40L and TNFα while maintaining binding force for each antigen.

In addition, it was confirmed for the bispecific antibody of the present invention through an experiment on in vitro blockade assay capable of simultaneously binding OX40L and TNFα that each of the signaling pathways by binding of immune cells to OX40L and human OX40 and binding of TNFα and the TNFα receptor is effectively suppressed through treatment with the bispecific antibody (Table 35 and FIG. 5). These results show that the bispecific antibody specific for OX40L and TNFα of the present invention efficiently blocks the binding of OX40 and the TNFα receptor, which are respective receptors, so as to provide an effect of alleviating the overactive immune system, and also simultaneously binds to OX40L and TNFα so as to effectively treat diseases associated with the target.

The present invention may provide a nucleic acid (polynucleotide) encoding a bispecific antibody specifically binding to the OX40L and TNFα, an expression vector including the nucleic acid (polynucleotide), and a transformant having the expression vector introduced thereinto.

In the present invention, the nucleic acid (polynucleotide) associated with the bispecific antibody specifically binding to OX40L and TNFα, the expression vector, the transformant, and the introduction are the same as described above, if not contradictory to each other.

The present invention may provide a method for preparing a bispecific antibody specifically binding to OX40L and TNFα.

Specifically, the preparation method may provide an antibody bispecifically binding to OX40L and TNFα, the method including the steps of: (a) producing a bispecific antibody by culturing a transformant into which an expression vector including a nucleic acid (polynucleotide) encoding a bispecific antibody specifically binding to OX40L and TNFα is introduced; and (b) recovering the bispecific antibody produced in above step (a).

A method for preparing the bispecific antibody specifically binding to OX40L and TNFα of the present invention may be applied substantially the same as the preparation method described in the anti-OX40L antibody or the antigen-binding fragment thereof, if not contradictory to each other.

The present invention may provide a pharmaceutical composition for preventing or treating autoimmune diseases or inflammatory diseases, including an anti-OX40L antibody or an antigen-binding fragment thereof, or the bispecific antibody specifically binding to OX40L and TNFα.

The pharmaceutical composition may further include a pharmaceutically acceptable carrier.

As used herein, the term "pharmaceutically acceptable carrier" may refer to a carrier or a diluent, which neither irritates organisms nor inhibits the biological activity and properties of an injected compound. In the composition formulated into a liquid solution, the pharmaceutically acceptable carrier used may include saline solution, sterilized water, Ringer's solution, buffered saline, albumin injection solution, dextrose solution, maltodextrin solution, glycerol, ethanol, which are suitable for sterilization and the living body, and a mixture of at least one component thereof, and other conventional additives such as antioxidants, buffer solutions, bacteriostatic agents, etc., may be added thereto, if needed. Also, such pharmaceutical composition may be formulated into injectable dosage forms such as aqueous solutions, suspensions, emulsions, etc., pills, capsules, granules or tablets in such a way that diluents, dispersing agents, surfactants, binders and lubricants are additionally added thereto.

The anti-OX40L antibody or the antigen-binding fragment thereof, or the bispecific antibody specifically binding to OX40L and TNFα may bind to OX40L so as to inhibit the binding to OX40L receptor, and thus may be involved in suppressing hyperactive immune cells. The OX40L/OX40 receptor is the same as described above.

Furthermore, the bispecific antibody may bind to TNFα in addition to OX40L to inhibit an interaction between TNFα and the TNFα receptor, thereby regulating the differentiation of immune cells, etc., through a mechanism different from a mechanism of action of OX40L/OX40 signaling pathway, and may be also involved in suppressing various autoimmune diseases.

Thus, the pharmaceutical composition of the present invention may remarkably and effectively prevent or treat autoimmune diseases or inflammatory diseases and may increase safety while minimizing side effects.

In the present invention, autoimmune diseases or inflammatory diseases may include rheumatoid arthritis. Rheumatoid arthritis may be an disease which is classified as an inflammatory disease or an autoimmune disease. As used herein, the terms "autoimmune disease", "inflammatory disease" or "autoimmune disease and inflammatory disease" may include rheumatoid arthritis.

As used herein, the term "prevention" may refer to suppressing or delaying the occurrence of a disease, and suppressing or delaying the reoccurrence of the disease in a subject whose disease has been treated.

As used herein, the term "treatment" may refer to all the acts, in which the symptoms of autoimmune diseases get better or take a favorable turn by administering the composition.

The present invention may provide a method for preventing or treating autoimmune diseases or inflammatory diseases, by using the anti-OX40L antibody or the antigen-binding fragment thereof, or the bispecific antibody specifically binding to OX40L and TNFα.

The present invention may provide a method for preventing or treating autoimmune diseases or inflammatory diseases by using a pharmaceutical composition including the anti-OX40L antibody or the antigen-binding fragment thereof, or the bispecific antibody specifically binding to OX40L and TNFα.

The method for preventing or treating autoimmune diseases or inflammatory diseases may include a step of administering the anti-OX40L antibody or the antigen-binding fragment thereof, or the bispecific antibody specifically binding to OX40L and TNFα into an individual.

The method for preventing or treating autoimmune diseases or inflammatory diseases may include a step of administering a pharmaceutical composition including the anti-OX40L antibody or the antigen-binding fragment thereof, or the bispecific antibody specifically binding to OX40L and TNFα into an individual.

The individual may be an individual having developed or suspected of developing autoimmune diseases or inflammatory diseases, and specifically may include mammals, birds, etc., such as cows, pigs, sheep, chickens, dogs, humans, etc., but is not particularly limited thereto.

The pharmaceutical composition may be in various oral or parenteral dosage forms. In case of formulating a preparation, the preparation may be prepared by using diluents or excipients such as fillers, extenders, binders, humectants, disintegrants, surfactants, etc., which are generally used. A solid preparation for oral administration may include tablets, pills, powders, granules, capsules, etc., and this solid preparation may be prepared by mixing at least one excipient, for example, starch, calcium carbonate, sucrose, lactose, gelatin, etc., in at least one compound. In addition, lubricants such as magnesium stearate, talc, etc., may be used in addition to simple excipients. A liquid preparation for oral administration may include suspensions, liquids for internal use, emulsions, syrups, etc., but may also include various excipients, for example, humectants, sweetening agents, flavoring agents, preservatives, etc. in addition to water and liquid paraffin, which are the frequently used simple diluents. A preparation for parenteral administration may include sterile aqueous solutions, non-aqueous solvents, suspensions, emulsions, lyophilized preparations and suppositories. The non-aqueous solvent and the suspending agent may include propylene glycol, polyethylene glycol, vegetable oil like olive oil, injectable ester like ethyl oleate, etc. A base of the suppository may include witepsol, macrogol, tween 61, cacao butter, laurinum, glycerogelatin, etc.

The pharmaceutical composition may have any one formulation selected from the group consisting of tablets, pills, powders, granules, capsules, suspensions, liquids for internal use, emulsions, syrups, sterile solutions, non-aqueous solvents, suspending agents, freeze-dried preparations, and suppositories.

According to the present invention, the anti-OX40L antibody or the antigen-binding fragment thereof, or the bispecific antibody specifically binding to OX40L and TNFα, or the pharmaceutical composition may be administered in a pharmaceutically effective amount. As used herein, the term "pharmaceutically effective amount" may refer to an amount enough to treat a disease at a reasonable benefit/risk ratio applicable to medical treatment, and a level of effective dose may be determined according to factors including an individual's type, severity, age, gender, a type of cancer, activity of a drug, sensitivity to the drug, an administration time, an administration route and excretion rate, a treatment period and a concurrently used drug, as well as other factors well known in a medical field. The composition of the present invention may be administered as an individual therapeutic agent or in combination with other therapeutic agents, and may be administered sequentially or simultaneously with a conventional therapeutic agent. And, the composition may be administered in a single or multiple manner. Considering all the above factors, it is important to carry out an administration by an amount, in which the maximum effect may be achieved by the minimum amount without a side effect, and the amount may be determined by those skilled in the art.

In this case, the composition may be administered in a pharmaceutically effective amount in a single or multiple manner. In this case, the composition may be administered in the form of liquid, powder, aerosol, capsule, enteric-coated tablet or capsule, or suppository. The route of administration may include intraperitoneal administration, intravenous administration, intramuscular administration, subcutaneous administration, endothelial administration, oral administration, topical administration, intranasal administration, intrapulmonary administration, rectal administration, etc., but is not limited thereto. However, when orally administered, peptide is digested. Thus, in case of the oral composition, an active agent thereof needs to be coated or formulated to be protected from degradation in the stomach. In addition, the pharmaceutical composition may be administered by any device capable of moving an active substance to the target cell.

Furthermore, the present invention may provide a use of the anti-OX40L antibody or the antigen-binding fragment thereof, or the bispecific antibody in the manufacture of a medicament for preventing or treating autoimmune diseases or inflammatory diseases.

The present invention may provide a use of a pharmaceutical composition including the anti-OX₄oL antibody or the antigen-binding fragment thereof, or the bispecific antibody in the manufacture of a medicament for preventing or treating autoimmune diseases or inflammatory diseases.

The present invention may provide a use of the anti-OX₄oL antibody or the antigen-binding fragment thereof, or the bispecific antibody for preventing or treating autoimmune diseases or inflammatory diseases.

The present invention may provide a use of a pharmaceutical composition including the anti-OX₄oL antibody or the antigen-binding fragment thereof, or the bispecific antibody for preventing or treating autoimmune diseases or inflammatory diseases.

The anti-OX₄oL antibody or the antigen-binding fragment thereof, or the bispecific antibody specifically binding to OX40L and TNFα, the pharmaceutical composition, autoimmune diseases or inflammatory diseases, prevention or treatment may be the same as described above, if not contradictory to each other.

The present invention may provide a diagnostic composition which includes the anti-OX₄oL antibody or the antigen-binding fragment thereof, or the bispecific antibody specifically binding to OX₄oL and TNFα and detects OX₄oL protein in a biological sample isolated from an individual suspected of having an autoimmune disease or an inflammatory disease through an antigen-antibody reaction.

In examples of the present invention, the diagnostic composition may diagnose the occurrence of autoimmune diseases or inflammatory diseases.

The anti-OX₄oL antibody or the antigen-binding fragment thereof, or the bispecific antibody specifically binding to OX₄oL and TNFα, autoimmune diseases and inflammatory diseases may be the same as described above, if not contradictory to each other.

As used herein, the term "diagnosis" may refer to identifying the presence or properties of a pathological condition. For the purpose of the present invention, the diagnosis is to confirm whether an autoimmune disease or an inflammatory disease has occurred or not.

According to the present invention, in the method for preventing or treating autoimmune diseases or inflammatory diseases, the anti-OX₄oL antibody or the antigen-binding fragment thereof or the diagnostic composition may be used to determine autoimmune diseases or inflammatory diseases by measuring a level of OX40L protein in a sample isolated from an individual suspected of having an autoimmune disease or an inflammatory disease through the anti-OX₄oL antibody or the antigen-binding fragment thereof, or the bispecific antibody specifically binding to OX₄oL and TNFα according to the present invention, and comparing the measured level of OX₄oL protein with that of a control sample of a normal person or a patient.

For this purpose, the methods for measuring a level of protein may include Western blot, Enzyme Linked Immunosorbent Assay (ELISA), radioimmunological assay (otA: badioimmunoassay), badioimmunodiffusion, Ouchterlony immunodiiffusion, rocket immunoelectrophoresis, immunohistochemical staining, immunoprecipitation assay, complement fixation assay, FACS and protein chip, etc., but are not limited thereto. Through the above-described analysis methods, the level of OX₄oL protein in an individual suspected of having an autoimmune disease may be compared with that of a normal control group, thereby diagnosing the occurrence of autoimmune disease in a suspected patient.

According to the present invention, the composition for diagnosing autoimmune diseases or inflammatory diseases may further include, without limitation, those known in the art as necessary to perform a method for measuring the level of protein in addition to the antibody of the present invention.

In case that the diagnostic composition of the present invention includes the bispecific antibody specifically binding to OX₄oL and TNFα, the diagnostic composition may be a composition for detecting the TNFα protein in a biological sample isolated from an individual suspected of having an autoimmune disease or an inflammatory disease through an antigen-antibody reaction. Specifically, in case that the diagnostic composition of the present invention includes the bispecific antibody specifically binding to OX₄oL and TNFα, the diagnostic composition may be a composition for detecting at least one of OX₄oL protein and TNFα protein in a biological sample isolated from an individual suspected of having an autoimmune disease or an inflammatory disease through an antigen-antibody reaction.

The anti-OX₄oL antibody or the antigen-binding fragment thereof, or the bispecific antibody specifically binding to OX₄oL and TNFα, or the diagnostic composition may be used to determine autoimmune diseases or inflammatory diseases by measuring at least one level of OX₄oL protein and TNFα protein in a sample isolated from an individual suspected of having an autoimmune disease or an inflammatory disease.

The method for measuring a level of protein may be the same as described above, if not contradictory to each other.

The present invention may provide a method for diagnosing autoimmune diseases or inflammatory diseases or a method for providing information on diagnosis by using the anti-OX₄oL antibody or the antigen-binding fragment thereof, the bispecific antibody specifically binding to OX₄oL and TNFα, or the diagnostic composition thereof. Specifically, the present invention may provide a method for diagnosing autoimmune diseases or inflammatory diseases or method for providing information on diagnosis of autoimmune diseases or inflammatory diseases, the method including the steps of: (a) measuring a level of OX₄oL protein in a sample isolated from an individual suspected of having an autoimmune disease or an inflammatory disease by using the anti-OX₄oL antibody or the antigen-binding fragment thereof, or the bispecific antibody specifically binding to OX₄oL and TNFα; and (b) determining autoimmune diseases or inflammatory diseases by using the level of OX₄oL protein measured in above step (a).

For example, the step of determining autoimmune diseases or inflammatory diseases by using the measured level of OX₄oL protein may be performed by comparing the measured level of OX₄oL protein with that of a normal person and/or a patient.

The present invention may provide a method for diagnosing autoimmune diseases or inflammatory diseases or a method for providing information on diagnosis of autoimmune diseases or inflammatory diseases, the method including the steps of: (a) measuring a level of at least one of OX₄oL protein and TNFα protein in a sample isolated from an individual suspected of having an autoimmune disease by using the bispecific antibody specifically binding to OX40L and TNFα; and (b) determining autoimmune diseases or inflammatory diseases by using the level of at least one of OX₄oL protein and TNFα protein as measured in above step (a).

For example, the step of determining autoimmune diseases or inflammatory diseases by using the measured level of at least one of OX₄oL protein and TNFα protein may be performed by comparing the measured level of at least one of OX₄oL protein and TNFα protein with that of at least one of OX₄oL protein and TNFα protein in a normal person and/or a patient.

The present invention may provide a kit for providing information on diagnosis of autoimmune diseases or inflammatory diseases, including the anti-OX40L antibody or the antigen-binding fragment thereof, or the bispecific antibody specifically binding to OX₄oL and TNFα.

The anti-OX₄oL antibody or the antigen-binding fragment thereof, or the bispecific antibody specifically binding to OX₄oL and TNFα, autoimmune diseases, inflammatory diseases, individual, diagnosis, and a step (method) of measuring a level of protein may be the same as described above, if not contradictory to each other.

As used herein, the term "sample" may include samples such as whole blood, serum, blood, plasma, saliva, urine, sputum, lymph, cerebrospinal fluid, and intercellular fluid, which show a difference in the expression level of OX₄oL in patients with autoimmune diseases, but is not limited thereto.

### Advantageous Effects

According to the present invention, an anti-OX₄oL antibody or an antigen-binding fragment thereof specifically binds to OX₄oL so as to effectively inhibit the binding of receptors, and is also excellent in immunosuppressive ability, thereby providing a remarkably excellent effect on the field of the treatment and diagnosis of autoimmune diseases and inflammatory diseases.

Furthermore, a bispecific antibody specifically binding to above OX₄oL and TNFα shows strong affinity not only for OX₄oL, but also for TNFα, maintains binding in the body for a long period of time, and thus is excellent in immunosuppressive ability, thereby showing a remarkably excellent effect on the field of the treatment and diagnosis of autoimmune diseases and inflammatory diseases.

### Brief Description of the Drawings

FIG. 1 shows the structure of an anti-OX₄oL antibody and a bispecific antibody capable of simultaneously binding to OX₄oL and TNFα. In FIG. 1, CH₁, CH₂ and CH3 represent a constant region of a heavy chain, CL represents a constant region of a light chain, and a part marked with stripes (or hatches) represents a variable region of each chain (CDR region (white) and framework region (colored)).
FIG. 2 shows the results of confirming an anti-OX₄oL antibody and a bispecific antibody capable of simultaneously binding to OX₄oL and TNFα with SDS-PAGE after producing the same.
FIG. 3 shows the results of epitope mapping of OX₄oL antigen for an anti-OX₄oL antibody through analysis with HDX-MS.
FIG. 4 shows the results of measuring whether or not a bispecific antibody may simultaneously bind to antigens such as OX₄oL and TNFα through the Biacore analysis method. FIG. 4 shows a graph in which a horizontal axis is Time (o=capture_level) and a vertical axis is Response (o= capture_level).
FIG. 5 shows the results of in vitro blockade assay of an anti-OX₄oL antibody and a bispecific antibody capable of simultaneously binding to OX₄oL and TNFα.
FIG. 6 shows the results of confirming the effect of an anti-OX₄oL antibody and a bispecific antibody capable of simultaneously binding to OX₄oL and TNFα on IL-2 secretion of T cells.

### Mode for Invention

Hereinafter, the present invention will be described with reference to examples. However, the following exemplary embodiments are provided only for the purpose of illustrating the present invention, and thus the present invention is not limited thereto.

### Example 1: Selection of OX₄oL-specific antibody clones

### Example 1-1: Preparation of OX40L antigen

An antigen of human OX₄oL was used by obtaining a human OX₄oL protein (Cat# OXL-H52Q8) provided by Aero Biosystems, which was prepared by fusing a histidine tag to the N-terminus of amino acid sequences no. 51 to 183 (Q51 to L183) of the amino acid sequence (SEQ ID NO: 1) of the human OX₄oL of Accession No. NP_003317 by using a domain exposed outside the cell.

The amino acid sequence of the human OX04L protein (antigen) provided above by Aero Biosystems was specified in SEQ ID NO: 2. The antigen as provided above was produced in HEK293 cells and has a size of 16.9 kDa.

**[Table 2] OX₄oL antigen protein**

| **Type** | **SEQ ID NO:** | **Sequence** |
|---|---|---|
| OX40L antigen protein (OX40L) | **1** | MERVQPLEEN VGNAARPRFE RNKLLLVASV IQGLGLLLCF TYICLHFSAL QVSHRYPRIQ SIKVQFTEYK KEKGFILTSQ KEDEIMKVQN NSVIINCDGF YLISLKGYFS QEVNISLHYQ KDEEPLFQLK KVRSVNSLMV ASLTYKDKVY LNVTTDNTSL DDFHVNGGEL ILIHQNPGEF CVL |
| OX40L antigen protein (OX40L-his) | **2** | HHHHHH QVSHRYPRIQ SIKVQFTEYK KEKGFILTSQ KEDEIMKVQN NSVIINCDGF YLISLKGYFS QEVNISLHYQ KDEEPLFQLK KVRSVNSLMV ASLTYKDKVY LNVTTDNTSL DDFHVNGGEL ILIHQNPGEF CVL |

### Example 1-2: Preparation of human library phage

The 7.5 X 10¹⁰ human-derived scFv library cells with diversity were cultured in a 2X YT-glucose-Mgcl2-chloramphenicol (CM) medium at 37°C until the absorbance of the culture fluid became OD₆₀₀=0.5-0.7.

The cells were infected with helper phage and cultured at 37°C for about one hour. After centrifuging the cultured cells (5000 rpm, 4°C, 10 minutes), 2×YT-IPTG-Mgcl₂-kanamycine (KM)-CM medium was added, and the cells were remixed and cultured in a 30°C shaking incubator for 16 hours. The cultured cells were centrifuged (5000 rpm, 4°C, 10 minutes), after which 4% PEG (Sigma, 81253) and 3% NaCl (Junsei, 1905-0350) were added to and well dissolved in supernatant, and reacted in ice for about one hour. After centrifugation again (7500 rpm, 4°C, 30 minutes), DPBS (Wellgene, LB001-02) was added to pellets, dissolved, and centrifuged (10000 rpm, 4°C, 10 minutes) so as to obtain supernatant containing library phage, and then put into a new tube and stored at 5±3 °C.

### Example 1-3: Preparation of immune library phage

An immune library phage was prepared by using Balb/C Mouse and SD RAT, and the human OX₄oL antigen (SEQ ID NO: 2) of Example 1-1.

Ten Balb/C mice (seven weeks old) and four male SD RATs (eight weeks old) were acclimatized and then immunized with the human OX₄oL antigen on days 0, 21, 42, and 63, after which the spleen was removed on day 84 to elute RNA therefrom. By using the eluted RNA, cDNA was synthesized, and a heavy chain variable region and a light chain variable region were amplified. The heavy chain variable region and the light chain variable region were mixed to amplify DNA in the form of scFv, after which the DNA was inserted into a phage vector (pYG100) to prepare the RAT-derived immune scFv library cells. The prepared cells were cultured in a 2× Yf-glucose-Mgcl2-chloramphenicol (CM) medium at 37°C until the absorbance of the culture fluid became OD₆₀₀=0.5-0.7.

The cultured cells were infected with helper phage and cultured at 37°C for about one hour. After centrifuging the cultured cells (5000 rpm, 4°C, 10 minutes), 2×YT-IPTG-Mgcl₂-kanamycine (KM)-CM medium was added, and the cells were remixed and cultured in a 30°C shaking incubator for 16 hours. The cultured cells were centrifuged (5000 rpm, 4°C, 10 minutes), after which 4% PEG (Sigma, 81253) and 3% NaCl (Junsei, 1905-0350) were added to and well dissolved in supernatant, and reacted in ice for about one hour. After centrifugation again (7500 rpm, 4°C, 30 minutes), DPBS (Wellgene, LB001-02) was added to pellets and dissolved, after which centrifugation (10000 rpm, 4°C, 10 minutes) was performed to obtain supernatant containing library phage, and then put into a new tube and stored at 5±3 °C.

### Example 1-4: Panning through phage display

To screen for OX₄oL antibodies binding to human OX40L, a solution containing the human OX₄oL protein of Example 1-1 was added into an immunotube at a concentration of 1 to 10 µg/mL to adsorb OX₄oL protein on the surface of the immunotube at 5±3°C overnight, after which 1% bovine serum albumin solution was added to the test tube to protect the surface on which OX₄oL was not adsorbed. After emptying the test tube, a 10¹² CFU of human antibody phage library (Example 1-2) or an immune phage library (Example 1-3) dispersed in 1% bovine serum albumin solution was placed in the test tube and bound to the antigen. Non-specifically bound phage was washed 5 to 20 times with PBS-T (Phosphate buffered saline-0.05% Tween 20) solution and further washed 1 to 5 times with DPBS so as to recover the remaining antigen-specific phage antibodies by using 0.1 M TAE solution.

The recovered phage was neutralized with 1M Tris buffer (pH 7.5), and infected with XLiBlue E. coli at 37°C for one hour, after which the infected E. coli was smeared on a SOBCG plate by using glass beads and cultured in a 37°C incubator for about 16 hours. On the following day, the cultured E. coli was suspended in 4 mℓ of superbroth (SB)-carbenicillin culture fluid, after which 15% glycerol was added, so that a portion thereof was stored at -80°C and 50 µℓ of the residue was cultured at 37°C with the addition of 2% glucose solution into 20 mℓ of SB-carbenicillin culture fluid. When the absorbance of the culture fluid reached 0.6 at 600 nm, the culture fluid was removed by centrifugation and was suspended again in 20 mℓ of SB-carbenicillin culture fluid, after which 10¹² PFU of M13 helper phage was added, slowly stirred and incubated at 37°C. On the next day, the culture fluid was centrifuged, after which only the culture fluid was taken and polyethylene glycol and sodium chloride (NaCl) were added, so that the resulting solution was precipitated at 4°C for 30 minutes and centrifuged. The supernatant was removed, and the precipitated phage was suspended in 1 mℓ of PBS, after which the resulting suspension was used as a library to repeat the above panning process three to five times, thereby amplifying/concentrating antigen-specific clones.

### Example 1-5: Screening for specific clones after phage panning

In order to screen for antibodies (scFv) binding to the human OX40L protein, antigen-specific clones were screened by using one of the following two methods.

First, after panning, single colonies were obtained by smear culture on agar medium, and inoculated and cultured in 1 to 1.5 mL of culture fluid, and then induced with IPTG to express the scFv type protein in E. coli. The E. coli culture fluid was centrifuged to obtain a supernatant, which was then used to confirm the binding of the recombinant human OX₄oL antigen to scFv by using ELISA technique (Steinberger. Rader and Barbas III. 2000. Phage display vectors. In: Phage Display Laboratory Manual. 1sted. ColdSpringHarborLaboratoryPress. NY. USA. pp.11.9-11.12). The bound scFv was detected by using a horseradish peroxidase (HRP)-anti-His antibody and a tetramethylbenzidine (TMB) substrate.

Second, after panning, single phages were obtained from the SOBCG plate, inoculated into a deep well plate in which 1 mL of medium was dispensed respectively, and cultured with shaking at 37°C for 16 hours. The amplified cells were diluted 10 times, inoculated again in a deep well plate, and cultured in a shaking incubator at 37°C until 0.5 at OD₆₀₀. Upon reaching 0.5 at OD₆₀₀, M13 helper phage was placed at the level of 10⁹ CFU and infected in a 37°C static incubator for 30 minutes and in a 37°C shaking incubator for 30 minutes. After the infection was completed, the cells were precipitated by centrifugation and the supernatant was obtained to confirm the scFv binding to the human OX₄oL antigen by using ELISA technique. The bound scFv was detected by using a horseradish peroxidase (HRP)-anti-M13 antibody and a tetramethylbenzidine (TMB) substrate.

The antigen-specific antibody (scFv) clones identified from the above were analyzed through a sequencing technique.

### Example 2: Preparation of anti-OX40L antibody

Based on the sequence for the variable region (scFv) of the antibody obtained in above Example 1-5, a heavy chain variable region was linked to a heavy chain constant region (SEQ ID NO: 8), and a light chain variable region was linked to a light chain constant region (SEQ ID NO: 10), so that antibodies were prepared. The antibodies were named 02C09, Hu3F07, 10H07, 21G07, and I3F07.

**[Table 3] Heavy and light chain constant regions**

| **Constant region** | | |
|---|---|---|
| Amino acid Sequence | Heavy chain (SEQ ID NO: 8) | ASTKGPSVFPLAPCSRSTSESTAALGCLVKDYFPEPVTVSWNSGAL TSGVHTFPAVLQSSGLYSLSSVVTVPSSSLGTKTYTCNVDHKPSNT |
| | | KVDKRVESKYGPPCPPCPAPEFLGGPSVFLFPPKPKDTLMISRTPE VTCVVVDVSQEDPEVQFNWYVDGVEVHNAKTKPREEQFNSTYRV VSVLTVLHQDWLNGKEYKCKVSNKGLPSSIEKTISKAKGQPREPQ VYTLPPSQEEMTKNQVSLTCLVKGFYPSDIAVEWESNGQPENNYK TTPPVLDSDGSFFLYSRLTVDKSRWQEGNVFSCSVMHEALHNHY TQKSLSLSLGK |
| | Light chain (SEQ ID NO: 10) | |
| Nucleic acid (DNA) sequence | Heavy chain (SEQ ID NO: 9) | GCTTCCACCAAGGGCCCATCCGTCTTCCCCCTGGCGCCCTGCTCC AGGAGCACCTCCGAGAGCACAGCCGCCCTGGGCTGCCTGGTCAA GGACTACTTCCCCGAACCGGTGACGGTGTCGTGGAACTCAGGCG CCCTGACCAGCGGCGTGCACACCTTCCCGGCTGTCCTACAGTCCT CAGGACTCTACTCCCTCAGCAGCGTGGTGACCGTGCCCTCCAGCA GCTTGGGCACGAAGACCTACACCTGCAACGTAGATCACAAGCCCA GCAACACCAAGGTGGACAAGAGAGTTGAGTCCAAATATGGTCCC CCATGCCCACCATGCCCAGCACCTGAGTTCCTGGGGGGACCATCA GTCTTCCTGTTCCCCCCAAAACCCAAGGACACTCTCATGATCTCCC GGACCCCTGAGGTCACGTGCGTGGTGGTGGACGTGAGCCAGGAA GACCCCGAGGTCCAGTTCAACTGGTACGTGGATGGCGTGGAGGT GCATAATGCCAAGACAAAGCCGCGGGAGGAGCAGTTCAACAGCA CGTACCGTGTGGTCAGCGTCCTCACCGTCCTGCACCAGGACTGGC TGAACGGCAAGGAGTACAAGTGCAAGGTCTCCAACAAAGGCCTC CCGTCCTCCATCGAGAAAACCATCTCCAAAGCCAAAGGGCAGCCC CGAGAGCCACAGGTGTACACCCTGCCCCCATCCCAGGAGGAGAT GACCAAGAACCAGGTCAGCCTGACCTGCCTGGTCAAAGGCTTCTA CCCCAGCGACATCGCCGTGGAGTGGGAGAGCAATGGGCAGCCGG |
| | | AGAACAACTACAAGACCACGCCTCCCGTGCTGGACTCCGACGGCT CCTTCTTCCTCTACAGCAGGCTAACCGTGGACAAGAGCAGGTGGC AGGAGGGGAATGTCTTCTCATGCTCCGTGATGCATGAGGCTCTG CACAACCACTACACACAGAAGAGCCTCTCCCTGTCTCTGGGTAAA |
| | Light chain (SEQ ID NO: 11) | CGTACGGTGGCGGCGCCATCTGTCTTCATCTTCCCGCCATCTGAT GAGCAGTTGAAATCTGGAACTGCCTCTGTTGTGTGCCTGCTGAAT AACTTCTATCCCAGAGAGGCCAAAGTACAGTGGAAGGTGGATAA CGCCCTCCAATCGGGTAACTCCCAGGAGAGTGTCACAGAGCAGG ACAGCAAGGACAGCACCTACAGCCTCAGCAGCACCCTGACGCTGA GCAAAGCAGACTACGAGAAACACAAAGTCTACGCCTGCGAAGTC ACCCATCAGGGCCTGAGCTCGCCCGTCACAAAGAGCTTCAACAGG GGAGAGTGT |

### (1) Anti-OX40L antibody 02C09

The 02C09 antibody included heavy chain CDR1 represented by SEQ ID NO: 12; heavy chain CDR2 represented by SEQ ID NO: 15; and heavy chain CDR3 represented by SEQ ID NO: 19; light chain CDR1 represented by SEQ ID NO: 23; light chain CDR2 represented by SEQ ID NO: 25; and light chain CDR3 represented by SEQ ID NO: 27.

Anti-OX40L antibody 02C09 was prepared by using an expression vector pcDNA3.1 (Invitrogen) and an animal cell line of FreeStyle^{™} 293-F (Invitrogen).

Floating FreeStyle^{™} 293-F animal cells, which were transfected with an expression vector containing a gene encoding anti-OX₄oL antibody 02C09 by using PEI, a polymer for enhancing intracellular gene transfer efficiency, were cultured at 200 mL per bottle in a 500 mL Erlenmeyer flask for culture (Corning) and cultured in large quantities, if necessary.

The transfected FreeStyle^{™} 293-F cells were cultured in suspension under the conditions of 37°C and 8% CO₂, and the medium was cultured by using FreeStyle^{™} 293 Expression Medium AGT^{™} (Invitrogen, AG1000D9P1). At the time of overexpression, 500 µg of PEI (Polysciences, 23966-2) and 125 µg of DNA to be overexpressed were mixed in 5 mL of the culture medium. About 24 hours after adding DNA-PEI, 10 mL of 10% soytone (BD, 212488) was added and further cultured for about five days, and then only the supernatant was obtained and used for purifying antibodies.

For purification of antibodies, the antibodies were first purified from the culture fluid by using a recombinant Protein-A Sepharose column. If it is necessary to improve purity, a second purification was performed by using the first purification product. In this case, the purification was performed by using Superdex200 gel filtration chromatography or hydroxyapatite chromatography.

**[Table 4] CDR sequence of 02C09 antibody**

| **Type** | | **No.** | **Sequence** | **Type** | | **No.** | **Sequence** |
|---|---|---|---|---|---|---|---|
| **Heavy chain** | **CDR1** | **12** | YAWMS | **Light chain** | **CDR1** | **23** | RASQSIGRWLA |
| | **CDR2** | **15** | RIKGEPDGGTTEYADSVKG | | **CDR2** | **25** | KATILES |
| | **CDR3** | **19** | RRGLDV | | **CDR3** | **27** | QQYNSYPYT |

**[Table 5] Variable region sequence of 02C09 antibody**

| 02C09 | | |
|---|---|---|
| Amino acid Sequence | Heavy chain (SEQ ID NO: 37) | QMQLVQSGGGLVKPGGSLRVSCAASGFTFNYAWMSWVRQAP GKGLEWVGRIKGEPDGGTTEYADSVKGRFTISRDNSKNTLY |
| | | LQMNSLRAEDTAVYYCVSRRGLDVWGQGTTVTVSS |
| | Light chain (SEQ ID NO: 38) | DIQMTQSPSSLSASLGDRVTITCRASQSIGRWLAWYQQKPGN APKLLIYKATILESGVPSRFSGSGSGTEFALTISSLQPDDFSAYYC QQYNSYPYTFGQGTKLEIK |
| Nucleic acid (DNA) sequence | Heavy chain (SEQ ID NO: 39) | CAAATGCAGCTTGTTCAATCCGGCGGAGGCCTGGTTAAGCCTG GCGGATCTCTGAGAGTGTCTTGTGCCGCCAGCGGCTTCACCTTC AATTACGCCTGGATGAGCTGGGTCCGACAGGCCCCTGGAAAAG GACTGGAATGGGTCGGAAGAATCAAGGGCGAGCCTGATGGCGG AACCACAGAGTACGCCGATAGCGTGAAGGGCAGATTCACCATC AGCCGGGACAACAGCAAGAACACCCTGTACCTGCAGATGAACT CCCTGAGAGCCGAGGACACAGCCGTGTACTACTGTGTGTCTAG AAGAGGCCTGGATGTGTGGGGCCAGGGAACAACAGTGACAGTT TCTTCT |
| | Light chain (SEQ ID NO: 40) | GATATCCAGATGACACAGAGCCCTAGCAGCCTGTCTGCCTCTCT GGGCGATAGAGTGACCATCACATGTCGGGCCAGCCAGTCTATT GGACGGTGGCTGGCCTGGTATCAGCAAAAGCCTGGAAACGCCC CTAAGCTGCTGATCTACAAGGCCACCATCCTGGAAAGCGGAGT GCCTTCTAGATTTTCTGGCAGCGGCTCTGGCACCGAGTTTGCCC TGACAATTAGCTCCCTGCAGCCTGACGACTTCAGCGCCTACTAC TGCCAGCAGTACAACAGCTACCCCTACACCTTCGGCCAGGGCAC AAAGCTGGAAATCAAG |

### (2) Anti-OX40L antibody Hu3F07

Hu3F07 antibody included heavy chain CDR1 represented by SEQ ID NO: 13; heavy chain CDR2 represented by SEQ ID NO: 16; and heavy chain CDR3 represented by SEQ ID NO: 20; light chain CDR1 represented by SEQ ID NO: 24; light chain CDR2 represented by SEQ ID NO: 26; and light chain CDR3 represented by SEQ ID NO: 28.

Anti-OX40L antibody Hu3F07 was prepared by using an expression vector pcDNA3.1 (Invitrogen) and an animal cell line of FreeStyle^{™} 293-F (Invitrogen). Specific culture conditions, culture methods, and purification methods were performed in substantially the same manner as those described above in (1) anti-OX₄oL antibody 02C09.

**[Table 6] CDR sequence of Hu3F07 antibody**

| **Hu3F07** | | **No.** | **Sequence** | **Hu3F07** | | **No.** | **Sequence** |
|---|---|---|---|---|---|---|---|
| **Heavy chain** | **CDR1** | **13** | DYWMH | **Light chain** | **CDR1** | **24** | RSSQSLVHSNGNTYLH |
| | **CDR2** | **16** | EIDPPNGRTNYNEKFKS | | **CDR2** | **26** | KVSNRFS |
| | **CDR3** | **20** | GIYYVDY | | **CDR3** | **28** | SQSTHVPPT |

**[Table 7] Variable region sequence of Hu3F07 antibody**

| **Hu3F07** | | |
|---|---|---|
| Amino acid Sequence | Heavy chain (SEQ ID NO: 41) | QVQLVQSGAEVKKPGASVKVSCKASGFTFPDYWMHWVRQAP GQGLEWMGEIDPPNGRTNYNEKFKSRVTMTRDTSISTAYME LSRLRSDDTAVYYCARGIYYVDYWGQGTTVTVSS |
| | Light chain (SEQ ID NO: 42) | DIVMTQSPLSLPVTLGEPASISCRSSQSLVHSNGNTYLHWYLQ KPGQSPQLLIYKVSNRFSGVPDRFSGSGSGTDFTLKISRVEAED VGVYYCSQSTHVPPTFGGGTKLEIK |
| Nucleic acid | Heavy chain (SEQ ID NO: 43) | CAAGTTCAACTGGTTCAGTCTGGCGCCGAAGTGAAGAAACCAG GCGCCAGCGTGAAGGTGTCCTGCAAGGCCTCTGGCTTCACATTC |
| (DNA) sequence | | CCCGACTACTGGATGCACTGGGTTCGACAGGCTCCAGGACAGG GACTCGAATGGATGGGCGAGATCGATCCTCCTAACGGCCGGAC CAACTACAACGAGAAGTTCAAGAGCAGAGTGACCATGACCAGA GACACCAGCATCAGCACCGCCTACATGGAACTGAGCAGACTGA GAAGCGACGACACCGCCGTGTACTACTGTGCCAGAGGCATCTA CTACGTGGACTACTGGGGCCAGGGCACCACAGTGACAGTTAGC TCT |
| | Light chain (SEQ ID NO: 44) | GATATCGTGATGACACAGAGCCCTCTGAGCCTGCCTGTGACACT GGGAGAACCTGCCAGCATCAGCTGTAGAAGCAGCCAGAGCCTG GTGCACAGCAACGGCAATACCTACCTGCACTGGTATCTGCAGAA GCCCGGACAGTCTCCCCAGCTGCTGATCTACAAGGTGTCCAACA GATTCAGCGGCGTGCCCGATAGATTTTCTGGCAGCGGCTCTGG CACCGACTTCACCCTGAAGATTAGCAGAGTGGAAGCCGAGGAC GTGGGCGTGTACTACTGTAGCCAGTCTACCCACGTGCCACCTAC CTTTGGCGGCGGAACAAAGCTGGAAATCAAG |

### (3) Anti-OX40L antibody 10H07

10H07 antibody included heavy chain CDR1 represented by SEQ ID NO: 13; heavy chain CDR2 represented by SEQ ID NO: 17; and heavy chain CDR3 represented by SEQ ID NO: 21; light chain CDR1 represented by SEQ ID NO: 24; light chain CDR2 represented by SEQ ID NO: 26; and light chain CDR3 represented by SEQ ID NO: 29.

Anti-OX40L antibody 10H07 was prepared by using an expression vector pcDNA3.1 (Invitrogen) and an animal cell line of FreeStyle^{™} 293-F (Invitrogen). Detailed culture conditions, culture methods, and purification methods were performed in substantially the same manner as those described above in (1) anti-OX₄oL antibody 02C09.

**[Table 8] CDR sequence of 10H07 antibody**

| **10H07** | | **No.** | **Sequence** | **10H07** | | **No.** | **Sequence** |
|---|---|---|---|---|---|---|---|
| **Heavy chain** | **CDR1** | **13** | DYWMH | **Light chain** | **CDR1** | **24** | RSSQSLVHSNGNTYLH |
| | **CDR2** | **17** | EIDPGNGRTNYNEKFKN | | **CDR2** | **26** | KVSNRFS |
| | **CDR3** | **21** | EGAGRGFPY | | **CDR3** | **29** | SQSTHVPWT |

**[Table 9] Variable region sequence of 10H07 antibody**

| **10H07** | | |
|---|---|---|
| Amino acid Sequence | Heavy chain | QVYLQQSGAELVKPGASVKLSCKASGYTFTDYWMHWVKQR PGQGLEWIGEIDPGNGRTNYNEKFKNKATLSVDKSSNTA YMQFSSLTSEDSAVFYCASEGAGRGFPYWGQGTLVTVSA |
| | (SEQ ID NO: 45) | |
| | Light chain | DVVLTQTPLSLPVSLGDQASISCRSSQSLVHSNGNTYLHW YLQKPGQSPKLLIYKVSNRFSGVPDRFSGSGSGTDFTLKISR VEAEDLGVYFCSQSTHVPWTFGGGTKLEIK |
| | (SEQ ID NO: 46) | |
| Nucleic acid | Heavy chain | CAGGTTTATCTACAGCAGTCTGGGGCTGAACTGGTGAAGCC TGGGGCTTCAGTGAAGCTGTCCTGCAAGGCTTCTGGCTACA CCTTCACCGACTACTGGATGCACTGGGTGAAGCAGAGGCCT GGACAAGGCCTTGAGTGGATTGGAGAGATTGATCCTGGCAA CGGTCGTACTAACTACAATGAGAAGTTCAAGAACAAGGCCA CACTGAGTGTAGACAAATCCTCCAATACAGCCTACATGCAAT TCAGCAGCCTGACATCTGAGGACTCTGCGGTCTTTTACTGTG CAAGCGAGGGAGCTGGACGTGGATTTCCTTACTGGGGCCAA |
| | (SEQ ID NO: 47) | |
| (DNA) sequence | | |
| | | GATGTTGTGCTGACCCAAACTCCACTCTCCCTGCCTGTCAGT CTTGGAGATCAAGCCTCCATCTCTTGCAGATCTAGTCAGAGC CTTGTACACAGTAATGGAAACACCTATTTACATTGGTACCTG CAGAAGCCAGGCCAGTCTCCAAAGCTCCTGATCTACAAAGTT TCCAACCGATTTTCTGGGGTCCCAGACAGGTTCAGTGGCAGT GGATCAGGGACAGATTTCACACTCAAGATCAGCAGAGTGGA GGCTGAGGATCTGGGAGTTTATTTCTGCTCTCAAAGTACACA TGTTCCGTGGACGTTCGGTGGAGGCACCAAGCTGGAAATCA AA |
| | Light chain | GGGACTCTGGTCACTGTCTCCGCA |
| | (SEQ ID NO: 48) | |

### (4) Anti-OX40L antibody 21G07

The 21G07 antibody included heavy chain CDR1 represented by SEQ ID NO: 14; heavy chain CDR2 represented by SEQ ID NO: 18; and heavy chain CDR3 represented by SEQ ID NO: 22; light chain CDR1 represented by SEQ ID NO: 24; light chain CDR2 represented by SEQ ID NO: 26; and light chain CDR3 represented by SEQ ID NO: 30.

Anti-OX40L antibody 21G07 was prepared by using an expression vector pcDNA3.1 (Invitrogen) and an animal cell line of FreeStyle^{™} 293-F (Invitrogen). Detailed culture conditions, culture methods, and purification methods were performed in substantially the same manner as those described above in (1) anti-OX₄oL antibody 02C09.

**[Table 10] CDR sequence of 21G07 antibody**

| **21G07** | | **No.** | **Sequence** | **21G07** | | **No.** | **Sequence** |
|---|---|---|---|---|---|---|---|
| **Heavy chain** | **CDR1** | **14** | NYWMQ | **Light chain** | **CDR1** | **24** | RSSQSLVHSNGNTYLH |
| | **CDR2** | **18** | EIDPSDGRTNYNEKFKN | | **CDR2** | **26** | KVSNRFS |
| | **CDR3** | **22** | EVSLRSMDY | | **CDR3** | **30** | SQSTHVPFT |

**[Table 11] Variable region sequence of 21G07 antibody**

| **21G07** | | |
|---|---|---|
| Amino acid Sequence | Heavy chain | QVQLQQSGAELVKPGASVKLSCKASGYTFTNYWMQWVKQ RPGQGLEWIGEIDPSDGRTNYNEKFKNKATLTVDRSSSTA YIKLSSLTSEDSAVYYCAREVSLRSMDYWGQGTSVTVSS |
| | (SEQ ID NO: 49) | |
| | Light chain | DIVLTQSPLSLPVSLGDQASISCRSSQSLVHSNGNTYLHWY LQKPGQSPKLLIYKVSNRFSGVPDRFSGSGSGTDFTLKISRV EAEDLGVYFCSQSTHVPFTFGSGTKLEIK |
| | (SEQ ID NO: 50) | |
| Nucleic acid (DNA) sequence | Heavy chain | CAGGTCCAACTGCAGCAGTCTGGGGCTGAACTGGTGAAGCC TGGGGCTTCAGTGAAGCTGTCCTGCAAGGCTTCTGGCTACA CCTTCACCAACTACTGGATGCAATGGGTGAAACAGAGGCCC GGACAAGGCCTTGAGTGGATTGGAGAGATTGATCCTAGCGA CGGTCGTACTAACTACAATGAGAAGTTCAAGAACAAGGCCA CACTGACTGTAGACAGATCCTCCAGCACAGCCTACATAAAAC TCAGCAGCCTGACATCTGAGGACTCTGCGGTCTATTACTGTG CAAGAGAGGTTTCACTACGGTCAATGGACTACTGGGGCCAA GGGACCTCAGTCACCGTCTCCTCA |
| | (SEQ ID NO: 51) | |
| | Light chain | GACATTGTTCTCACCCAGTCTCCACTCTCCCTGCCTGTCAGT CTTGGAGATCAAGCCTCCATCTCTTGCAGATCTAGTCAGAGC CTTGTACACAGTAATGGAAACACCTATTTACATTGGTACCTG CAGAAGCCAGGCCAGTCTCCAAAGCTCCTGATCTACAAAGTT |
| | (SEQ ID NO: 52) | TCCAACCGATTTTCTGGGGTCCCAGACAGGTTCAGTGGCAGT GGATCAGGGACAGATTTCACACTCAAGATCAGCAGAGTGGA GGCTGAGGATCTGGGAGTTTATTTCTGCTCTCAAAGTACACA TGTTCCATTCACGTTCGGCTCGGGGACAAAGTTGGAAATAA AA |

### (5) Anti-OX40L antibody I3F07

I3F07 antibody included heavy chain CDR1 represented by SEQ ID NO: 13; heavy chain CDR2 represented by SEQ ID NO: 16; and heavy chain CDR3 represented by SEQ ID NO: 20; light chain CDR1 represented by SEQ ID NO: 24; light chain CDR2 represented by SEQ ID NO: 26; and light chain CDR3 represented by SEQ ID NO: 28.

Anti-OX40L antibody I3F07 was prepared by using an expression vector pcDNA3.1 (Invitrogen) and an animal cell line of FreeStyle^{™} 293-F (Invitrogen). Detailed culture conditions, culture methods, and purification methods were performed in substantially the same manner as those described above in (1) anti-OX₄oL antibody 02C09.

**[Table 12] CDR sequence of I3F07 antibody**

| **I3F07** | | **No.** | **Sequence** | **I3F07** | | **No.** | **Sequence** |
|---|---|---|---|---|---|---|---|
| **Heavy chain** | **CDR1** | **13** | DYWMH | **Light chain** | **CDR1** | **24** | RSSQSLVHSNGNTYLH |
| | **CDR2** | **16** | EIDPPNGRTNYNEKFKS | | **CDR2** | **26** | KVSNRFS |
| | **CDR3** | **20** | GIYYVDY | | **CDR3** | **28** | SQSTHVPPT |

**[Table 13] Variable region sequence of I3F07 antibody**

| **I3F07** | | |
|---|---|---|
| Amino acid Sequence | Heavy chain (SEQ ID NO: 53) | QIQLAQSGAELVKPGASVKLSCKASGFTFPDYWMHWVKQR PGQGLEWIGEIDPPNGRTNYNEKFKSKATLTVDSSSNTAY MQLSRLTSEDSAVYYCARGIYYVDYWGQGTTLTVSS |
| | Light chain (SEQ ID NO: 54) | DIVLTQSPLSLPVSLGDQASISCRSSQSLVHSNGNTYLHWY LQKPGQSPKLLIYKVSNRFSGVPDRFSGSGSGTDFTLKISRV EAEDLGVYFCSQSTHVPPTFGGGSKLEIK |
| Nucleic acid (DNA) sequence | Heavy chain (SEQ ID NO: 55) | CAGATTCAGCTGGCTCAGTCTGGCGCCGAACTTGTGAAACCT GGCGCCTCTGTGAAGCTGAGCTGCAAGGCCAGCGGCTTCAC CTTTCCTGACTACTGGATGCACTGGGTCAAGCAGAGGCCTG GACAGGGACTCGAATGGATCGGCGAGATCGATCCTCCTAAC GGCCGGACCAACTACAACGAGAAGTTCAAGAGCAAGGCCAC ACTGACCGTGGACAGCAGCAGCAATACCGCCTACATGCAGC TGAGCAGACTGACCTCTGAGGACAGCGCCGTGTACTACTGT GCCAGAGGCATCTACTACGTGGACTACTGGGGCCAGGGCAC AACCCTGACAGTTTCTTCT |
| | Light chain (SEQ ID NO: 56) | GATATCGTGCTGACACAGAGCCCTCTGAGCCTGCCTGTTTCT CTGGGAGATCAGGCCAGCATCAGCTGCAGATCCTCTCAGAG CCTGGTGCACAGCAACGGCAATACCTACCTGCACTGGTATCT GCAGAAGCCCGGCCAGTCTCCTAAGCTGCTGATCTACAAGG TGTCCAACAGGTTCAGCGGCGTGCCCGATAGATTTTCTGGCT CTGGCAGCGGCACCGACTTCACCCTGAAGATTTCTAGAGTG GAAGCCGAGGACCTGGGCGTGTACTTCTGTTCTCAGTCTACC CACGTGCCACCTACCTTTGGCGGCGGAAGCAAGCTGGAAAT CAAG |

### Example 3: Preparation of bispecific antibody targeting OX40L and TNFα

A bispecific antibody capable of binding to even human TNFα was prepared by connecting an antibody binding to human OX40L (02C09, Hu3F07, I₃FO₇) or a fragment thereof (scFv), which was prepared in Example 2, with an anti-TNFα antibody (TNFai antibody (Humira)) or a fragment thereof (scFv) through a linker (see FIG. 1).

Specifically, the bispecific antibody was prepared to take on (i) a form of connecting a heavy chain variable region and a light chain variable region of an anti-TNFα antibody at C-terminus of a heavy chain constant region of an anti-OX₄oL antibody through a linker; (ii) a form of connecting a heavy chain variable region and a light chain variable region of an anti-TNFα antibody at C-terminus of a light chain constant region of an anti-OX40L antibody through a linker; (iii) a form of connecting a heavy chain variable region and a light chain variable region of an anti-OX₄oL antibody at C-terminus of a heavy chain constant region of an anti-TNFα antibody through a linker; and (iv) a form of connecting a heavy chain variable region and a light chain variable region of an anti-OX₄oL antibody at C-terminus of a light chain constant region of an anti-TNFα antibody through a linker, and was named 02C09-TNFαi HC, 02C09-TNFαi LC, hu3F07-TNFai HC, hu3F07-TNFαi LC, TNFαi-02C09 HC, TNFαi-02C09 LC, TNFαi-hu3F07 HC, TNFαi-hu3F07 LC, I3F07-TNFαi HC, I3F07-TNFαi LC, TNFαi-I3F07 HC, and TNFai-I3F07 LC in the order of IgG-scFv-binding site, respectively.

The linker used was GGGGSGGGGSGGGGS represented by SEQ ID NO: 31.

**[Table 14] CDR of anti-TNFα antibody**

| **Anti-TNFα antibody** | | **No.** | **Sequence** | **Anti-TNFα antibody** | | **No.** | **Sequence** |
|---|---|---|---|---|---|---|---|
| **Heavy chain** | **CDR1** | **89** | DYAMH | **Light chain** | **CDR1** | **92** | RASQGIRNYLA |
| | **CDR2** | **90** | AITWNSGHIDYADSVEG | | **CDR2** | **93** | AASTLQS |
| | **CDR3** | **91** | VSYLSTASSLDY | | **CDR3** | **94** | QRYNRAPYT |

**[Table 15] Variable region sequence of anti-TNFα antibody**

| Anti-TNFα antibody | |
|---|---|
| Heavy chain (SEQ ID NO: 35) | |
| Light chain (SEQ ID NO: 36) | |

The TNFai antibody was prepared by substantially the same method as shown in the method for producing (preparing) an anti-OX₄oL antibody of above Example 2, in such a way that the heavy chain variable region of the TNFα antibody was linked to the heavy chain constant region (SEQ ID NO: 8), and the light chain variable region was linked to the light chain constant region (SEQ ID NO: 10).

### (1) Bispecific antibody 02C09-TNFαi HC

In case of the bispecific antibody 02C09-TNFαi HC, a binding fragment (ScFv) of TNFai antibody, in which a heavy chain variable region (SEQ ID NO: 35) of TNFai antibody (Humira) and a light chain variable region (SEQ ID NO: 36) of please change it TNFai antibody (Humira) are connected through a linker (SEQ ID NO: 32), was connected at C-terminus of a heavy chain constant region of anti-OX₄oL antibody 02C09 through a linker (SEQ ID NO: 31).

Bispecific antibody 02C09-TNFαi HC was prepared by using an expression vector pcDNA3.1 (Invitrogen) and an animal cell line of FreeStyle^{™} 293-F (Invitrogen). Detailed culture conditions, culture methods, and purification methods were performed in substantially the same manner as those described above in (1) anti-OX₄oL antibody 02C09.

**[Table 16] Heavy chain of 02C09-TNFαi HC**

| 02C09 heavy chain-linker- TNFai variable region | |
|---|---|
| Amino acid sequence (SEQ ID NO: 57) | QMQLVQSGGGLVKPGGSLRVSCAASGFTFNYAWMSWVRQAPGKGLE WVGRIKGEPDGGTTEYADSVKGRFTISRDNSKNTLYLQMNSLRAEDTA VYYCVSRRGLDVWGQGTTVTVSSASTKGPSVFPLAPCSRSTSESTAALGCLVK DYFPEPVTVSWNSGALTSGVHTFPAVLQSSGLYSLSSVVTVPSSSLGTKTYTCNVD HKPSNTKVDKRVESKYGPPCPPCPAPEFLGGPSVFLFPPKPKDTLMISRTPEVTCV VVDVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSC SVMHEALHNHYTQKSLSLSLGKGGGGSGGGGSGGGGSEVQLVESGGGLVQP GRSLRLSCAASGFTFDDYAMHWVRQAPGKGLEWVSAITWNSGHIDYADSVEGR FTISRDNAKNSLYLQMNSLRAEDTAVYYCAKVSYLSTASSLDYWGQGTLVTVSSG GGGSGGGGSGGGGSGGGGSDIQMTQSPSSLSASVGDRVTITCRASQGIRNYL AWYQQKPGKAPKLLIYAASTLQSGVPSRFSGSGSGTDFTLTISSLQPEDVATYYCQ |
| | RYNRAPYTFGQGTKVEIK |
| Nucleic acid (DNA) sequence (SEQ ID NO: 58) | CAAATGCAGCTTGTTCAATCCGGCGGAGGCCTGGTTAAGCCTGGCGGA TCTCTGAGAGTGTCTTGTGCCGCCAGCGGCTTCACCTTCAATTACGCCT GGATGAGCTGGGTCCGACAGGCCCCTGGAAAAGGACTGGAATGGGTCG GAAGAATCAAGGGCGAGCCTGATGGCGGAACCACAGAGTACGCCGATA GCGTGAAGGGCAGATTCACCATCAGCCGGGACAACAGCAAGAACACCC TGTACCTGCAGATGAACTCCCTGAGAGCCGAGGACACAGCCGTGTACT ACTGTGTGTCTAGAAGAGGCCTGGATGTGTGGGGCCAGGGAACAACAG TGACAGTTTCTTCTGCTTCCACCAAGGGCCCATCCGTCTTCCCCCTGGCGCCCT GCTCCAGGAGCACCTCCGAGAGCACAGCCGCCCTGGGCTGCCTGGTCAAGGACT ACTTCCCCGAACCGGTGACGGTGTCGTGGAACTCAGGCGCCCTGACCAGCGGCG TGCACACCTTCCCGGCTGTCCTACAGTCCTCAGGACTCTACTCCCTCAGCAGCGT GGTGACCGTGCCCTCCAGCAGCTTGGGCACGAAGACCTACACCTGCAACGTAGA TCACAAGCCCAGCAACACCAAGGTGGACAAGAGAGTTGAGTCCAAATATGGTCC CCCATGCCCACCATGCCCAGCACCTGAGTTCCTGGGGGGACCATCAGTCTTCCTG TTCCCCCCAAAACCCAAGGACACTCTCATGATCTCCCGGACCCCTGAGGTCACGT GCGTGGTGGTGGACGTGAGCCAGGAAGACCCCGAGGTCCAGTTCAACTGGTAC GTGGATGGCGTGGAGGTGCATAATGCCAAGACAAAGCCGCGGGAGGAGCAGTT CAACAGCACGTACCGTGTGGTCAGCGTCCTCACCGTCCTGCACCAGGACTGGCT GAACGGCAAGGAGTACAAGTGCAAGGTCTCCAACAAAGGCCTCCCGTCCTCCAT CGAGAAAACCATCTCCAAAGCCAAAGGGCAGCCCCGAGAGCCACAGGTGTACAC CCTGCCCCCATCCCAGGAGGAGATGACCAAGAACCAGGTCAGCCTGACCTGCCT GGTCAAAGGCTTCTACCCCAGCGACATCGCCGTGGAGTGGGAGAGCAATGGGCA GCCGGAGAACAACTACAAGACCACGCCTCCCGTGCTGGACTCCGACGGCTCCTT CTTCCTCTACAGCAGGCTAACCGTGGACAAGAGCAGGTGGCAGGAGGGGAATGT CTTCTCATGCTCCGTGATGCATGAGGCTCTGCACAACCACTACACACAGAAGAGC |
| | CTCTCCCTGTCTCTGGGTAAAGGTGGTGGCGGATCTGGCGGCGGAGGAAG CGGAGGCGGAGGCTCCGAAGTGCAACTGGTTGAATCTGGCGGAGGACTGGT GCAGCCTGGAAGAAGCCTGAGACTGTCTTGTGCCGCCAGCGGCTTCACCTTCGA TGATTATGCCATGCACTGGGTCCGACAGGCCCCTGGAAAAGGACTTGAATGGGT GTCCGCCATCACCTGGAACAGCGGCCACATCGATTACGCCGATAGCGTGGAAGG CCGGTTCACCATCAGCAGAGACAACGCCAAGAACAGCCTGTACCTGCAGATGAA CTCCCTGAGAGCCGAGGACACCGCCGTGTACTACTGTGCCAAAGTGTCTTACCT GAGCACCGCCTCCAGCCTGGATTATTGGGGACAGGGCACACTGGTCACAGTGTC CTCTGGTGGCGGAGGTTCTGGCGGAGGTGGTAGTGGTGGCGGTGGAAG TGGTGGCGGCGGATCTGATATCCAGATGACACAGAGCCCTAGCAGCCTGTCT GCCTCTGTGGGCGATAGAGTGACCATCACCTGTAGAGCCAGCCAGGGCATCAGA AACTACCTGGCCTGGTATCAGCAGAAGCCCGGAAAGGCCCCTAAGCTGCTGATC TATGCCGCCTCTACACTGCAGAGCGGCGTGCCATCTAGATTTTCTGGCAGCGGC TCTGGCACCGACTTCACCCTGACAATTTCTAGCCTGCAGCCTGAGGACGTGGCCA CCTACTACTGCCAGAGATACAACAGAGCCCCTTACACCTTCGGCCAGGGCACCAA GGTGGAAATCAAG |

### (2) Bispecific antibody 02C09-TNFαi LC

In case of 02C09-TNFαi LC, a binding fragment (ScFv) of TNFai antibody, in which a heavy chain variable region (SEQ ID NO: 35) of Humira and a light chain variable region (SEQ ID NO: 36) of Humira are connected through a linker (SEQ ID NO: 32), was connected at C-terminus of a light chain constant region of anti-OX₄oL antibody 02C09 through a linker (SEQ ID NO: 31).

Bispecific antibody 02C09-TNFαi LC was prepared by using an expression vector pcDNA3.1 (Invitrogen) and an animal cell line of FreeStyle^{™} 293-F (Invitrogen). Detailed culture conditions, culture methods, and purification methods were performed in substantially the same manner as those described above in (1) anti-OX₄oL antibody 02C09.

**[Table 17] Light chain of 02C09-TNFαi LC**

| 02C09 light chain-linker- TNFai variable region | |
|---|---|
| Amino acid sequence (SEQ ID NO: 59) | DIQMTQSPSSLSASLGDRVTITCRASQSIGRWLAWYQQKPGNAPKLLIY KATILESGVPSRFSGSGSGTEFALTISSLQPDDFSAYYCQQYNSYPYTFG QGTKLEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSPVTKSF NRGECGGGGSGGGGSGGGGSEVQLVESGGGLVQPGRSLRLSCAASGFTFDDY AMHWVRQAPGKGLEWVSAITWNSGHIDYADSVEGRFTISRDNAKNSLYLQMNS LRAEDTAVYYCAKVSYLSTASSLDYWGQGTLVTVSSGGGGSGGGGSGGGGSG GGGSDIQMTQSPSSLSASVGDRVTITCRASQGIRNYLAWYQQKPGKAPKLLIYAA STLQSGVPSRFSGSGSGTDFTLTISSLQPEDVATYYCQRYNRAPYTFGQGTKVEIK |
| Nucleic acid (DNA) sequence (SEQ ID NO: 60) | GATATCCAGATGACACAGAGCCCTAGCAGCCTGTCTGCCTCTCTGGGCG ATAGAGTGACCATCACATGTCGGGCCAGCCAGTCTATTGGACGGTGGC TGGCCTGGTATCAGCAAAAGCCTGGAAACGCCCCTAAGCTGCTGATCTA CAAGGCCACCATCCTGGAAAGCGGAGTGCCTTCTAGATTTTCTGGCAGC GGCTCTGGCACCGAGTTTGCCCTGACAATTAGCTCCCTGCAGCCTGACG ACTTCAGCGCCTACTACTGCCAGCAGTACAACAGCTACCCCTACACCTT CGGCCAGGGCACAAAGCTGGAAATCAAGCGTACGGTGGCGGCGCCATCTG TCTTCATCTTCCCGCCATCTGATGAGCAGTTGAAATCTGGAACTGCCTCTGTTGT GTGCCTGCTGAATAACTTCTATCCCAGAGAGGCCAAAGTACAGTGGAAGGTGGA TAACGCCCTCCAATCGGGTAACTCCCAGGAGAGTGTCACAGAGCAGGACAGCAA GGACAGCACCTACAGCCTCAGCAGCACCCTGACGCTGAGCAAAGCAGACTACGA |
| | GAAACACAAAGTCTACGCCTGCGAAGTCACCCATCAGGGCCTGAGCTCGCCCGT CACAAAGAGCTTCAACAGGGGAGAGTGTGGTGGTGGCGGATCTGGCGGCG GAGGAAGCGGAGGCGGAGGCTCCGAAGTGCAACTGGTTGAATCTGGCGGA GGACTGGTGCAGCCTGGAAGAAGCCTGAGACTGTCTTGTGCCGCCAGCGGCTTC ACCTTCGATGATTATGCCATGCACTGGGTCCGACAGGCCCCTGGAAAAGGACTT GAATGGGTGTCCGCCATCACCTGGAACAGCGGCCACATCGATTACGCCGATAGC GTGGAAGGCCGGTTCACCATCAGCAGAGACAACGCCAAGAACAGCCTGTACCTG CAGATGAACTCCCTGAGAGCCGAGGACACCGCCGTGTACTACTGTGCCAAAGTG TCTTACCTGAGCACCGCCTCCAGCCTGGATTATTGGGGACAGGGCACACTGGTC ACAGTGTCCTCTGGTGGCGGAGGTTCTGGCGGAGGTGGTAGTGGTGGCGGTGG AAGTGGTGGCGGCGGATCTGATATCCAGATGACACAGAGCCCTAGCAGCCTGTC TGCCTCTGTGGGCGATAGAGTGACCATCACCTGTAGAGCCAGCCAGGGCATCAG AAACTACCTGGCCTGGTATCAGCAGAAGCCCGGAAAGGCCCCTAAGCTGCTGAT CTATGCCGCCTCTACACTGCAGAGCGGCGTGCCATCTAGATTTTCTGGCAGCGG CTCTGGCACCGACTTCACCCTGACAATTTCTAGCCTGCAGCCTGAGGACGTGGCC ACCTACTACTGCCAGAGATACAACAGAGCCCCTTACACCTTCGGCCAGGGCACCA AGGTGGAAATCAAG |

### (3) Bispecific antibody Hu3F07-TNFαi HC

In case of Hu3F07-TNFαi HC, a binding fragment (ScFv) of TNFai antibody, in which a heavy chain variable region (SEQ ID NO: 35) of TNFai antibody and a light chain variable region (SEQ ID NO: 36) of TNFai antibody are connected through a linker (SEQ ID NO: 32), was connected at C-terminus of a heavy chain constant region of anti-OX₄oL antibody hu3F07 through a linker (SEQ ID NO: 31).

Bispecific antibody Hu3F07-TNFαi HC was prepared by using an expression vector pcDNA3.1 (Invitrogen) and an animal cell line of FreeStyle^{™} 293-F (Invitrogen). Detailed culture conditions, culture methods, and purification methods were performed in substantially the same manner as those described above in (1) anti-OX₄oL antibody 02C09.

**[Table 18] Heavy chain of Hu3F07-TNFαi HC**

| Hu3F07 heavy chain-linker- TNFai variable region | |
|---|---|
| Amino acid sequence | QVQLVQSGAEVKKPGASVKVSCKASGFTFPDYWMHWVRQAPGQGLE WMGEIDPPNGRTNYNEKFKSRVTMTRDTSISTAYMELSRLRSDDTAVY YCARGIYYVDYWGQGTTVTVSSASTKGPSVFPLAPCSRSTSESTAALGCLVKD YFPEPVTVSWNSGALTSGVHTFPAVLQSSGLYSLSSVVTVPSSSLGTKTYTCNVDH KPSNTKVDKRVESKYGPPCPPCPAPEFLGGPSVFLFPPKPKDTLMISRTPEVTCVV VDVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFSC SVMHEALHNHYTQKSLSLSLGKGGGGSGGGGSGGGGSEVQLVESGGGLVQP GRSLRLSCAASGFTFDDYAMHWVRQAPGKGLEWVSAITWNSGHIDYADSVEGR FTISRDNAKNSLYLQMNSLRAEDTAVYYCAKVSYLSTASSLDYWGQGTLVTVSSG GGGSGGGGSGGGGSGGGGSDIQMTQSPSSLSASVGDRVTITCRASQGIRNYL AWYQQKPGKAPKLLIYAASTLQSGVPSRFSGSGSGTDFTLTISSLQPEDVATYYCQ RYNRAPYTFGQGTKVEIK |
| (SEQ ID NO: 61) | |
| Nucleic acid (DNA) sequence | CAAGTTCAACTGGTTCAGTCTGGCGCCGAAGTGAAGAAACCAGGCGCC AGCGTGAAGGTGTCCTGCAAGGCCTCTGGCTTCACATTCCCCGACTACT GGATGCACTGGGTTCGACAGGCTCCAGGACAGGGACTCGAATGGATGG GCGAGATCGATCCTCCTAACGGCCGGACCAACTACAACGAGAAGTTCA AGAGCAGAGTGACCATGACCAGAGACACCAGCATCAGCACCGCCTACA |
| (SEQ ID NO: 62) | |
| | TGGAACTGAGCAGACTGAGAAGCGACGACACCGCCGTGTACTACTGTG CCAGAGGCATCTACTACGTGGACTACTGGGGCCAGGGCACCACAGTGA CAGTTAGCTCTGCTTCCACCAAGGGCCCATCCGTCTTCCCCCTGGCGCCCTGCT CCAGGAGCACCTCCGAGAGCACAGCCGCCCTGGGCTGCCTGGTCAAGGACTACT TCCCCGAACCGGTGACGGTGTCGTGGAACTCAGGCGCCCTGACCAGCGGCGTGC ACACCTTCCCGGCTGTCCTACAGTCCTCAGGACTCTACTCCCTCAGCAGCGTGGT GACCGTGCCCTCCAGCAGCTTGGGCACGAAGACCTACACCTGCAACGTAGATCA CAAGCCCAGCAACACCAAGGTGGACAAGAGAGTTGAGTCCAAATATGGTCCCCC ATGCCCACCATGCCCAGCACCTGAGTTCCTGGGGGGACCATCAGTCTTCCTGTTC CCCCCAAAACCCAAGGACACTCTCATGATCTCCCGGACCCCTGAGGTCACGTGCG TGGTGGTGGACGTGAGCCAGGAAGACCCCGAGGTCCAGTTCAACTGGTACGTG GATGGCGTGGAGGTGCATAATGCCAAGACAAAGCCGCGGGAGGAGCAGTTCAA CAGCACGTACCGTGTGGTCAGCGTCCTCACCGTCCTGCACCAGGACTGGCTGAA CGGCAAGGAGTACAAGTGCAAGGTCTCCAACAAAGGCCTCCCGTCCTCCATCGA GAAAACCATCTCCAAAGCCAAAGGGCAGCCCCGAGAGCCACAGGTGTACACCCT GCCCCCATCCCAGGAGGAGATGACCAAGAACCAGGTCAGCCTGACCTGCCTGGT CAAAGGCTTCTACCCCAGCGACATCGCCGTGGAGTGGGAGAGCAATGGGCAGCC GGAGAACAACTACAAGACCACGCCTCCCGTGCTGGACTCCGACGGCTCCTTCTTC CTCTACAGCAGGCTAACCGTGGACAAGAGCAGGTGGCAGGAGGGGAATGTCTTC TCATGCTCCGTGATGCATGAGGCTCTGCACAACCACTACACACAGAAGAGCCTCT CCCTGTCTCTGGGTAAAGGTGGTGGCGGATCTGGCGGCGGAGGAAGCGG AGGCGGAGGCTCCGAAGTGCAACTGGTTGAATCTGGCGGAGGACTGGTGCAG CCTGGAAGAAGCCTGAGACTGTCTTGTGCCGCCAGCGGCTTCACCTTCGATGAT TATGCCATGCACTGGGTCCGACAGGCCCCTGGAAAAGGACTTGAATGGGTGTCC GCCATCACCTGGAACAGCGGCCACATCGATTACGCCGATAGCGTGGAAGGCCGG TTCACCATCAGCAGAGACAACGCCAAGAACAGCCTGTACCTGCAGATGAACTCC CTGAGAGCCGAGGACACCGCCGTGTACTACTGTGCCAAAGTGTCTTACCTGAGC |
| | ACCGCCTCCAGCCTGGATTATTGGGGACAGGGCACACTGGTCACAGTGTCCTCT GGTGGCGGAGGTTCTGGCGGAGGTGGTAGTGGTGGCGGTGGAAGTGGTGGCG GCGGATCTGATATCCAGATGACACAGAGCCCTAGCAGCCTGTCTGCCTCTGTGG GCGATAGAGTGACCATCACCTGTAGAGCCAGCCAGGGCATCAGAAACTACCTGG CCTGGTATCAGCAGAAGCCCGGAAAGGCCCCTAAGCTGCTGATCTATGCCGCCT CTACACTGCAGAGCGGCGTGCCATCTAGATTTTCTGGCAGCGGCTCTGGCACCG ACTTCACCCTGACAATTTCTAGCCTGCAGCCTGAGGACGTGGCCACCTACTACTG CCAGAGATACAACAGAGCCCCTTACACCTTCGGCCAGGGCACCAAGGTGGAAAT CAAG |

### (4) Bispecific antibody Hu3F07-TNFαi LC

In case of Hu3F07-TNFαi LC, a binding fragment (ScFv) of TNFai antibody, in which a heavy chain variable region (SEQ ID NO: 35) of TNFai antibody and a light chain variable region (SEQ ID NO: 36) of TNFai antibody are connected through a linker (SEQ ID NO: 32), was connected at C-terminus of a light chain constant region of anti-OX₄oL antibody hu3F07 through a linker (SEQ ID NO: 31).

Bispecific antibody Hu3F07-TNFαi LC was prepared by using an expression vector pcDNA3.1 (Invitrogen) and an animal cell line of FreeStyle^{™} 293-F. Detailed culture conditions, culture methods, and purification methods were performed in substantially the same manner as those described above in (1) anti-OX₄oL antibody 02C09.

**[Table 19] Light chain of Hu3F07-TNFαi LC**

| Hu3F07 light chain-linker- TNFai variable region | |
|---|---|
| Amino acid sequence | DIVMTQSPLSLPVTLGEPASISCRSSQSLVHSNGNTYLHWYLQKPGQSP QLLIYKVSNRFSGVPDRFSGSGSGTDFTLKISRVEAEDVGVYYCSQSTH VPPTFGGGTKLEIKRTVAAPSVFIFPPSDEQLKSGTASVVCLLNNFYPREAKVQ WKVDNALQSGNSQESVTEQDSKDSTYSLSSTLTLSKADYEKHKVYACEVTHQGL SSPVTKSFNRGECGGGGSGGGGSGGGGSEVQLVESGGGLVQPGRSLRLSCAAS GFTFDDYAMHWVRQAPGKGLEWVSAITWNSGHIDYADSVEGRFTISRDNAKNS LYLQMNSLRAEDTAVYYCAKVSYLSTASSLDYWGQGTLVTVSSGGGGSGGGGS GGGGSGGGGSDIQMTQSPSSLSASVGDRVTITCRASQGIRNYLAWYQQKPGKA PKLLIYAASTLQSGVPSRFSGSGSGTDFTLTISSLQPEDVATYYCQRYNRAPYTFGQ GTKVEIK |
| (SEQ ID NO: 63) | |
| Nucleic acid (DNA) sequence (SEQ ID NO: 64) | GATATCGTGATGACACAGAGCCCTCTGAGCCTGCCTGTGACACTGGGA GAACCTGCCAGCATCAGCTGTAGAAGCAGCCAGAGCCTGGTGCACAGC AACGGCAATACCTACCTGCACTGGTATCTGCAGAAGCCCGGACAGTCTC CCCAGCTGCTGATCTACAAGGTGTCCAACAGATTCAGCGGCGTGCCCGA TAGATTTTCTGGCAGCGGCTCTGGCACCGACTTCACCCTGAAGATTAGC AGAGTGGAAGCCGAGGACGTGGGCGTGTACTACTGTAGCCAGTCTACC CACGTGCCACCTACCTTTGGCGGCGGAACAAAGCTGGAAATCAAGCGTA CGGTGGCGGCGCCATCTGTCTTCATCTTCCCGCCATCTGATGAGCAGTTGAAATC TGGAACTGCCTCTGTTGTGTGCCTGCTGAATAACTTCTATCCCAGAGAGGCCAAA GTACAGTGGAAGGTGGATAACGCCCTCCAATCGGGTAACTCCCAGGAGAGTGTC ACAGAGCAGGACAGCAAGGACAGCACCTACAGCCTCAGCAGCACCCTGACGCTG AGCAAAGCAGACTACGAGAAACACAAAGTCTACGCCTGCGAAGTCACCCATCAG GGCCTGAGCTCGCCCGTCACAAAGAGCTTCAACAGGGGAGAGTGTGGTGGTG GCGGATCTGGCGGCGGAGGAAGCGGAGGCGGAGGCTCCGAAGTGCAAC TGGTTGAATCTGGCGGAGGACTGGTGCAGCCTGGAAGAAGCCTGAGACTGTCTT GTGCCGCCAGCGGCTTCACCTTCGATGATTATGCCATGCACTGGGTCCGACAGG CCCCTGGAAAAGGACTTGAATGGGTGTCCGCCATCACCTGGAACAGCGGCCACA |
| | TCGATTACGCCGATAGCGTGGAAGGCCGGTTCACCATCAGCAGAGACAACGCCA AGAACAGCCTGTACCTGCAGATGAACTCCCTGAGAGCCGAGGACACCGCCGTGT ACTACTGTGCCAAAGTGTCTTACCTGAGCACCGCCTCCAGCCTGGATTATTGGG GACAGGGCACACTGGTCACAGTGTCCTCTGGTGGCGGAGGTTCTGGCGGAG GTGGTAGTGGTGGCGGTGGAAGTGGTGGCGGCGGATCTGATATCCAGAT GACACAGAGCCCTAGCAGCCTGTCTGCCTCTGTGGGCGATAGAGTGACCATCAC CTGTAGAGCCAGCCAGGGCATCAGAAACTACCTGGCCTGGTATCAGCAGAAGCC CGGAAAGGCCCCTAAGCTGCTGATCTATGCCGCCTCTACACTGCAGAGCGGCGT GCCATCTAGATTTTCTGGCAGCGGCTCTGGCACCGACTTCACCCTGACAATTTCT AGCCTGCAGCCTGAGGACGTGGCCACCTACTACTGCCAGAGATACAACAGAGCC CCTTACACCTTCGGCCAGGGCACCAAGGTGGAAATCAAG |

### (5) Bispecific antibody TNFαi-02C09 HC

In case of TNFαi-02C09 HC, a binding fragment (ScFv) of an anti-OX₄oL antibody 02C09, in which a heavy chain variable region (SEQ ID NO: 37) of anti-OX₄oL antibody 02C09 and a light chain variable region (SEQ ID NO: 38) of anti-OX₄oL antibody 02C09 are connected through a linker (SEQ ID NO: 32), was connected at C-terminus of a heavy chain constant region of TNFai antibody (Humira) through a linker (SEQ ID NO: 31).

Bispecific antibody TNFαi-02C09 HC was prepared by using an expression vector pcDNA3.1 (Invitrogen) and an animal cell line of FreeStyle^{™} 293-F. Detailed culture conditions, culture methods, and purification methods were performed in substantially the same manner as those described above in (1) anti-OX₄oL antibody 02C09.

**[Table 20] Heavy chain of TNFαi-02C09 HC**

| TNFai heavy chain-linker- anti-OX₄oL antibody 02C09 variable region | |
|---|---|
| Amino acid sequence (SEQ ID NO: 65) | EVQLVESGGGLVQPGRSLRLSCAASGFTFDDYAMHWVRQAPGKGLEW VSAITWNSGHIDYADSVEGRFTISRDNAKNSLYLQMNSLRAEDTAVYY CAKVSYLSTASSLDYWGQGTLVTVSSASTKGPSVFPLAPCSRSTSESTAALGC LVKDYFPEPVTVSWNSGALTSGVHTFPAVLQSSGLYSLSSVVTVPSSSLGTKTYTC NVDHKPSNTKVDKRVESKYGPPCPPCPAPEFLGGPSVFLFPPKPKDTLMISRTPE VTCVVVDVSQEDPEVQFNWYVDGVEVHNAKTKPREEQFNSTYRVVSVLTVLHQ DWLNGKEYKCKVSNKGLPSSIEKTISKAKGQPREPQVYTLPPSQEEMTKNQVSLT CLVKGFYPSDIAVEWESNGQPENNYKTTPPVLDSDGSFFLYSRLTVDKSRWQEG NVFSCSVMHEALHNHYTQKSLSLSLGKGGGGSGGGGSGGGGSQMQLVQSGG GLVKPGGSLRVSCAASGFTFNYAWMSWVRQAPGKGLEWVGRIKGEPDGGTTEY ADSVKGRFTISRDNSKNTLYLQMNSLRAEDTAVYYCVSRRGLDVWGQGTTVTVS SGGGGSGGGGSGGGGSGGGGSDIQMTQSPSSLSASLGDRVTITCRASQSIGR WLAWYQQKPGNAPKLLIYKATILESGVPSRFSGSGSGTEFALTISSLQPDDFSAYY CQQYNSYPYTFGQGTKLEIK |
| Nucleic acid (DNA) sequence (SEQ ID NO: 66) | GAAGTGCAACTGGTTGAATCTGGCGGAGGACTGGTGCAGCCTGGAAGA AGCCTGAGACTGTCTTGTGCCGCCAGCGGCTTCACCTTCGATGATTATG CCATGCACTGGGTCCGACAGGCCCCTGGAAAAGGACTTGAATGGGTGT CCGCCATCACCTGGAACAGCGGCCACATCGATTACGCCGATAGCGTGG AAGGCCGGTTCACCATCAGCAGAGACAACGCCAAGAACAGCCTGTACC TGCAGATGAACTCCCTGAGAGCCGAGGACACCGCCGTGTACTACTGTG CCAAAGTGTCTTACCTGAGCACCGCCTCCAGCCTGGATTATTGGGGACA GGGCACACTGGTCACAGTGTCCTCTGCTTCCACCAAGGGCCCATCCGTCTTC CCCCTGGCGCCCTGCTCCAGGAGCACCTCCGAGAGCACAGCCGCCCTGGGCTGC CTGGTCAAGGACTACTTCCCCGAACCGGTGACGGTGTCGTGGAACTCAGGCGCC |
| | CTGACCAGCGGCGTGCACACCTTCCCGGCTGTCCTACAGTCCTCAGGACTCTACT CCCTCAGCAGCGTGGTGACCGTGCCCTCCAGCAGCTTGGGCACGAAGACCTACA CCTGCAACGTAGATCACAAGCCCAGCAACACCAAGGTGGACAAGAGAGTTGAGT CCAAATATGGTCCCCCATGCCCACCATGCCCAGCACCTGAGTTCCTGGGGGGAC CATCAGTCTTCCTGTTCCCCCCAAAACCCAAGGACACTCTCATGATCTCCCGGAC CCCTGAGGTCACGTGCGTGGTGGTGGACGTGAGCCAGGAAGACCCCGAGGTCC AGTTCAACTGGTACGTGGATGGCGTGGAGGTGCATAATGCCAAGACAAAGCCGC GGGAGGAGCAGTTCAACAGCACGTACCGTGTGGTCAGCGTCCTCACCGTCCTGC ACCAGGACTGGCTGAACGGCAAGGAGTACAAGTGCAAGGTCTCCAACAAAGGCC TCCCGTCCTCCATCGAGAAAACCATCTCCAAAGCCAAAGGGCAGCCCCGAGAGC CACAGGTGTACACCCTGCCCCCATCCCAGGAGGAGATGACCAAGAACCAGGTCA GCCTGACCTGCCTGGTCAAAGGCTTCTACCCCAGCGACATCGCCGTGGAGTGGG AGAGCAATGGGCAGCCGGAGAACAACTACAAGACCACGCCTCCCGTGCTGGACT CCGACGGCTCCTTCTTCCTCTACAGCAGGCTAACCGTGGACAAGAGCAGGTGGC AGGAGGGGAATGTCTTCTCATGCTCCGTGATGCATGAGGCTCTGCACAACCACT ACACACAGAAGAGCCTCTCCCTGTCTCTGGGTAAAGGTGGTGGCGGATCTGG CGGCGGAGGAAGCGGAGGCGGAGGCTCCCAAATGCAGCTTGTTCAATCCG GCGGAGGCCTGGTTAAGCCTGGCGGATCTCTGAGAGTGTCTTGTGCCGCCAGCG GCTTCACCTTCAATTACGCCTGGATGAGCTGGGTCCGACAGGCCCCTGGAAAAG GACTGGAATGGGTCGGAAGAATCAAGGGCGAGCCTGATGGCGGAACCACAGAG TACGCCGATAGCGTGAAGGGCAGATTCACCATCAGCCGGGACAACAGCAAGAAC ACCCTGTACCTGCAGATGAACTCCCTGAGAGCCGAGGACACAGCCGTGTACTAC TGTGTGTCTAGAAGAGGCCTGGATGTGTGGGGCCAGGGAACAACAGTGACAGT TTCTTCTGGTGGCGGAGGTTCTGGCGGAGGTGGTAGTGGTGGCGGTGG AAGTGGTGGCGGCGGATCTGATATCCAGATGACACAGAGCCCTAGCAGCCTG TCTGCCTCTCTGGGCGATAGAGTGACCATCACATGTCGGGCCAGCCAGTCTATT GGACGGTGGCTGGCCTGGTATCAGCAAAAGCCTGGAAACGCCCCTAAGCTGCTG |
| | ATCTACAAGGCCACCATCCTGGAAAGCGGAGTGCCTTCTAGATTTTCTGGCAGC GGCTCTGGCACCGAGTTTGCCCTGACAATTAGCTCCCTGCAGCCTGACGACTTCA GCGCCTACTACTGCCAGCAGTACAACAGCTACCCCTACACCTTCGGCCAGGGCAC AAAGCTGGAAATCAAG |

### (6) Bispecific antibody TNFαi-02C09 LC

In case of TNFαi-02C09 LC, a binding fragment (ScFv) of an anti-OX₄oL antibody 02C09, in which a heavy chain variable region (SEQ ID NO: 37) of anti-OX₄oL antibody 02C09 and a light chain variable region (SEQ ID NO: 38) of anti-OX40L antibody 02C09 are connected through a linker (SEQ ID NO: 32), was connected at C-terminus of a light chain constant region of TNFai antibody through a linker (SEQ ID NO: 31).

Bispecific antibody TNFαi-02C09 LC was prepared by using an expression vector pcDNA3.1 (Invitrogen) and an animal cell line of FreeStyle^{™} 293-F. Detailed culture conditions, culture methods, and purification methods were performed in substantially the same manner as those described above in (1) anti-OX₄oL antibody 02C09.

**[Table 21] Light chain of TNFαi-02C09 LC**

| TNFai light chain-linker- anti-OX₄oL antibody 02C09 variable region | |
|---|---|
| Amino acid sequence (SEQ ID NO: 67) | DIQMTQSPSSLSASVGDRVTITCRASQGIRNYLAWYQQKPGKAPKLLIYA ASTLQSGVPSRFSGSGSGTDFTLTISSLQPEDVATYYCQRYNRAPYTFGQG TKVEIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNRGEC |
| | GGGGSGGGGSGGGGSQMQLVQSGGGLVKPGGSLRVSCAASGFTFNYAWMSWV RQAPGKGLEWVGRIKGEPDGGTTEYADSVKGRFTISRDNSKNTLYLQMNSLRAED TAVYYCVSRRGLDVWGQGTTVTVSSGGGGSGGGGSGGGGSGGGGSDIQMTQS PSSLSASLGDRVTITCRASQSIGRWLAWYQQKPGNAPKLLIYKATILESGVPSRFSGS GSGTEFALTISSLQPDDFSAYYCQQYNSYPYTFGQGTKLEIK |
| Nucleic acid (DNA) sequence (SEQ ID NO: 68) | GATATCCAGATGACACAGAGCCCTAGCAGCCTGTCTGCCTCTGTGGGCG ATAGAGTGACCATCACCTGTAGAGCCAGCCAGGGCATCAGAAACTACCT GGCCTGGTATCAGCAGAAGCCCGGAAAGGCCCCTAAGCTGCTGATCTAT GCCGCCTCTACACTGCAGAGCGGCGTGCCATCTAGATTTTCTGGCAGCGG CTCTGGCACCGACTTCACCCTGACAATTTCTAGCCTGCAGCCTGAGGACG TGGCCACCTACTACTGCCAGAGATACAACAGAGCCCCTTACACCTTCGGC CAGGGCACCAAGGTGGAAATCAAGCGTACGGTGGCGGCGCCATCTGTCTTCA TCTTCCCGCCATCTGATGAGCAGTTGAAATCTGGAACTGCCTCTGTTGTGTGCCTG CTGAATAACTTCTATCCCAGAGAGGCCAAAGTACAGTGGAAGGTGGATAACGCCC TCCAATCGGGTAACTCCCAGGAGAGTGTCACAGAGCAGGACAGCAAGGACAGCAC CTACAGCCTCAGCAGCACCCTGACGCTGAGCAAAGCAGACTACGAGAAACACAAA GTCTACGCCTGCGAAGTCACCCATCAGGGCCTGAGCTCGCCCGTCACAAAGAGCT TCAACAGGGGAGAGTGTGGTGGTGGCGGATCTGGCGGCGGAGGAAGCGGA GGCGGAGGCTCCCAAATGCAGCTTGTTCAATCCGGCGGAGGCCTGGTTAAGCCT GGCGGATCTCTGAGAGTGTCTTGTGCCGCCAGCGGCTTCACCTTCAATTACGCCTG GATGAGCTGGGTCCGACAGGCCCCTGGAAAAGGACTGGAATGGGTCGGAAGAAT CAAGGGCGAGCCTGATGGCGGAACCACAGAGTACGCCGATAGCGTGAAGGGCAG ATTCACCATCAGCCGGGACAACAGCAAGAACACCCTGTACCTGCAGATGAACTCCC TGAGAGCCGAGGACACAGCCGTGTACTACTGTGTGTCTAGAAGAGGCCTGGATGT GTGGGGCCAGGGAACAACAGTGACAGTTTCTTCTGGTGGCGGAGGTTCTGGC GGAGGTGGTAGTGGTGGCGGTGGAAGTGGTGGCGGCGGATCTGATATCC AGATGACACAGAGCCCTAGCAGCCTGTCTGCCTCTCTGGGCGATAGAGTGACCAT |
| | CACATGTCGGGCCAGCCAGTCTATTGGACGGTGGCTGGCCTGGTATCAGCAAAAG CCTGGAAACGCCCCTAAGCTGCTGATCTACAAGGCCACCATCCTGGAAAGCGGAG TGCCTTCTAGATTTTCTGGCAGCGGCTCTGGCACCGAGTTTGCCCTGACAATTAGC TCCCTGCAGCCTGACGACTTCAGCGCCTACTACTGCCAGCAGTACAACAGCTACCC CTACACCTTCGGCCAGGGCACAAAGCTGGAAATCAAG |

### (7) Bispecific antibody TNFαi-hu3F07 HC

In case of TNFαi-hu3F07 HC, a binding fragment (ScFv) of an anti-OX₄oL antibody hu3F07, in which a heavy chain variable region (SEQ ID NO: 41) of anti-OX40L antibody hu3F07 and a light chain variable region (SEQ ID NO: 42) of anti-OX₄oL antibody hu3F07 are connected through a linker (SEQ ID NO: 32), was connected at C-terminus of a heavy chain constant region of TNFai antibody through a linker (SEQ ID NO: 31).

Bispecific antibody TNFαi-hu3F07 HC was prepared by using an expression vector pcDNA3.1 (Invitrogen) and an animal cell line of FreeStyle^{™} 293-F. Detailed culture conditions, culture methods, and purification methods were performed in substantially the same manner as those described above in (1) anti-OX₄oL antibody 02C09.

**[Table 22] Heavy chain of TNFαi-hu3F07 HC**

| TNFai heavy chain-linker- anti-OX₄oL antibody hu3F07 variable region | |
|---|---|
| Amino acid sequence | EVQLVESGGGLVQPGRSLRLSCAASGFTFDDYAMHWVRQAPGKGLEW VSAITWNSGHIDYADSVEGRFTISRDNAKNSLYLQMNSLRAEDTAVYY CAKVSYLSTASSLDYWGQGTLVTVSSASTKGPSVFPLAPCSRSTSESTAALGC |
| (SEQ ID NO: 69) | LVKDYFPEPVTVSWNSGALTSGVHTFPAVLQSSGLYSLSSVVTVPSSSLGTKTYTC NVDHKPSNTKVDKRVESKYGPPCPPCPAPEFLGGPSVFLFPPKPKDTLMISRTPE VTCVVVDVSQEDPEVQFNWYVDGVEVHNAKTKPREEQFNSTYRVVSVLTVLHQ DWLNGKEYKCKVSNKGLPSSIEKTISKAKGQPREPQVYTLPPSQEEMTKNQVSLT CLVKGFYPSDIAVEWESNGQPENNYKTTPPVLDSDGSFFLYSRLTVDKSRWQEG NVFSCSVMHEALHNHYTQKSLSLSLGKGGGGSGGGGSGGGGSQVQLVQSGA EVKKPGASVKVSCKASGFTFPDYWMHWVRQAPGQGLEWMGEIDPPNGRTNYN EKFKSRVTMTRDTSISTAYMELSRLRSDDTAVYYCARGIYYVDYWGQGTTVTVSS GGGGSGGGGSGGGGSGGGGSDIVMTQSPLSLPVTLGEPASISCRSSQSLVHSN GNTYLHWYLQKPGQSPQLLIYKVSNRFSGVPDRFSGSGSGTDFTLKISRVEAEDV GVYYCSQSTHVPPTFGGGTKLEIK |
| Nucleic acid (DNA) sequence (SEQ ID NO: 70) | GAAGTGCAACTGGTTGAATCTGGCGGAGGACTGGTGCAGCCTGGAAGA AGCCTGAGACTGTCTTGTGCCGCCAGCGGCTTCACCTTCGATGATTATG CCATGCACTGGGTCCGACAGGCCCCTGGAAAAGGACTTGAATGGGTGT CCGCCATCACCTGGAACAGCGGCCACATCGATTACGCCGATAGCGTGG AAGGCCGGTTCACCATCAGCAGAGACAACGCCAAGAACAGCCTGTACC TGCAGATGAACTCCCTGAGAGCCGAGGACACCGCCGTGTACTACTGTG CCAAAGTGTCTTACCTGAGCACCGCCTCCAGCCTGGATTATTGGGGACA GGGCACACTGGTCACAGTGTCCTCTGCTTCCACCAAGGGCCCATCCGTCTTC CCCCTGGCGCCCTGCTCCAGGAGCACCTCCGAGAGCACAGCCGCCCTGGGCTGC CTGGTCAAGGACTACTTCCCCGAACCGGTGACGGTGTCGTGGAACTCAGGCGCC CTGACCAGCGGCGTGCACACCTTCCCGGCTGTCCTACAGTCCTCAGGACTCTACT CCCTCAGCAGCGTGGTGACCGTGCCCTCCAGCAGCTTGGGCACGAAGACCTACA CCTGCAACGTAGATCACAAGCCCAGCAACACCAAGGTGGACAAGAGAGTTGAGT CCAAATATGGTCCCCCATGCCCACCATGCCCAGCACCTGAGTTCCTGGGGGGAC CATCAGTCTTCCTGTTCCCCCCAAAACCCAAGGACACTCTCATGATCTCCCGGAC CCCTGAGGTCACGTGCGTGGTGGTGGACGTGAGCCAGGAAGACCCCGAGGTCC |
| | |
| | AGTTCAACTGGTACGTGGATGGCGTGGAGGTGCATAATGCCAAGACAAAGCCGC GGGAGGAGCAGTTCAACAGCACGTACCGTGTGGTCAGCGTCCTCACCGTCCTGC ACCAGGACTGGCTGAACGGCAAGGAGTACAAGTGCAAGGTCTCCAACAAAGGCC TCCCGTCCTCCATCGAGAAAACCATCTCCAAAGCCAAAGGGCAGCCCCGAGAGC CACAGGTGTACACCCTGCCCCCATCCCAGGAGGAGATGACCAAGAACCAGGTCA GCCTGACCTGCCTGGTCAAAGGCTTCTACCCCAGCGACATCGCCGTGGAGTGGG AGAGCAATGGGCAGCCGGAGAACAACTACAAGACCACGCCTCCCGTGCTGGACT CCGACGGCTCCTTCTTCCTCTACAGCAGGCTAACCGTGGACAAGAGCAGGTGGC AGGAGGGGAATGTCTTCTCATGCTCCGTGATGCATGAGGCTCTGCACAACCACT ACACACAGAAGAGCCTCTCCCTGTCTCTGGGTAAAGGTGGTGGCGGATCTGG CGGCGGAGGAAGCGGAGGCGGAGGCTCCCAAGTTCAACTGGTTCAGTCTG GCGCCGAAGTGAAGAAACCAGGCGCCAGCGTGAAGGTGTCCTGCAAGGCCTCT GGCTTCACATTCCCCGACTACTGGATGCACTGGGTTCGACAGGCTCCAGGACAG GGACTCGAATGGATGGGCGAGATCGATCCTCCTAACGGCCGGACCAACTACAAC GAGAAGTTCAAGAGCAGAGTGACCATGACCAGAGACACCAGCATCAGCACCGCC TACATGGAACTGAGCAGACTGAGAAGCGACGACACCGCCGTGTACTACTGTGCC AGAGGCATCTACTACGTGGACTACTGGGGCCAGGGCACCACAGTGACAGTTAGC TCTGGTGGCGGAGGTTCTGGCGGAGGTGGTAGTGGTGGCGGTGGAAGT GGTGGCGGCGGATCTGATATCGTGATGACACAGAGCCCTCTGAGCCTGCCTG TGACACTGGGAGAACCTGCCAGCATCAGCTGTAGAAGCAGCCAGAGCCTGGTGC ACAGCAACGGCAATACCTACCTGCACTGGTATCTGCAGAAGCCCGGACAGTCTC CCCAGCTGCTGATCTACAAGGTGTCCAACAGATTCAGCGGCGTGCCCGATAGAT TTTCTGGCAGCGGCTCTGGCACCGACTTCACCCTGAAGATTAGCAGAGTGGAAG CCGAGGACGTGGGCGTGTACTACTGTAGCCAGTCTACCCACGTGCCACCTACCT TTGGCGGCGGAACAAAGCTGGAAATCAAG |

### (8) Bispecific antibody TNFαi-hu3F07 LC

In case of TNFαi-hu3F07 LC, a binding fragment (ScFv) of an anti-OX₄oL antibody hu3F07, in which a heavy chain variable region (SEQ ID NO: 41) of anti-OX₄oL antibody hu3F07 and a light chain variable region (SEQ ID NO: 42) of anti-OX₄oL antibody hu3F07 are connected through a linker (SEQ ID NO: 32), was connected at C-terminus of a light chain constant region of TNFai antibody through a linker (SEQ ID NO: 31).

Bispecific antibody TNFαi-02C09 LC was prepared by using an expression vector pcDNA3.1 (Invitrogen) and an animal cell line of FreeStyle^{™} 293-F. Detailed culture conditions, culture methods, and purification methods were performed in substantially the same manner as those described above in (1) anti-OX₄oL antibody 02C09.

**[Table 23] Light chain of TNFαi-hu3F07 LC**

| TNFai light chain-linker- anti-OX₄oL antibody hu3F07 variable region | |
|---|---|
| Amino acid sequence (SEQ ID NO: 71) | DIQMTQSPSSLSASVGDRVTITCRASQGIRNYLAWYQQKPGKAPKLLIY AASTLQSGVPSRFSGSGSGTDFTLTISSLQPEDVATYYCQRYNRAPYTFG QGTKVEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSPVTKSF NRGECGGGGSGGGGSGGGGSQVQLVQSGAEVKKPGASVKVSCKASGFTFPDY WMHWVRQAPGQGLEWMGEIDPPNGRTNYNEKFKSRVTMTRDTSISTAYMELS RLRSDDTAVYYCARGIYYVDYWGQGTTVTVSSGGGGSGGGGSGGGGSGGGG SDIVMTQSPLSLPVTLGEPASISCRSSQSLVHSNGNTYLHWYLQKPGQSPQLLIYK VSNRFSGVPDRFSGSGSGTDFTLKISRVEAEDVGVYYCSQSTHVPPTFGGGTKLEI K |
| Nucleic acid (DNA) sequence (SEQ ID NO: 72) | GATATCCAGATGACACAGAGCCCTAGCAGCCTGTCTGCCTCTGTGGGCG ATAGAGTGACCATCACCTGTAGAGCCAGCCAGGGCATCAGAAACTACC TGGCCTGGTATCAGCAGAAGCCCGGAAAGGCCCCTAAGCTGCTGATCT ATGCCGCCTCTACACTGCAGAGCGGCGTGCCATCTAGATTTTCTGGCAG CGGCTCTGGCACCGACTTCACCCTGACAATTTCTAGCCTGCAGCCTGAG GACGTGGCCACCTACTACTGCCAGAGATACAACAGAGCCCCTTACACCT TCGGCCAGGGCACCAAGGTGGAAATCAAGCGTACGGTGGCGGCGCCATCT GTCTTCATCTTCCCGCCATCTGATGAGCAGTTGAAATCTGGAACTGCCTCTGTTG TGTGCCTGCTGAATAACTTCTATCCCAGAGAGGCCAAAGTACAGTGGAAGGTGG ATAACGCCCTCCAATCGGGTAACTCCCAGGAGAGTGTCACAGAGCAGGACAGCA AGGACAGCACCTACAGCCTCAGCAGCACCCTGACGCTGAGCAAAGCAGACTACG AGAAACACAAAGTCTACGCCTGCGAAGTCACCCATCAGGGCCTGAGCTCGCCCG TCACAAAGAGCTTCAACAGGGGAGAGTGTGGTGGTGGCGGATCTGGCGGCG GAGGAAGCGGAGGCGGAGGCTCCCAAGTTCAACTGGTTCAGTCTGGCGCCG AAGTGAAGAAACCAGGCGCCAGCGTGAAGGTGTCCTGCAAGGCCTCTGGCTTCA CATTCCCCGACTACTGGATGCACTGGGTTCGACAGGCTCCAGGACAGGGACTCG AATGGATGGGCGAGATCGATCCTCCTAACGGCCGGACCAACTACAACGAGAAGT TCAAGAGCAGAGTGACCATGACCAGAGACACCAGCATCAGCACCGCCTACATGG AACTGAGCAGACTGAGAAGCGACGACACCGCCGTGTACTACTGTGCCAGAGGCA TCTACTACGTGGACTACTGGGGCCAGGGCACCACAGTGACAGTTAGCTCTGGTG GCGGAGGTTCTGGCGGAGGTGGTAGTGGTGGCGGTGGAAGTGGTGGC GGCGGATCTGATATCGTGATGACACAGAGCCCTCTGAGCCTGCCTGTGACACT GGGAGAACCTGCCAGCATCAGCTGTAGAAGCAGCCAGAGCCTGGTGCACAGCAA CGGCAATACCTACCTGCACTGGTATCTGCAGAAGCCCGGACAGTCTCCCCAGCT GCTGATCTACAAGGTGTCCAACAGATTCAGCGGCGTGCCCGATAGATTTTCTGG CAGCGGCTCTGGCACCGACTTCACCCTGAAGATTAGCAGAGTGGAAGCCGAGGA CGTGGGCGTGTACTACTGTAGCCAGTCTACCCACGTGCCACCTACCTTTGGCGG |
| | CGGAACAAAGCTGGAAATCAAG |

### (9) Bispecific antibody I3F07-TNFαi HC

In case of above bispecific antibody I3F07-TNFαi HC, a binding fragment (ScFv) of TNFai antibody, in which a heavy chain variable region (SEQ ID NO: 35) of TNFai antibody (Humira) and a light chain variable region (SEQ ID NO: 36) of TNFai antibody (Humira) are connected through a linker (SEQ ID NO: 32), was connected at C-terminus of a heavy chain constant region of anti-OX₄oL antibody I3F07 through a linker (SEQ ID NO: 31).

Bispecific antibody I3F07-TNFαi HC was prepared by using an expression vector pcDNA3.1 (Invitrogen) and an animal cell line of FreeStyle^{™} 293-F (Invitrogen). Detailed culture conditions, culture methods, and purification methods were performed in substantially the same manner as those described above in (1) anti-OX₄oL antibody 02C09.

**[Table 24] Heavy chain of I3F07-TNFαi HC**

| I3F07 heavy chain-linker-TNFai variable region | |
|---|---|
| Amino acid sequence (SEQ ID NO: 73) | QIQLAQSGAELVKPGASVKLSCKASGFTFPDYWMHWVKQRPGQGLE WIGEIDPPNGRTNYNEKFKSKATLTVDSSSNTAYMQLSRLTSEDSAVYY CARGIYYVDYWGQGTTLTVSSASTKGPSVFPLAPCSRSTSESTAALGCLVKDY FPEPVTVSWNSGALTSGVHTFPAVLQSSGLYSLSSVVTVPSSSLGTKTYTCNVDHK PSNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLNG |
| | KEYKCKVSNKGLPSSIEKTISKAKGQPREPQVYTLPPSQEEMTKNQVSLTCLVKGF YPSDIAVEWESNGQPENNYKTTPPVLDSDGSFFLYSRLTVDKSRWQEGNVFSCSV MHEALHNHYTQKSLSLSLGKGGGGSGGGGSGGGGSEVQLVESGGGLVQPGR SLRLSCAASGFTFDDYAMHWVRQAPGKGLEWVSAITWNSGHIDYADSVEGRFTI SRDNAKNSLYLQMNSLRAEDTAVYYCAKVSYLSTASSLDYWGQGTLVTVSSGGG GSGGGGSGGGGSGGGGSDIQMTQSPSSLSASVGDRVTITCRASQGIRNYLAW YQQKPGKAPKLLIYAASTLQSGVPSRFSGSGSGTDFTLTISSLQPEDVATYYCQRY NRAPYTFGQGTKVEIK |
| Nucleic acid (DNA) sequence (SEQ ID NO: 74) | CAGATTCAGCTGGCTCAGTCTGGCGCCGAACTTGTGAAACCTGGCGCCT CTGTGAAGCTGAGCTGCAAGGCCAGCGGCTTCACCTTTCCTGACTACTG GATGCACTGGGTCAAGCAGAGGCCTGGACAGGGACTCGAATGGATCGG CGAGATCGATCCTCCTAACGGCCGGACCAACTACAACGAGAAGTTCAA GAGCAAGGCCACACTGACCGTGGACAGCAGCAGCAATACCGCCTACAT GCAGCTGAGCAGACTGACCTCTGAGGACAGCGCCGTGTACTACTGTGC CAGAGGCATCTACTACGTGGACTACTGGGGCCAGGGCACAACCCTGAC AGTTTCTTCTGCTTCCACCAAGGGCCCATCCGTCTTCCCCCTGGCGCCCTGCTC CAGGAGCACCTCCGAGAGCACAGCCGCCCTGGGCTGCCTGGTCAAGGACTACTT CCCCGAACCGGTGACGGTGTCGTGGAACTCAGGCGCCCTGACCAGCGGCGTGCA CACCTTCCCGGCTGTCCTACAGTCCTCAGGACTCTACTCCCTCAGCAGCGTGGTG ACCGTGCCCTCCAGCAGCTTGGGCACGAAGACCTACACCTGCAACGTAGATCAC AAGCCCAGCAACACCAAGGTGGACAAGAGAGTTGAGTCCAAATATGGTCCCCCA TGCCCACCATGCCCAGCACCTGAGTTCCTGGGGGGACCATCAGTCTTCCTGTTCC CCCCAAAACCCAAGGACACTCTCATGATCTCCCGGACCCCTGAGGTCACGTGCGT GGTGGTGGACGTGAGCCAGGAAGACCCCGAGGTCCAGTTCAACTGGTACGTGG ATGGCGTGGAGGTGCATAATGCCAAGACAAAGCCGCGGGAGGAGCAGTTCAAC AGCACGTACCGTGTGGTCAGCGTCCTCACCGTCCTGCACCAGGACTGGCTGAAC GGCAAGGAGTACAAGTGCAAGGTCTCCAACAAAGGCCTCCCGTCCTCCATCGAG |
| | AAAACCATCTCCAAAGCCAAAGGGCAGCCCCGAGAGCCACAGGTGTACACCCTG CCCCCATCCCAGGAGGAGATGACCAAGAACCAGGTCAGCCTGACCTGCCTGGTC AAAGGCTTCTACCCCAGCGACATCGCCGTGGAGTGGGAGAGCAATGGGCAGCC GGAGAACAACTACAAGACCACGCCTCCCGTGCTGGACTCCGACGGCTCCTTCTTC CTCTACAGCAGGCTAACCGTGGACAAGAGCAGGTGGCAGGAGGGGAATGTCTTC TCATGCTCCGTGATGCATGAGGCTCTGCACAACCACTACACACAGAAGAGCCTCT CCCTGTCTCTGGGTAAAGGTGGTGGCGGATCTGGCGGCGGAGGAAGCGG AGGCGGAGGCTCCGAAGTGCAACTGGTTGAATCTGGCGGAGGACTGGTGCAG CCTGGAAGAAGCCTGAGACTGTCTTGTGCCGCCAGCGGCTTCACCTTCGATGAT TATGCCATGCACTGGGTCCGACAGGCCCCTGGAAAAGGACTTGAATGGGTGTCC GCCATCACCTGGAACAGCGGCCACATCGATTACGCCGATAGCGTGGAAGGCCGG TTCACCATCAGCAGAGACAACGCCAAGAACAGCCTGTACCTGCAGATGAACTCC CTGAGAGCCGAGGACACCGCCGTGTACTACTGTGCCAAAGTGTCTTACCTGAGC ACCGCCTCCAGCCTGGATTATTGGGGACAGGGCACACTGGTCACAGTGTCCTCT GGTGGCGGAGGTTCTGGCGGAGGTGGTAGTGGTGGCGGTGGAAGTGG TGGCGGCGGATCTGATATCCAGATGACACAGAGCCCTAGCAGCCTGTCTGCCT CTGTGGGCGATAGAGTGACCATCACCTGTAGAGCCAGCCAGGGCATCAGAAACT ACCTGGCCTGGTATCAGCAGAAGCCCGGAAAGGCCCCTAAGCTGCTGATCTATG CCGCCTCTACACTGCAGAGCGGCGTGCCATCTAGATTTTCTGGCAGCGGCTCTG GCACCGACTTCACCCTGACAATTTCTAGCCTGCAGCCTGAGGACGTGGCCACCTA CTACTGCCAGAGATACAACAGAGCCCCTTACACCTTCGGCCAGGGCACCAAGGT GGAAATCAAG |

### (10) Bispecific antibody I3F07-TNFαi LC

In case of I3F07-TNFαi LC, a binding fragment (ScFv) of TNFai antibody, in which a heavy chain variable region (SEQ ID NO: 35) of Humira and a light chain variable region (SEQ ID NO: 36) of Humira are connected through a linker (SEQ ID NO: 32), was connected at C-terminus of a light chain constant region of anti-OX₄oL antibody I3F07 through a linker (SEQ ID NO: 31).

Bispecific antibody I3F07-TNFαi LC was prepared by using an expression vector pcDNA3.1 (Invitrogen) and an animal cell line of FreeStyle^{™} 293-F (Invitrogen). Detailed culture conditions, culture methods, and purification methods were performed in substantially the same manner as those described above in (1) anti-OX₄oL antibody 02C09.

**[Table 25] Light chain of I3F07-TNFαi LC**

| I3F07 light chain-linker-TNFai variable region | |
|---|---|
| Amino acid sequence (SEQ ID NO: 75) | DIVLTQSPLSLPVSLGDQASISCRSSQSLVHSNGNTYLHWYLQKPGQSP KLLIYKVSNRFSGVPDRFSGSGSGTDFTLKISRVEAEDLGVYFCSQSTHV PPTFGGGSKLEIKRTVAAPSVFIFPPSDEQLKSGTASVVCLLNNFYPREAKVQW KVDNALQSGNSQESVTEQDSKDSTYSLSSTLTLSKADYEKHKVYACEVTHQGLSS PVTKSFNRGECGGGGSGGGGSGGGGSEVQLVESGGGLVQPGRSLRLSCAASG FTFDDYAMHWVRQAPGKGLEWVSAITWNSGHIDYADSVEGRFTISRDNAKNSL YLQMNSLRAEDTAVYYCAKVSYLSTASSLDYWGQGTLVTVSSGGGGSGGGGSG GGGSGGGGSDIQMTQSPSSLSASVGDRVTITCRASQGIRNYLAWYQQKPGKAP KLLIYAASTLQSGVPSRFSGSGSGTDFTLTISSLQPEDVATYYCQRYNRAPYTFGQG TKVEIK |
| Nucleic acid (DNA) sequence (SEQ ID NO: 76) | GATATCGTGCTGACACAGAGCCCTCTGAGCCTGCCTGTTTCTCTGGGAG ATCAGGCCAGCATCAGCTGCAGATCCTCTCAGAGCCTGGTGCACAGCA ACGGCAATACCTACCTGCACTGGTATCTGCAGAAGCCCGGCCAGTCTCC TAAGCTGCTGATCTACAAGGTGTCCAACAGGTTCAGCGGCGTGCCCGA |
| | TAGATTTTCTGGCTCTGGCAGCGGCACCGACTTCACCCTGAAGATTTCT AGAGTGGAAGCCGAGGACCTGGGCGTGTACTTCTGTTCTCAGTCTACCC ACGTGCCACCTACCTTTGGCGGCGGAAGCAAGCTGGAAATCAAGCGTAC GGTGGCGGCGCCATCTGTCTTCATCTTCCCGCCATCTGATGAGCAGTTGAAATCT GGAACTGCCTCTGTTGTGTGCCTGCTGAATAACTTCTATCCCAGAGAGGCCAAA GTACAGTGGAAGGTGGATAACGCCCTCCAATCGGGTAACTCCCAGGAGAGTGTC ACAGAGCAGGACAGCAAGGACAGCACCTACAGCCTCAGCAGCACCCTGACGCTG AGCAAAGCAGACTACGAGAAACACAAAGTCTACGCCTGCGAAGTCACCCATCAG GGCCTGAGCTCGCCCGTCACAAAGAGCTTCAACAGGGGAGAGTGTGGTGGTG GCGGATCTGGCGGCGGAGGAAGCGGAGGCGGAGGCTCCGAAGTGCAAC TGGTTGAATCTGGCGGAGGACTGGTGCAGCCTGGAAGAAGCCTGAGACTGTCTT GTGCCGCCAGCGGCTTCACCTTCGATGATTATGCCATGCACTGGGTCCGACAGG CCCCTGGAAAAGGACTTGAATGGGTGTCCGCCATCACCTGGAACAGCGGCCACA TCGATTACGCCGATAGCGTGGAAGGCCGGTTCACCATCAGCAGAGACAACGCCA AGAACAGCCTGTACCTGCAGATGAACTCCCTGAGAGCCGAGGACACCGCCGTGT ACTACTGTGCCAAAGTGTCTTACCTGAGCACCGCCTCCAGCCTGGATTATTGGG GACAGGGCACACTGGTCACAGTGTCCTCTGGTGGCGGAGGTTCTGGCGGAG GTGGTAGTGGTGGCGGTGGAAGTGGTGGCGGCGGATCTGATATCCAGAT GACACAGAGCCCTAGCAGCCTGTCTGCCTCTGTGGGCGATAGAGTGACCATCAC CTGTAGAGCCAGCCAGGGCATCAGAAACTACCTGGCCTGGTATCAGCAGAAGCC CGGAAAGGCCCCTAAGCTGCTGATCTATGCCGCCTCTACACTGCAGAGCGGCGT GCCATCTAGATTTTCTGGCAGCGGCTCTGGCACCGACTTCACCCTGACAATTTCT AGCCTGCAGCCTGAGGACGTGGCCACCTACTACTGCCAGAGATACAACAGAGCC CCTTACACCTTCGGCCAGGGCACCAAGGTGGAAATCAAG |

### (11) Bispecific antibody TNFαi-I3F07 HC

In case of TNFαi-I3F07 HC, a binding fragment (ScFv) of an anti-OX₄oL antibody I3F07, in which a heavy chain variable region (SEQ ID NO: 53) of anti-OX₄oL antibody I3F07 and a light chain variable region (SEQ ID NO: 54) of anti-OX₄oL antibody I3F07 are connected through a linker (SEQ ID NO: 32), was connected at C-terminus of a heavy chain constant region of TNFai antibody through a linker (SEQ ID NO: 31).

Bispecific antibody TNFαi-I3F07 HC was prepared by using an expression vector pcDNA3.1 (Invitrogen) and an animal cell line of FreeStyle^{™} 293-F. Detailed culture conditions, culture methods, and purification methods were performed in substantially the same manner as those described above in (1) anti-OX40L antibody 02C09.

**[Table 26] Heavy chain of TNFαi-I3F07 HC**

| TNFai heavy chain-linker-anti-OX40L antibody I3F07 variable region | |
|---|---|
| Amino acid sequence (SEQ ID NO: 77) | EVQLVESGGGLVQPGRSLRLSCAASGFTFDDYAMHWVRQAPGKGLEW VSAITWNSGHIDYADSVEGRFTISRDNAKNSLYLQMNSLRAEDTAVYY CAKVSYLSTASSLDYWGQGTLVTVSSASTKGPSVFPLAPCSRSTSESTAALGC LVKDYFPEPVTVSWNSGALTSGVHTFPAVLQSSGLYSLSSVVTVPSSSLGTKTYTC NVDHKPSNTKVDKRVESKYGPPCPPCPAPEFLGGPSVFLFPPKPKDTLMISRTPE VTCVVVDVSQEDPEVQFNWYVDGVEVHNAKTKPREEQFNSTYRVVSVLTVLHQ DWLNGKEYKCKVSNKGLPSSIEKTISKAKGQPREPQVYTLPPSQEEMTKNQVSLT CLVKGFYPSDIAVEWESNGQPENNYKTTPPVLDSDGSFFLYSRLTVDKSRWQEG NVFSCSVMHEALHNHYTQKSLSLSLGKGGGGSGGGGSGGGGSQIQLAQSGAE LVKPGASVKLSCKASGFTFPDYWMHWVKQRPGQGLEWIGEIDPPNGRTNYNEK FKSKATLTVDSSSNTAYMQLSRLTSEDSAVYYCARGIYYVDYWGQGTTLTVSSGG GGSGGGGSGGGGSGGGGSDIVLTQSPLSLPVSLGDQASISCRSSQSLVHSNGN TYLHWYLQKPGQSPKLLIYKVSNRFSGVPDRFSGSGSGTDFTLKISRVEAEDLGVY |
| | FCSQSTHVPPTFGGGSKLEIK |
| Nucleic acid (DNA) sequence (SEQ ID NO: 78) | GAAGTGCAACTGGTTGAATCTGGCGGAGGACTGGTGCAGCCTGGAAGA AGCCTGAGACTGTCTTGTGCCGCCAGCGGCTTCACCTTCGATGATTATG CCATGCACTGGGTCCGACAGGCCCCTGGAAAAGGACTTGAATGGGTGT CCGCCATCACCTGGAACAGCGGCCACATCGATTACGCCGATAGCGTGG AAGGCCGGTTCACCATCAGCAGAGACAACGCCAAGAACAGCCTGTACC TGCAGATGAACTCCCTGAGAGCCGAGGACACCGCCGTGTACTACTGTG CCAAAGTGTCTTACCTGAGCACCGCCTCCAGCCTGGATTATTGGGGACA GGGCACACTGGTCACAGTGTCCTCTGCTTCCACCAAGGGCCCATCCGTCTTC CCCCTGGCGCCCTGCTCCAGGAGCACCTCCGAGAGCACAGCCGCCCTGGGCTGC CTGGTCAAGGACTACTTCCCCGAACCGGTGACGGTGTCGTGGAACTCAGGCGCC CTGACCAGCGGCGTGCACACCTTCCCGGCTGTCCTACAGTCCTCAGGACTCTACT CCCTCAGCAGCGTGGTGACCGTGCCCTCCAGCAGCTTGGGCACGAAGACCTACA CCTGCAACGTAGATCACAAGCCCAGCAACACCAAGGTGGACAAGAGAGTTGAGT CCAAATATGGTCCCCCATGCCCACCATGCCCAGCACCTGAGTTCCTGGGGGGAC CATCAGTCTTCCTGTTCCCCCCAAAACCCAAGGACACTCTCATGATCTCCCGGAC CCCTGAGGTCACGTGCGTGGTGGTGGACGTGAGCCAGGAAGACCCCGAGGTCC AGTTCAACTGGTACGTGGATGGCGTGGAGGTGCATAATGCCAAGACAAAGCCGC GGGAGGAGCAGTTCAACAGCACGTACCGTGTGGTCAGCGTCCTCACCGTCCTGC ACCAGGACTGGCTGAACGGCAAGGAGTACAAGTGCAAGGTCTCCAACAAAGGCC TCCCGTCCTCCATCGAGAAAACCATCTCCAAAGCCAAAGGGCAGCCCCGAGAGC CACAGGTGTACACCCTGCCCCCATCCCAGGAGGAGATGACCAAGAACCAGGTCA GCCTGACCTGCCTGGTCAAAGGCTTCTACCCCAGCGACATCGCCGTGGAGTGGG AGAGCAATGGGCAGCCGGAGAACAACTACAAGACCACGCCTCCCGTGCTGGACT CCGACGGCTCCTTCTTCCTCTACAGCAGGCTAACCGTGGACAAGAGCAGGTGGC AGGAGGGGAATGTCTTCTCATGCTCCGTGATGCATGAGGCTCTGCACAACCACT |
| | ACACACAGAAGAGCCTCTCCCTGTCTCTGGGTAAAGGTGGTGGCGGATCTGG CGGCGGAGGAAGCGGAGGCGGAGGCTCCCAGATTCAGCTGGCTCAGTCTG GCGCCGAACTTGTGAAACCTGGCGCCTCTGTGAAGCTGAGCTGCAAGGCCAGCG GCTTCACCTTTCCTGACTACTGGATGCACTGGGTCAAGCAGAGGCCTGGACAGG GACTCGAATGGATCGGCGAGATCGATCCTCCTAACGGCCGGACCAACTACAACG AGAAGTTCAAGAGCAAGGCCACACTGACCGTGGACAGCAGCAGCAATACCGCCT ACATGCAGCTGAGCAGACTGACCTCTGAGGACAGCGCCGTGTACTACTGTGCCA GAGGCATCTACTACGTGGACTACTGGGGCCAGGGCACAACCCTGACAGTTTCTT CTGGTGGCGGAGGTTCTGGCGGAGGTGGTAGTGGTGGCGGTGGAAGT GGTGGCGGCGGATCTGATATCGTGCTGACACAGAGCCCTCTGAGCCTGCCTG TTTCTCTGGGAGATCAGGCCAGCATCAGCTGCAGATCCTCTCAGAGCCTGGTGC ACAGCAACGGCAATACCTACCTGCACTGGTATCTGCAGAAGCCCGGCCAGTCTC CTAAGCTGCTGATCTACAAGGTGTCCAACAGGTTCAGCGGCGTGCCCGATAGAT TTTCTGGCTCTGGCAGCGGCACCGACTTCACCCTGAAGATTTCTAGAGTGGAAG CCGAGGACCTGGGCGTGTACTTCTGTTCTCAGTCTACCCACGTGCCACCTACCTT TGGCGGCGGAAGCAAGCTGGAAATCAAG |

### (12) Bispecific antibody TNFαi-I3F07 LC

In case of TNFαi-I3F07 LC, a binding fragment (ScFv) of an anti-OX40L antibody I3F07, in which a heavy chain variable region (SEQ ID NO: 53) of anti-OX40L antibody I3F07 and a light chain variable region (SEQ ID NO: 54) of anti-OX40L antibody I3F07 are connected through a linker (SEQ ID NO: 32), was connected at C-terminus of a light chain constant region of TNFai antibody through a linker (SEQ ID NO: 31).

Bispecific antibody TNFαi-I3F07 LC was prepared by using an expression vector pcDNA3.1 (Invitrogen) and an animal cell line of FreeStyle^{™} 293-F. Detailed culture conditions, culture methods, and purification methods were performed in substantially the same manner as those described above in (1) anti-OX40L antibody 02C09.

**[Table 27] Light chain of TNFαi-I3F07 LC**

| TNFai light chain-linker-anti-OX40L antibody I3F07 variable region | |
|---|---|
| Amino acid sequence (SEQ ID NO: 79) | DIQMTQSPSSLSASVGDRVTITCRASQGIRNYLAWYQQKPGKAPKLLIY AASTLQSGVPSRFSGSGSGTDFTLTISSLQPEDVATYYCQRYNRAPYTFG QGTKVEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSPVTKSF NRGECGGGGSGGGGSGGGGSQIQLAQSGAELVKPGASVKLSCKASGFTFPDY WMHWVKQRPGQGLEWIGEIDPPNGRTNYNEKFKSKATLTVDSSSNTAYMQLSR LTSEDSAVYYCARGIYYVDYWGQGTTLTVSSGGGGSGGGGSGGGGSGGGGSD IVLTQSPLSLPVSLGDQASISCRSSQSLVHSNGNTYLHWYLQKPGQSPKLLIYKVS NRFSGVPDRFSGSGSGTDFTLKISRVEAEDLGVYFCSQSTHVPPTFGGGSKLEIK |
| Nucleic acid (DNA) sequence (SEQ ID NO: 80) | GATATCCAGATGACACAGAGCCCTAGCAGCCTGTCTGCCTCTGTGGGCG ATAGAGTGACCATCACCTGTAGAGCCAGCCAGGGCATCAGAAACTACC TGGCCTGGTATCAGCAGAAGCCCGGAAAGGCCCCTAAGCTGCTGATCT ATGCCGCCTCTACACTGCAGAGCGGCGTGCCATCTAGATTTTCTGGCAG CGGCTCTGGCACCGACTTCACCCTGACAATTTCTAGCCTGCAGCCTGAG GACGTGGCCACCTACTACTGCCAGAGATACAACAGAGCCCCTTACACCT TCGGCCAGGGCACCAAGGTGGAAATCAAGCGTACGGTGGCGGCGCCATCT GTCTTCATCTTCCCGCCATCTGATGAGCAGTTGAAATCTGGAACTGCCTCTGTTG TGTGCCTGCTGAATAACTTCTATCCCAGAGAGGCCAAAGTACAGTGGAAGGTGG ATAACGCCCTCCAATCGGGTAACTCCCAGGAGAGTGTCACAGAGCAGGACAGCA AGGACAGCACCTACAGCCTCAGCAGCACCCTGACGCTGAGCAAAGCAGACTACG AGAAACACAAAGTCTACGCCTGCGAAGTCACCCATCAGGGCCTGAGCTCGCCCG |
| | TCACAAAGAGCTTCAACAGGGGAGAGTGTGGTGGTGGCGGATCTGGCGGCG GAGGAAGCGGAGGCGGAGGCTCCCAGATTCAGCTGGCTCAGTCTGGCGCCG AACTTGTGAAACCTGGCGCCTCTGTGAAGCTGAGCTGCAAGGCCAGCGGCTTCA CCTTTCCTGACTACTGGATGCACTGGGTCAAGCAGAGGCCTGGACAGGGACTCG AATGGATCGGCGAGATCGATCCTCCTAACGGCCGGACCAACTACAACGAGAAGT TCAAGAGCAAGGCCACACTGACCGTGGACAGCAGCAGCAATACCGCCTACATGC AGCTGAGCAGACTGACCTCTGAGGACAGCGCCGTGTACTACTGTGCCAGAGGCA TCTACTACGTGGACTACTGGGGCCAGGGCACAACCCTGACAGTTTCTTCTGGTG GCGGAGGTTCTGGCGGAGGTGGTAGTGGTGGCGGTGGAAGTGGTGGC GGCGGATCTGATATCGTGCTGACACAGAGCCCTCTGAGCCTGCCTGTTTCTCT GGGAGATCAGGCCAGCATCAGCTGCAGATCCTCTCAGAGCCTGGTGCACAGCAA CGGCAATACCTACCTGCACTGGTATCTGCAGAAGCCCGGCCAGTCTCCTAAGCT GCTGATCTACAAGGTGTCCAACAGGTTCAGCGGCGTGCCCGATAGATTTTCTGG CTCTGGCAGCGGCACCGACTTCACCCTGAAGATTTCTAGAGTGGAAGCCGAGGA CCTGGGCGTGTACTTCTGTTCTCAGTCTACCCACGTGCCACCTACCTTTGGCGGC GGAAGCAAGCTGGAAATCAAG |

In addition, according to substantially the same method as shown in the method for producing (preparing) the anti-OX40L antibody of above Example 2, an anti-OX40L antibody (hereinafter, Ref. Ab or O4L), which was a reference antibody, was prepared by using a sequence (SEQ ID NOs: 33 and 34) of VH and VL of oxelumab, which was an anti-OX40L antibody, and an anti-TNFa antibody (hereinafter, Ref. TNFai or Humira), which was a reference antibody, was prepared by using a sequence (SEQ ID NOs: 35 and 36) of VH and VL of Humira, which was an anti-TNFa antibody.

**[Table 28] Heavy and light chain sequences of O4L**

| O4L | |
|---|---|
| Heavy chain (SEQ ID NO: 81) | EVQLLESGGGLVQPGGSLRLSCAASGFTFNSYAMSWVRQAPGKGLEWVSIISGSG GFTYYADSVKGRFTISRDNSRTTLYLQMNSLRAEDTAVYYCAKDRLVAPGTFDY WGQGALVTVSSASTKGPSVFPLAPCSRSTSESTAALGCLVKDYFPEPVTVSWNSG ALTSGVHTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIAVEWESNG QPENNYKTTPPVLDSDGSFFLYSRLTVDKSRWQEGNVFSCSVMHEALHNHYTQ KSLSLSLGK |
| Light chain (SEQ ID NO: 82) | DIQMTQSPSSLSASVGDRVTITCRASQGISSWLAWYQQKPEKAPKSLIYAASSLQS GVPSRFSGSGSGTDFTLTISSLQPEDFATYYCQQYNSYPYTFGQGTKLEIKRTVAAP SVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC |

**[Table 29] Heavy and light chain sequences of Ref. TNFai**

| Ref. TNFai | |
|---|---|
| Heavy chain (SEQ ID NO: 83) | EVQLVESGGGLVQPGRSLRLSCAASGFTFDDYAMHWVRQAPGKGLEWVSAITWNS GHIDYADSVEGRFTISRDNAKNSLYLQMNSLRAEDTAVYYCAKVSYLSTASSLDYWG QGTLVTVSSASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRVESKYGPPCP PCPAPEFLGGPSVFLFPPKPKDTLMISRTPEVTCVVVDVSQEDPEVQFNWYVDGVEV HNAKTKPREEQFNSTYRVVSVLTVLHQDWLNGKEYKCKVSNKGLPSSIEKTISKAKG QPREPQVYTLPPSQEEMTKNQVSLTCLVKGFYPSDIAVEWESNGQPENNYKTTPPVL DSDGSFFLYSRLTVDKSRWQEGNVFSCSVMHEALHNHYTQKSLSLSLGK |
| Light chain (SEQ ID NO: 84) | DIQMTQSPSSLSASVGDRVTITCRASQGIRNYLAWYQQKPGKAPKLLIYAASTLQSGV PSRFSGSGSGTDFTLTISSLQPEDVATYYCQRYNRAPYTFGQGTKVEIKRTVAAPSVFI FPPSDEQLKSGTASVVCLLNNFYPREAKVQWKVDNALQSGNSQESVTEQDSKDSTYS LSSTLTLSKADYEKHKVYACEVTHQGLSSPVTKSFNRGEC |

According to substantially the same method as shown in the method for producing (preparing) the bispecific antibody of above Example 3, anti-OX40L-TNFαi HC and LC antibodies (hereinafter, O4L-TNFαi HC and O4L-TNFαi LC, respectively) were prepared by using the prepared antibody O4L and above Ref. TNFai.

**[Table 30] Heavy chain sequence of O4L-TNFαi HC**

| O4L heavy chain-linker-TNFai variable region | |
|---|---|
| Amino acid sequence (SEQ ID NO: 85) | EVQLLESGGGLVQPGGSLRLSCAASGFTFNSYAMSWVRQAPGKGLEWVSIISGSG GFTYYADSVKGRFTISRDNSRTTLYLQMNSLRAEDTAVYYCAKDRLVAPGTFDY WGQGALVTVSSASTKGPSVFPLAPCSRSTSESTAALGCLVKDYFPEPVTVSWNSG ALTSGVHTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIAVEWESNG QPENNYKTTPPVLDSDGSFFLYSRLTVDKSRWQEGNVFSCSVMHEALHNHYTQ KSLSLSLGKGGGGSGGGGSGGGGSEVQLVESGGGLVQPGRSLRLSCAASGFTF DDYAMHWVRQAPGKGLEWVSAITWNSGHIDYADSVEGRFTISRDNAKNSLYLQ MNSLRAEDTAVYYCAKVSYLSTASSLDYWGQGTLVTVSSGGGGSGGGGSGGG GSGGGGSDIQMTQSPSSLSASVGDRVTITCRASQGIRNYLAWYQQKPGKAPKLL IYAASTLQSGVPSRFSGSGSGTDFTLTISSLQPEDVATYYCQRYNRAPYTFGQGTK |
| | VEIK |
| Nucleic acid (DNA) sequence (SEQ ID NO: 86) | GAGGTGCAGCTGCTGGAAAGCGGCGGAGGACTGGTGCAGCCTGGCGG CAGCCTGAGACTGTCTTGCGCCGCCAGCGGCTTCACCTTCAACAGCTAC GCCATGAGCTGGGTCCGACAGGCCCCTGGCAAGGGCCTGGAATGGGTG TCCATCATCAGCGGCAGCGGCGGCTTTACCTACTACGCCGACAGCGTGA AGGGCCGGTTCACCATCAGCCGGGACAACAGCCGGACCACCCTGTACC TGCAGATGAACAGCCTGCGGGCCGAGGACACCGCCGTGTACTACTGCG CCAAGGACAGACTGGTGGCCCCAGGCACCTTCGACTATTGGGGACAGG GCGCCCTCGTGACCGTGTCCTCTGCTTCCACCAAGGGCCCATCCGTCTTCCC CCTGGCGCCCTGCTCCAGGAGCACCTCCGAGAGCACAGCCGCCCTGGGCTGCCT GGTCAAGGACTACTTCCCCGAACCGGTGACGGTGTCGTGGAACTCAGGCGCCCT GACCAGCGGCGTGCACACCTTCCCGGCTGTCCTACAGTCCTCAGGACTCTACTCC CTCAGCAGCGTGGTGACCGTGCCCTCCAGCAGCTTGGGCACGAAGACCTACACC TGCAACGTAGATCACAAGCCCAGCAACACCAAGGTGGACAAGAGAGTTGAGTCC AAATATGGTCCCCCATGCCCACCATGCCCAGCACCTGAGTTCCTGGGGGGACCA TCAGTCTTCCTGTTCCCCCCAAAACCCAAGGACACTCTCATGATCTCCCGGACCC CTGAGGTCACGTGCGTGGTGGTGGACGTGAGCCAGGAAGACCCCGAGGTCCAG TTCAACTGGTACGTGGATGGCGTGGAGGTGCATAATGCCAAGACAAAGCCGCGG GAGGAGCAGTTCAACAGCACGTACCGTGTGGTCAGCGTCCTCACCGTCCTGCAC CAGGACTGGCTGAACGGCAAGGAGTACAAGTGCAAGGTCTCCAACAAAGGCCTC CCGTCCTCCATCGAGAAAACCATCTCCAAAGCCAAAGGGCAGCCCCGAGAGCCA CAGGTGTACACCCTGCCCCCATCCCAGGAGGAGATGACCAAGAACCAGGTCAGC CTGACCTGCCTGGTCAAAGGCTTCTACCCCAGCGACATCGCCGTGGAGTGGGAG AGCAATGGGCAGCCGGAGAACAACTACAAGACCACGCCTCCCGTGCTGGACTCC GACGGCTCCTTCTTCCTCTACAGCAGGCTAACCGTGGACAAGAGCAGGTGGCAG GAGGGGAATGTCTTCTCATGCTCCGTGATGCATGAGGCTCTGCACAACCACTAC |
| | ACACAGAAGAGCCTCTCCCTGTCTCTGGGTAAAGGTGGTGGCGGATCTGGCG GCGGAGGAAGCGGAGGCGGAGGCTCCGAAGTGCAACTGGTTGAATCTGGC GGAGGACTGGTGCAGCCTGGAAGAAGCCTGAGACTGTCTTGTGCCGCCAGCGG CTTCACCTTCGATGATTATGCCATGCACTGGGTCCGACAGGCCCCTGGAAAAGG ACTTGAATGGGTGTCCGCCATCACCTGGAACAGCGGCCACATCGATTACGCCGA TAGCGTGGAAGGCCGGTTCACCATCAGCAGAGACAACGCCAAGAACAGCCTGTA CCTGCAGATGAACTCCCTGAGAGCCGAGGACACCGCCGTGTACTACTGTGCCAA AGTGTCTTACCTGAGCACCGCCTCCAGCCTGGATTATTGGGGACAGGGCACACT GGTCACAGTGTCCTCTGGTGGCGGAGGTTCTGGCGGAGGTGGTAGTGGT GGCGGTGGAAGTGGTGGCGGCGGATCTGATATCCAGATGACACAGAGCCC TAGCAGCCTGTCTGCCTCTGTGGGCGATAGAGTGACCATCACCTGTAGAGCCAG CCAGGGCATCAGAAACTACCTGGCCTGGTATCAGCAGAAGCCCGGAAAGGCCCC TAAGCTGCTGATCTATGCCGCCTCTACACTGCAGAGCGGCGTGCCATCTAGATTT TCTGGCAGCGGCTCTGGCACCGACTTCACCCTGACAATTTCTAGCCTGCAGCCTG AGGACGTGGCCACCTACTACTGCCAGAGATACAACAGAGCCCCTTACACCTTCG GCCAGGGCACCAAGGTGGAAATCAAG |

**[Table 31] Light chain of O4L-TNFαi LC**

| O4L light chain-linker-TNFai variable region | |
|---|---|
| Amino acid sequence (SEQ ID NO: 87) | DIQMTQSPSSLSASVGDRVTITCRASQGISSWLAWYQQKPEKAPKSLIY AASSLQSGVPSRFSGSGSGTDFTLTISSLQPEDFATYYCQQYNSYPYTFG QGTKLEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSPVTKSF NRGECGGGGSGGGGSGGGGSEVQLVESGGGLVQPGRSLRLSCAASGFTFDDY AMHWVRQAPGKGLEWVSAITWNSGHIDYADSVEGRFTISRDNAKNSLYLQMNS LRAEDTAVYYCAKVSYLSTASSLDYWGQGTLVTVSSGGGGSGGGGSGGGGSG |
| | GACATCCAGATGACCCAGAGCCCCAGCAGCCTGAGCGCCAGCGTGGGC GACAGAGTGACCATCACCTGTCGGGCCAGCCAGGGCATCAGCAGCTGG CTGGCCTGGTATCAGCAGAAGCCCGAGAAGGCCCCCAAGAGCCTGATC TACGCCGCCAGCTCTCTGCAGAGCGGCGTGCCCAGCAGATTCAGCGGC AGCGGCTCCGGCACCGACTTCACCCTGACCATCAGCAGCCTGCAGCCCG AGGACTTCGCCACCTACTACTGCCAGCAGTACAACAGCTACCCCTACAC CTTCGGCCAGGGCACCAAGCTGGAAATCAAGCGTACGGTGGCGGCGCCAT CTGTCTTCATCTTCCCGCCATCTGATGAGCAGTTGAAATCTGGAACTGCCTCTGT TGTGTGCCTGCTGAATAACTTCTATCCCAGAGAGGCCAAAGTACAGTGGAAGGT GGATAACGCCCTCCAATCGGGTAACTCCCAGGAGAGTGTCACAGAGCAGGACAG CAAGGACAGCACCTACAGCCTCAGCAGCACCCTGACGCTGAGCAAAGCAGACTA CGAGAAACACAAAGTCTACGCCTGCGAAGTCACCCATCAGGGCCTGAGCTCGCC CGTCACAAAGAGCTTCAACAGGGGAGAGTGTGGTGGTGGCGGATCTGGCGG CGGAGGAAGCGGAGGCGGAGGCTCCGAAGTGCAACTGGTTGAATCTGGCG GAGGACTGGTGCAGCCTGGAAGAAGCCTGAGACTGTCTTGTGCCGCCAGCGGCT TCACCTTCGATGATTATGCCATGCACTGGGTCCGACAGGCCCCTGGAAAAGGAC TTGAATGGGTGTCCGCCATCACCTGGAACAGCGGCCACATCGATTACGCCGATA GCGTGGAAGGCCGGTTCACCATCAGCAGAGACAACGCCAAGAACAGCCTGTACC TGCAGATGAACTCCCTGAGAGCCGAGGACACCGCCGTGTACTACTGTGCCAAAG TGTCTTACCTGAGCACCGCCTCCAGCCTGGATTATTGGGGACAGGGCACACTGG TCACAGTGTCCTCTGGTGGCGGAGGTTCTGGCGGAGGTGGTAGTGGTGG CGGTGGAAGTGGTGGCGGCGGATCTGATATCCAGATGACACAGAGCCCTAG CAGCCTGTCTGCCTCTGTGGGCGATAGAGTGACCATCACCTGTAGAGCCAGCCA GGGCATCAGAAACTACCTGGCCTGGTATCAGCAGAAGCCCGGAAAGGCCCCTAA GCTGCTGATCTATGCCGCCTCTACACTGCAGAGCGGCGTGCCATCTAGATTTTCT |
| Nucleic acid (DNA) sequence (SEQ ID NO: 88) | GGGSDIQMTQSPSSLSASVGDRVTITCRASQGIRNYLAWYQQKPGKAPKLLIYAA STLQSGVPSRFSGSGSGTDFTLTISSLQPEDVATYYCQRYNRAPYTFGQGTKVEIK |
| | GGCAGCGGCTCTGGCACCGACTTCACCCTGACAATTTCTAGCCTGCAGCCTGAG GACGTGGCCACCTACTACTGCCAGAGATACAACAGAGCCCCTTACACCTTCGGCC AGGGCACCAAGGTGGAAATCAAG |

### Experimental Example 1: Antibody analysis

The anti-OX40L antibody obtained in Example 2 and the bispecific antibody obtained in Example 3 were analyzed by SDS-PAGE, and the results thereof are shown in FIG. 2.

### Experimental Example 2: Epitope of OX40L antigen for anti-OX40L antibody

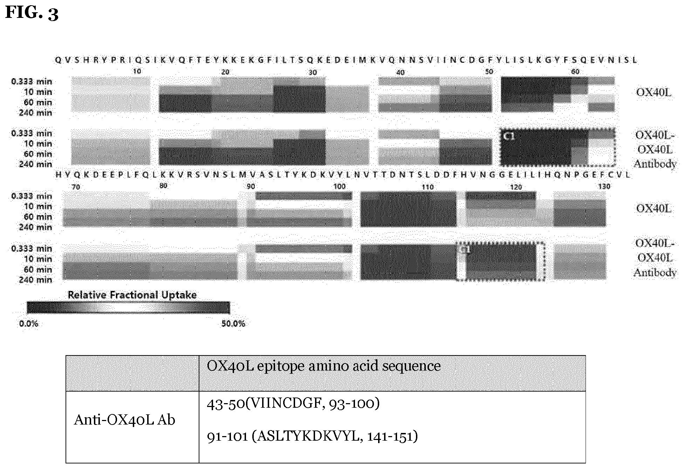

The epitope of the OX40L antigen for the anti-OX40L antibody was analyzed by using HDX-MS technique. 0.12-2.00 M Urea, 0.12-1.00 M TCEP (pH 2.6) quench buffer and pepsin column were used and labeled with D₂O-based buffer solution to make an analysis at five time points. Data was processed by using PLGS and DynamX so as to obtain the results as shown in FIG. 3.

### Experimental Example 3: Antibody properties

### Experimental Example 3-1. Analysis of equilibrium dissociation constant (K_{D}) for antigen of anti-OX40L antibody and bispecific antibody

The affinity for antigen was analyzed as follows with regard to the anti-OX40L antibody and the bispecific antibody for simultaneously controlling OX40L and TNFα, which were isolated and purified above in Examples 2 and 3.

Among the above antibodies, the binding force of the anti-OX40L antibody for human OX40L (SEQ ID NO: 2) was confirmed, and the binding force of the OX40L and TNFα bispecific antibody for human OX40L (SEQ ID NO: 2) and human TNFα (TNF-H5228 of Aero Biosystems) was confirmed, respectively (Table 32).

A surface plasmon resonance (SPR) analysis was performed by using Bicore T200, and a running buffer used was HBS-EP (10 mM HEPES, pH7.4, 150 mM NaCl, 3 mM EDTA, 0.15% surfactant P20). A human antibody capture kit (anti-hFc antibody) was fixed onto a surface of CM5 chip through an amine coupling method. Human OX40L or human TNFα were diluted to 10 nM with the running buffer, and then serially diluted 1/2 to make an analysis at five concentration intervals. The concentration of the antibodies was confirmed by sterilizing and filtering the antibodies through a 0.2 µm filter and measuring the absorbance (A280). An analytical sample was prepared with high purity/high concentration so that the minimum dilution factor could be 100 or more, thereby minimizing a buffer effect. A regeneration step was provided between all the analysis intervals so that the baseline of the experiment could be kept constant, and the Biacore analysis results are as shown in Table 32 and FIG. 4.

In FIG. 4, Ag1 represents human OX40L, and Ag2 represents human TNFα.

**[Table 32]**

| Antigen | Antibody | ka (1/Ms) | kd (1/s) | KD(M) | Rmax(RU) | Chi²(RU²) | U-value | SE(ka) | SE(kd) |
|---|---|---|---|---|---|---|---|---|---|
| OX40L | Hu3F07 | 1.27E+06 | 3.57E-04 | 2.82E-10 | 40.4 | 0.2 | 3.1 | 8.00E+02 | 1.70E-06 |
| | 10H07 | 1.69E+06 | 4.98E-04 | 2.95E-10 | 38 | 0.8 | 4.1 | 1.90E+03 | 3.00E-06 |
| | 21G07 | 1.08E+06 | 6.50E-04 | 6.03E-10 | 36.7 | 0.4 | 2.4 | 1.10E+03 | 2.70E-06 |
| | 02C09 | 1.16E+06 | 4.08E-04 | 3.52E-10 | 38 | 0.7 | 5.3 | 1.40E+03 | 3.10E-06 |
| | O4L-TNFαi HC | 1.23E+06 | 5.76E-04 | 4.67E-10 | 25.8 | 0.1 | 2.4 | 3.80E+03 | 2.10E-06 |
| | 02C09-TNFαi HC | 1.07E+06 | 5.03E-04 | 4.72E-10 | 32.4 | 0.4 | 3.1 | 1.20E+03 | 3.00E-06 |
| | Hu3F07-TNFαi HC | 1.38E+06 | 6.19E-04 | 4.50E-10 | 26.8 | 0.1 | 1.9 | 1.00E+03 | 2.00E-06 |
| TNFα | O4L-TNFαi HC | 4.38E+05 | 2.03E-04 | 4.64E-10 | 49.1 | 0.8 | 6.9 | 3.40E+02 | 2.20E-06 |
| | 02C09-TNFαi HC | 4.16E+05 | 9.55E-05 | 2.30E-10 | 85.7 | 1.9 | 11.6 | 1.10E+03 | 1.90E-06 |
| | Hu3F07-TNFαi HC | 4.55E+05 | 2.03E-04 | 4.46E-10 | 48 | 0.7 | 6.9 | 3.50E+02 | 2.20E-06 |

| | | | | | | | | | |
|---|---|---|---|---|---|---|---|---|---|
| (In the table above, aE+b represents a X 10^{b}, and aE-b represents a X 10^{-b}.) | | | | | | | | | |

As confirmed from above Table 32, it could be confirmed that the anti-OX40L antibody of Example 2 strongly binds to OX40L, while the bispecific antibody of Example 3 strongly binds to both OX40L and TNFα. Specifically, it was confirmed that both the anti-OX40L antibody and the bispecific antibody show binding force for OX40L at the level of nM, and in particular the bispecific antibody shows binding force for TNFα at the level of nM, too. The above results suggest that the bispecific antibody maintains a high level of binding capacity for each antigen without interruption.

### Experimental Example 3-2. Test on thermal stability of anti-OX40L antibody and bispecific antibody

A test was conducted to confirm the thermal stability properties with regard to the bispecific antibody of Example 3 and the anti-OX40L antibody of Example 2 (Table 33).

The antibodies were diluted in DPBS to reach 3 µM/45 µL, mixed with 5 µL of 200x sypro orange dye (#S6650, Thermo), and dispensed by 50 µL into qPCR Tube (#B77009, B57651, bioplastics). The qPCR was performed by using Biorad CFX96 real time PCR device. The qPCR conditions were completed by reacting at 25°C for 30 seconds and then reacting at each temperature for one minute and finally at 25°C for 10 seconds while increasing a temperature by 1°C up to 99°C. A melting temperature (Tₘ) was used as a rate constant at which the antibody structure is unfolded, and the results thereof are as shown in Table 33 below.

**[Table 33]**

| **Sample** | **Melt Tₘ(°C)** | **Sample** | **Melt Tₘ(°C)** |
|---|---|---|---|
| Hu3F07 | 63 | Hu3F07-TNFαi HC | 64.5 |
| 02C09 | 64 | Hu3F07-TNFαi LC | 59.5 |
| 10H07 | 59 | 02C09-TNFαi HC | 61.5 |
| 21G07 | 59 | 02C09-TNFαi LC | 60.5 |

As confirmed from the above table, it was found that the anti-OX40L antibody and the bispecific antibody show a melting temperature between 59 and 65°C, suggesting that the bispecific antibody also has thermal stability similar to the anti-OX40L antibody.

### Experimental Example 3-3. Pharmacokinetics (PK) analysis of anti-OX40L antibody and bispecific antibody

An analysis was performed to confirm the pharmacokinetics when administering the bispecific antibody of Example 3 and the anti-OX40L antibody of Example 2 (Table 34).

An experiment animal used was the male rat of the Sprague-Dawley strain, which has been widely used in pharmacokinetics tests due to its constant drug response and a stable supply system. Three male Sprague-Dawley (SD) rats (seven weeks old) were used without fasting, respectively and a single IV bolus was administered at 5 mpk (2.5-2.4 mg/ml). After administration, blood was collected at about 150 µl/time point through the jugular vein a total of 10 times at 3 min, 3, 8, 24, 48, 72, 96, 120, 144, 168 hrs, and separated by using sodium heparin an anticoagulant, and a sample was stored in a cryogenic freezer immediately after obtaining about 70 µ of plasma through centrifugation (12,000 rpm, 3 min). During the experiment, general symptoms were observed at least once a day. After the last blood collection, the experimental animals were euthanized by inhalation of CO₂.

The plasma obtained from the PK test was analyzed through a device called Gyrolab xPlore^{®} (Cat. #P0020300, GYROS PROTEIN) and Gyrolab PK kit (Cat. # P0020499, GYROS PROTEIN). From the result values obtained by Gyrolab, parameter values such as AUC(last), AUC(inf), Cₘₐₓ, Tₘₐₓ, Half life, etc., were obtained by using BA Calc 2007 1.0.0 or PK Solver 2.0 program, and the results thereof are as shown in Table 34.

**[Table 34]**

| **Sample** | **Half life (Day)** | **AUC(Last) (µg hr/mL)** | **AUC(Inf) (µg hr/mL)** | **Cmax (µg/mL)** |
|---|---|---|---|---|
| Hu3F07 | 3.6 | 5273 | 7863 | 107 |
| Hu3F07-TNFαi HC | 5.1 | 5299 | 8453 | 101 |
| Hu3F07-TNFαi LC | 3.7 | 4848 | 6918 | 91 |
| 02C09 | 4.0 | 3958 | 5680 | 83 |
| 02C09-TNFαi HC | 6.0 | 1348 | 2144 | 41 |
| 02C09-TNFαi LC | 3.6 | 3425 | 4406 | 89 |

It was observed that the half-life of the anti-OX40L antibody of Example 2 is 3.6 and 4.0 days, respectively, and that of the bispecific antibody of Example 3 is 3.6 to 6.0 days.

Thus, it could be understood that the half-life may be about two weeks or more in humans, too.

### Experimental Example 4: Efficacy of antibody

### Experimental Example 4-1. Evaluation of signal inhibitory ability of antibody (1)

An experiment on the activity evaluation of the anti-OX40L antibody of Example 2 and the bispecific antibody of Example 3 (Hu3F07-TNFαi HC, 02C09-TNFαi HC, O4L-TNFai HC) was performed by using OX40L/OX40 blockade bioassay kit (Promega CS197706) and TNFα/TNFαRc blockade bioassay kit (Promega CS177503) (Table 35, FIG. 5).
(1) The activity of the anti-OX40L antibody of Example 2 and the bispecific antibody of Example 3 was evaluated by using an OX40L/OX40 blockade bioassay kit.

NFκB-luc2/OX40 Jurkat cells were placed in RPMI1640 (10% FBS) culture fluid and were stationary cultured at 37°C, 5% CO₂ incubator overnight. On the next day, the antigens and antibodies to be reacted with the cells were prepared. When OX40L antigen was added to the cells in RPMI1640 (10% FBS) culture fluid so that the final concentration could reach 15 ng/mL. The anti-OX40L antibody, the bispecific antibody, and the control antibody were first diluted to a final concentration of 33 µg/mL, and then serially diluted by 1/3 in nine steps. The 10th concentration was 0 and replaced with a culture fluid so as to obtain a concentration gradient of 10 steps in total. Each of the prepared antigen diluted solutions and antibody diluted solutions was dispensed 25 µL into the cells. Each sample was dispensed to be repeated three times. After dispensing, a total of cells, antigen diluted solution, and antibody diluted solution were made 100 µL and reacted in a CO₂ incubator for about five hours.

75 µL of Bio-Glo was dispensed and reacted for about 10 minutes, after which the sample was analyzed by luminescence on a microplatereader, and then analyzed by 4-parameters (X-axis log (concentration)).

The final IC₅₀ was calculated by converting the IC₅₀ analysis results into nM at µg/mL, which is the concentration unit of the antibody, so that the anti-OX40L antibody can be represented by 150 kDa and the bispecific antibody can be represented by 200 kDa.

(2) The bispecific antibody activity was evaluated by using TNFα/TNFαRc blockade bioassay kit.

NPκB-RE HEK293 cell DMEM (10% FBS) was placed in the culture fluid and was stationary cultured at 37°C, 5% CO₂ incubator. The antigens and antibodies to be reacted with the cells were prepared. TNFα antibodies were diluted in DMEM (10% FBS) culture fluid so that the final concentration may reach 3 ng/mL when being reacted with the cells. The bispecific antibody and Humira (Ref. TNFai), which was a control antibody, were diluted in DMEM (10% FBS) culture fluid with an initial concentration of 10 nM, and then serially diluted by 1/2 in eight steps, thereby obtaining the final concentration, in which the initial concentration reaches 0.8 nM when being reacted with the cells.

The prepared antigen diluted solutions and antibody diluted solutions were mixed at a ratio of 3:2 and dispensed by 20 µL into the cells. Each sample was dispensed to be repeated twice, and reacted in a CO₂ incubator for about four hours after dispensing.

100 µL of Bio-Glo was dispensed and reacted, after which the sample was analyzed with luminescence on a microplatereader, and then analyzed by 4-parameters (X-axis log (concentration)) to calculate IC₅₀.

Above IC₅₀ values are as shown in Table 35 below and FIG. 5.

**[Table 35]**

| **Analysis** | **Sample** | **IC50 (nM)** | **IC₅₀(µg/mL)** |
|---|---|---|---|
| OX40L blockade assay | Hu3F07 | 0.20 | 0.030 |
| | 02C09 | 0.78 | 0.117 |
| | 21G07 | 0.85 | 0.128 |
| | 10H07 | 0.41 | 0.061 |
| | Hu3F07-TNFαi HC | 0.21 | 0.041 |
| | 02C09-TNFαi HC | 0.59 | 0.117 |
| | O4L-TNFαi HC | 1.23 | 0.246 |
| | Ref. Ab | 1.95 | 0.293 |
| TNFα blockade assay | Hu3F07-TNFαi HC | 0.057 | - |
| | 02C09-TNFαi HC | 0.067 | - |
| | O4L-TNFαi HC | 0.056 | - |
| | Humira (Ref, TNFαi) | 0.063 | - |

As can understood from above Table 35 and FIG. 5, it could be found that the bispecific antibody and the anti-OX40L antibody according to Examples 2 and 3 show an OX40L blocking ability at the level of 1.3 × 10⁻⁹ M (nM) or less, and in particular the anti-OX40L antibody shows the OX40L blocking ability at the level of 0.9 × 10⁻⁹ M (nM) or less, and the bispecific antibody shows the TNFα blocking ability at the level of 1 × 10⁻⁹ M (nM) or less, thereby providing a remarkably excellent blocking ability. In other words, it can be understood that the anti-OX40L antibody of the present invention shows an excellent OX40L blocking ability, and the bispecific antibody prepared by using the antibody also shows an excellent blocking ability not only for OX40L, but also for TNFα.

### Experimental Example 4-2. Evaluation of signal inhibitory ability of antibody (2)

The activity of the bispecific antibody (02C09-TNFαi LC, TNFαi-02C09 HC, TNFai 02C09 LC) of Example 3 was evaluated by using the TNFα/TNFαRc blockade bioassay kit (Promega CS177503) (Table 36). The detailed conditions and methods for evaluation were substantially the same as described above in the evaluation of signal inhibitory ability of the antibody (1) in Experimental Example 4-1.

**[Table 36]**

| **Analysis** | **Sample** | **IC50 (nM)** |
|---|---|---|
| TNFα blockade assay | 02C09-TNFαi LC | 0.058 |
| | TNFαi-02C09 HC | 0.057 |
| | TNFαi 02C09 LC | 0.055 |

As can understood from above Table 36, it could be found that the bispecific antibodies 02C09-TNFαi LC, TNFαi-02C09 HC and TNFai 02C09 LC of Example 3 show a TNFα blocking ability at the level of 1 × 10⁻⁹ M (nM) or less, thereby providing an excellent blocking ability.

### Experimental Example 4-3. Evaluation of signal inhibitory ability of antibody (3)

The activity of the bispecific antibody (I3F07-TNFαi HC, I3F07-TNFαi LC, TNFαi-I3F07 HC, TNFαi-I3F07 LC) of Example 3 was evaluated by using the OX40L/OX40 blockade bioassay kit (Promega CS197706) and TNFα/TNFαRc blockade bioassay kit (Promega CS177503) (Table 37). The detailed conditions and methods for evaluation were substantially the same as described above in the evaluation of signal inhibitory ability of the antibody (1) in Experimental Example 4-1.

**[Table 37]**

| **Analysis** | **Sample** | **IC50 (nM)** |
|---|---|---|
| OX40L blockade assay | I3F07-TNFαi HC | 0.91 |
| | I3F07-TNFαi LC | 0.94 |
| | TNFαi-I3F07 HC | 0.80 |
| | TNFαi-I3F07 LC | 0.71 |
| TNFα blockade assay | I3F07-TNFαi HC | 0.055 |
| | I3F07-TNFαi LC | 0.077 |
| | TNFαi-I3F07 HC | 0.054 |
| | TNFαi-I3F07 LC | 0.065 |

As can understood from above Table 37, it could be found that the bispecific antibody of Example 3 shows an OX40L blocking ability at the level of 1 × 10⁻⁹ M (nM) or less and a TNFα blocking ability at the level of 1 × 10⁻⁹ M (nM) or less, thereby providing an excellent blocking ability.

### Experimental Example 4-4. Evaluation of antibody inhibitory ability for immune cell activity

In order to evaluate the efficacy of the anti-OX40L antibody of Example 2 and the bispecific antibody of Example 3, the ability to inhibit the activity of immune cells was confirmed by using peripheral blood mononuclear cells (PBMC) of a normal person and a patient with rheumatoid arthritis (RA) (FIG. 6).

By the above method, T cells were isolated from the PBMCs of the normal person and the RA patient, and the level of decreased secretion of IL-2, a cytokine secreted when T cells were activated, was analyzed.

To divide T cells in the normal and patient PBMCs, respectively, anti CD3 (R&D systems, MAB100) was diluted at 100 ng/well and attached to a 96 well for about 16 hours (5±3). After removing the attached solution, the cells were washed with PBS. After diluting DNase I in LGM-3 (10% FBS, 1% P/S) to reach 20 U/mL, the normal PBMC was added to prepare a mixed solution, centrifuged (200 g, 15 minutes) to remove the supernatant, diluted and dispensed so that the normal PBMC could reach 1×10⁶ cells/mL by using LGM-3 (10% FBS, 1% P/S). Human OX40L (ACROBIOSYSTEMS, OXL-H52Q8) and human TNFα (SINO BIOLOGICAL, 10602-HNA) were diluted in LGM-3 (10% FBS, 1% P/S) and added by 50 µL to the dispensed normal PBMC. An anti-OX40L antibody and a bispecific antibody were prepared at 800 ng/mL with LGM-3 (10% FBS, 1% P/S), and then diluted to 1 nM. The diluted antibodies were dispensed by 50 µL into a plate containing T cells isolated from PBMC, human OX40L, and human TNFα, and mixed. In about 24 to 72 hours later, only the supernatant was collected and IL-2 was measured, and the results are shown in FIG. 6.

In FIG. 6, A represents the results of the normal PBMC-derived T cell assay, and B represents the results of the patient PBMC-derived T cell assay.

As understood from FIG. 6, it was confirmed that IL-2 shows a tendency to decrease when the bispecific antibody of the present invention is administered. In particular, it can be confirmed from the results of PBMC-derived T cells in RA patients that the bispecific antibody of the present invention significantly reduced IL-2 expression in PBMC-derived T cells in RA patients compared to Humira (Ref. TNFai). This may mean that the bispecific antibody of the present invention acts as an effective therapeutic antibody for RA, and in particular acts effectively in RA patients refractory to Humira.

Thus, it can be understood that the anti-OX40L antibody and the bispecific antibody of the present invention alleviate the overactivated immune system and show an excellent therapeutic effect on autoimmune diseases.

## Claims

1. An anti-OX40L antibody or an antigen-binding fragment thereof, specifically binding to OX40L (OX40 ligand) and inhibiting an interaction between OX40L and an OX40 receptor.

2. The anti-OX40L antibody or the antigen-binding fragment thereof according to claim 1, wherein the anti-OX40L antibody or the antigen-binding fragment thereof binds to at least one epitope represented by an amino acid sequence selected from the group consisting of SEQ ID NOs: 3 and 4.

3. The anti-OX40L antibody or the antigen-binding fragment thereof according to claim 1 or 2, wherein the anti-OX40L antibody or the antigen-binding fragment thereof binds to human OX40L with a K_{D} of 1 × 10⁻⁹ M or less, and wherein the K_{D} is measured by surface plasmon resonance (Biacore) analysis.

4. The anti-OX40L antibody or the antigen-binding fragment thereof according to claim 1 or 2, wherein the anti-OX40L antibody or the antigen-binding fragment thereof comprises:
a heavy chain variable region comprising heavy chain CDR1 represented by one amino acid sequence selected from the group consisting of SEQ ID NOs: 12, 13 and 14; heavy chain CDR2 represented by one amino acid sequence selected from the group consisting of SEQ ID NOs: 15, 16, 17 and 18; and heavy chain CDR3 represented by one amino acid sequence selected from the group consisting of SEQ ID NOs: 19, 20, 21 and 22; and
a light chain variable region comprising light chain CDR1 represented by one amino acid sequence selected from the group consisting of SEQ ID NOs: 23 and 24; light chain CDR2 represented by one amino acid sequence selected from the group consisting of SEQ ID NOs: 25 and 26; and light chain CDR3 represented by one amino acid sequence selected from the group consisting of SEQ ID NOs: 27, 28, 29 and 30.

5. The anti-OX40L antibody or the antigen-binding fragment thereof according to claim 1 or 2, wherein the anti-OX40L antibody or the antigen-binding fragment thereof comprises:
(i) an antibody or an antigen-binding fragment thereof comprising: a heavy chain variable region comprising heavy chain CDR1 represented by SEQ ID NO: 12; heavy chain CDR2 represented by SEQ ID NO: 15; and heavy chain CDR3 represented by SEQ ID NO: 19, and a light chain variable region comprising heavy chain CDR1 represented by SEQ ID NO: 23; heavy chain CDR2 represented by SEQ ID NO: 25; and light chain CDR3 represented by SEQ ID NO: 27;
(ii) an antibody or an antigen-binding fragment thereof comprising: a heavy chain variable region comprising heavy chain CDR1 represented by SEQ ID NO: 13; heavy chain CDR2 represented by SEQ ID NO: 16; and heavy chain CDR3 represented by SEQ ID NO: 20, and a light chain variable region comprising light chain CDR1 represented by SEQ ID NO: 24; light chain CDR2 represented by SEQ ID NO: 26; and light chain CDR3 represented by SEQ ID NO: 28;
(iii) an antibody or an antigen-binding fragment thereof comprising: a heavy chain variable region comprising heavy chain CDR1 represented by SEQ ID NO: 13; heavy chain CDR2 represented by SEQ ID NO: 17; and heavy chain CDR3 represented by SEQ ID NO: 21, and a light chain variable region comprising light chain CDR1 represented by SEQ ID NO: 24; light chain CDR2 represented by SEQ ID NO: 26; and light chain CDR3 represented by SEQ ID NO: 29; or
(iv) an antibody or an antigen-binding fragment thereof comprising: a heavy chain variable region comprising heavy chain CDR1 represented by SEQ ID NO: 14; heavy chain CDR2 represented by SEQ ID NO: 18; and heavy chain CDR3 represented by SEQ ID NO: 22, and a light chain variable region comprising light chain CDR1 represented by SEQ ID NO: 24; light chain CDR2 represented by SEQ ID NO: 26; and light chain CDR3 represented by SEQ ID NO: 30.

6. The anti-OX40L antibody or the antigen-binding fragment thereof according to claim 1 or 2, wherein the anti-OX40L antibody or the antigen-binding fragment thereof comprises:
a heavy chain variable region represented by one amino acid sequence selected from the group consisting of SEQ ID NOs: 37, 41, 45, 49 and 53; and
a light chain variable region represented by one amino acid sequence selected from the group consisting of SEQ ID NOs: 38, 42, 46, 50 and 54.

7. The anti-OX40L antibody or the antigen-binding fragment thereof according to claim 1 or 2, wherein the anti-OX40L antibody or the antigen-binding fragment thereof comprises:
(a) a heavy chain variable region represented by SEQ ID NO: 37 and a light chain variable region represented by SEQ ID NO: 38;
(b) a heavy chain variable region represented by SEQ ID NO: 41 and a light chain variable region represented by SEQ ID NO: 42;
(c) a heavy chain variable region represented by SEQ ID NO: 45 and a light chain variable region represented by SEQ ID NO: 46;
(d) a heavy chain variable region represented by SEQ ID NO: 49 and a light chain variable region represented by SEQ ID NO: 50; or
(e) a heavy chain variable region represented by SEQ ID NO: 53 and a light chain variable region represented by SEQ ID NO: 54.

8. The anti-OX40L antibody or the antigen-binding fragment thereof according to claim 7, wherein the anti-OX40L antibody or the antigen-binding fragment thereof comprises:
a heavy chain constant region represented by one amino acid sequence selected from the group consisting of SEQ ID NOs: 5, 6, 7, and 8; and
a light chain constant region represented by an amino acid sequence of SEQ ID NO: 10.

9. A bispecific antibody, comprising: an anti-OX40L antibody or an antigen-binding fragment thereof specifically binding to OX40L; and an anti-TNFa antibody or an antigen-binding fragment thereof specifically binding to TNFα.

10. The bispecific antibody according to claim 9, wherein the bispecific antibody is a bispecific antibody in which the anti-OX40L antibody or the antigen-binding fragment thereof and the anti-TNFa antibody or the antigen-binding fragment thereof are linked to each other.

11. The bispecific antibody according to claim 10, wherein the anti-OX40L antibody or the antigen-binding fragment thereof binds to at least one epitope represented by an amino acid sequence selected from the group consisting of SEQ ID NOs: 3 and 4.

12. The bispecific antibody according to claim 10, wherein the anti-OX40L antibody or the antigen-binding fragment thereof comprises:
a heavy chain variable region comprising heavy chain CDR1 represented by one amino acid sequence selected from the group consisting of SEQ ID NOs: 12, 13 and 14; heavy chain CDR2 represented by one amino acid sequence selected from the group consisting of SEQ ID NOs: 15, 16, 17 and 18; and heavy chain CDR3 represented by one amino acid sequence selected from the group consisting of SEQ ID NOs: 19, 20, 21 and 22; and
a light chain variable region comprising light chain CDR1 represented by one amino acid sequence selected from the group consisting of SEQ ID NOs: 23 and 24; light chain CDR2 represented by one amino acid sequence selected from the group consisting of SEQ ID NOs: 25 and 26; and light chain CDR3 represented by one amino acid sequence selected from the group consisting of SEQ ID NOs: 27, 28, 29 and 30.

13. The bispecific antibody according to claim 10, wherein the anti-OX40L antibody or the antigen-binding fragment thereof comprises:
(i) an antibody or an antigen-binding fragment thereof comprising: a heavy chain variable region comprising heavy chain CDR1 represented by SEQ ID NO: 12; heavy chain CDR2 represented by SEQ ID NO: 15; and heavy chain CDR3 represented by SEQ ID NO: 19, and a light chain variable region comprising heavy chain CDR1 represented by SEQ ID NO: 23; heavy chain CDR2 represented by SEQ ID NO: 25; and light chain CDR3 represented by SEQ ID NO: 27;
(ii) an antibody or an antigen-binding fragment thereof comprising: a heavy chain variable region comprising heavy chain CDR1 represented by SEQ ID NO: 13; heavy chain CDR2 represented by SEQ ID NO: 16; and heavy chain CDR3 represented by SEQ ID NO: 20, and a light chain variable region comprising light chain CDR1 represented by SEQ ID NO: 24; light chain CDR2 represented by SEQ ID NO: 26; and light chain CDR3 represented by SEQ ID NO: 28;
(iii) an antibody or an antigen-binding fragment thereof comprising: a heavy chain variable region comprising heavy chain CDR1 represented by SEQ ID NO: 13; heavy chain CDR2 represented by SEQ ID NO: 17; and heavy chain CDR3 represented by SEQ ID NO: 21, and a light chain variable region comprising light chain CDR1 represented by SEQ ID NO: 24; light chain CDR2 represented by SEQ ID NO: 26; and light chain CDR3 represented by SEQ ID NO: 29; or
(iv) an antibody or an antigen-binding fragment thereof comprising: a heavy chain variable region comprising heavy chain CDR1 represented by SEQ ID NO: 14; heavy chain CDR2 represented by SEQ ID NO: 18; and heavy chain CDR3 represented by SEQ ID NO: 22, and a light chain variable region comprising light chain CDR1 represented by SEQ ID NO: 24; light chain CDR2 represented by SEQ ID NO: 26; and light chain CDR3 represented by SEQ ID NO: 30.

14. The bispecific antibody according to claim 10, wherein the anti-OX40L antibody or the antigen-binding fragment thereof comprises:
a heavy chain variable region represented by one amino acid sequence selected from the group consisting of SEQ ID NOs: 33, 37, 41, 45, 49 and 53; and
a light chain variable region represented by one amino acid sequence selected from the group consisting of SEQ ID NOs: 34, 38, 42, 46, 50 and 54.

15. The bispecific antibody according to claim 10, wherein the anti-OX40L antibody or the antigen-binding fragment thereof comprises:
(a) a heavy chain variable region represented by SEQ ID NO: 37 and a light chain variable region represented by SEQ ID NO: 38;
(b) a heavy chain variable region represented by SEQ ID NO: 41 and a light chain variable region represented by SEQ ID NO: 42;
(c) a heavy chain variable region represented by SEQ ID NO: 45 and a light chain variable region represented by SEQ ID NO: 46;
(d) a heavy chain variable region represented by SEQ ID NO: 49 and a light chain variable region represented by SEQ ID NO: 50;
(e) a heavy chain variable region represented by SEQ ID NO: 53 and a light chain variable region represented by SEQ ID NO: 54; or
(f) a heavy chain variable region having an amino acid sequence represented by SEQ ID NO: 33 and a light chain variable region having an amino acid sequence represented by SEQ ID NO: 34.

16. The bispecific antibody according to any one of claims 10 to 15, wherein the anti-TNFα antibody or the antigen-binding fragment thereof comprises:
a heavy chain variable region comprising heavy chain CDR1 represented by SEQ ID NO: 89; heavy chain CDR2 represented by SEQ ID NO: 90; and heavy chain CDR3 represented by SEQ ID NO: 91; and
a light chain variable region comprising light chain CDR1 represented by SEQ ID NO: 92; light chain CDR2 represented by SEQ ID NO: 93; and light chain CDR3 represented by SEQ ID NO: 94.

17. The bispecific antibody according to claim 16, wherein the anti-TNFa antibody or the antigen-binding fragment thereof comprises a heavy chain variable region represented by SEQ ID NO: 35 and a light chain variable region represented by SEQ ID NO: 36.

18. The bispecific antibody according to claim 16, wherein the bispecific antibody binds to human OX40L with a K_{D} of 1.5 × 10⁻⁹ M or less, and binds to human TNFα with a K_{D} of 1 × 10⁻⁹ M or less, and wherein the K_{D} is measured by surface plasmon resonance (Biacore) analysis.

19. The bispecific antibody according to claim 16, wherein the bispecific antibody is a bispecific antibody, in which an anti-TNFa antibody or an antigen-binding fragment thereof specifically binding to TNFα is linked to at least one terminus of the light and heavy chains of the anti-OX40L antibody.

20. The bispecific antibody according to claim 19, wherein the bispecific antibody is a bispecific antibody, in which the anti-TNFa antibody or the antigen-binding fragment thereof is linked to at least one C terminus of the light and heavy chains of the anti-OX40L antibody.

21. The bispecific antibody according to claim 16, wherein the bispecific antibody is a bispecific antibody, in which an anti-OX40L antibody or an antigen-binding fragment thereof specifically binding to OX40L is linked to at least one terminus of the light and heavy chains of the anti-TNFa antibody.

22. The bispecific antibody according to claim 21, wherein the anti-OX40L antibody or the antigen-binding fragment thereof is linked to at least one C terminus of the light and heavy chains of the anti-TNFa antibody.

23. The bispecific antibody according to claim 16, wherein the anti-OX40L antibody or the antigen-binding fragment thereof and the anti-TNFa antibody or the antigen-binding fragment thereof are linked by a linker.

24. The bispecific antibody according to claim 23, wherein the linker is represented by SEQ ID NO: 31 or SEQ ID NO: 32.

25. A nucleic acid encoding an antibody or an antigen-binding fragment thereof according to any one of claims 1 to 8 or a bispecific antibody according to any one of claims 9 to 24.

26. An expression vector comprising nucleic acid according to claim 25.

27. A transformant comprising the expression vector according to claim 26 introduced thereinto.

28. A method for producing an antibody or an antigen fragment thereof, or a bispecific antibody by using the transformant of claim 27.

29. A pharmaceutical composition for preventing or treating autoimmune diseases or inflammatory diseases, comprising an antibody or an antigen-binding fragment thereof according to any one of claims 1 to 8 or a bispecific antibody according to any one of claims 9 to 24.

30. The pharmaceutical composition according to claim 29, wherein the pharmaceutical composition prevents or treats rheumatoid arthritis.

31. A method for providing information on diagnosis of autoimmune diseases or inflammatory diseases by using an antibody or an antigen-binding fragment thereof according to any one of claims 1 to 8 or a bispecific antibody according to any one of claims 9 to 24.

32. A kit for providing information on diagnosis of autoimmune diseases or inflammatory diseases, comprising an antibody or an antigen-binding fragment thereof according to any one of claims 1 to 8 or a bispecific antibody according to any one of claims 9 to 24.

33. A method for preventing or treating autoimmune diseases or inflammatory diseases, the method comprising: administering a pharmaceutically effective amount of an antibody or an antigen-binding fragment thereof according to any one of claims 1 to 8 or a bispecific antibody according to any one of claims 9 to 24.

34. Use of an antibody or an antigen-binding fragment thereof according to any one of claims 1 to 8 or a bispecific antibody according to any one of claims 9 to 24 in the manufacture of a medicament for preventing or treating autoimmune diseases or inflammatory diseases.

35. Use of an antibody or an antigen-binding fragment thereof according to any one of claims 1 to 8 or a bispecific antibody according to any one of claims 9 to 24 for preventing or treating autoimmune diseases or inflammatory diseases.
